(19) **Europäisches Patentamt / European Patent Office / Office européen des brevets**

(11) **EP 3 963 335 B1**

(12) **EUROPEAN PATENT SPECIFICATION**

(45) Date of publication and mention of the grant of the patent:
**21.08.2024 Bulletin 2024/34**

(21) Application number: **20729893.6**

(22) Date of filing: **04.05.2020**

(51) International Patent Classification (IPC):
**G01N 33/68** (2006.01)    **A61K 39/00** (2006.01)
**A61P 35/00** (2006.01)    **G01N 33/574** (2006.01)
**G16B 15/30** (2019.01)    **G16B 20/20** (2019.01)
**G16H 50/30** (2018.01)

(52) Cooperative Patent Classification (CPC):
**G01N 33/6878; A61K 39/00; A61K 39/0011;**
**A61P 35/00; G01N 33/57492; G16B 15/30;**
**G16B 20/20; G16H 50/30;** A61K 2039/55561;
A61K 2039/572; A61K 2039/80; A61K 2039/82;
A61K 2039/852; G01N 2800/52; G01N 2800/7023;

(Cont.)

(86) International application number:
**PCT/US2020/031357**

(87) International publication number:
**WO 2020/227233 (12.11.2020 Gazette 2020/46)**

(54) **NEOANTIGENS IN CANCER**

NEOANTIGENE BEI KREBS

NÉOANTIGÈNES UTILISÉS DANS L'IDENTIFICATION DU CANCER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.05.2019 US 201962842800 P
31.07.2019 US 201962880965 P
08.11.2019 US 201962932651 P
08.11.2019 US 201962932654 P**

(43) Date of publication of application:
**09.03.2022 Bulletin 2022/10**

(60) Divisional application:
**22208033.5 / 4 198 513**

(73) Proprietors:
- **EpiVax Therapeutics, Inc.**
  **Providence, RI 02909 (US)**
- **New York University**
  **New York, NY 10012 (US)**
- **The University of Chicago**
  **Chicago, IL 60637 (US)**

(72) Inventors:
- **RICHARD, Guilhem**
  **New York, NY 10014 (US)**
- **SWEIS, Randy F.**
  **Chicago, IL 60637 (US)**
- **ARDITO, Matthew**
  **Providence, RI 02909 (US)**
- **GARCIA, Tzintzuni**
  **Chicago, IL 60637 (US)**
- **MOISE, Leonard**
  **Providence, RI 02909 (US)**
- **PRINCIOTTA, Michael F.**
  **New York, NY 10014 (US)**
- **BRIDON, Dominique**
  **New York, NY 10014 (US)**
- **MARTIN, William D.**
  **Providence, RI 02909 (US)**
- **BERDUGO, Gad**
  **New York, NY 10014 (US)**
- **BALAR, Arjun**
  **New York, NY 10016 (US)**
- **STEINBERG, Gary D.**
  **New York, NY 10016 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DE GROOT, Anne S.
  Providence, RI 02909 (US)

(74) Representative: **Schüssler, Andrea**
**Kanzlei Huber & Schüssler**
**Truderinger Strasse 246**
**81825 München (DE)**

(56) References cited:
- S. D. BROWN ET AL: "Neo-antigens predicted by tumor genome meta-analysis correlate with increased patient survival", GENOME RESEARCH, vol. 24, no. 5, 29 April 2014 (2014-04-29), US, pages 743 - 750, XP055262143, ISSN: 1088-9051, DOI: 10.1101/gr.165985.113
- MARTA LUKSZA ET AL: "A neoantigen fitness model predicts tumour response to checkpoint blockade immunotherapy", NATURE, 8 November 2017 (2017-11-08), London, XP055506171, ISSN: 0028-0836, DOI: 10.1038/nature24473
- BALACHANDRAN VINOD P ET AL: "Identification of unique neoantigen qualities in long-term survivors of pancreatic cancer", NATURE, NATURE PUBLISHING GROUP UK, LONDON, vol. 551, no. 7681, 1 November 2017 (2017-11-01), pages 512 - 516, XP037202983, ISSN: 0028-0836, [retrieved on 20171101], DOI: 10.1038/NATURE24462
- GIUSEPPEV MASUCCI ET AL: "Validation of biomarkers to predict response to immunotherapy in cancer: Volume I - pre-analytical and analytical validation", JOURNAL FOR IMMUNOTHERAPY OF CANCER, BIOMED CENTRAL LTD, LONDON, UK, vol. 4, no. 1, 15 November 2016 (2016-11-15), pages 1 - 25, XP021241446, DOI: 10.1186/S40425-016-0178-1
- JIANDA YUAN ET AL: "Novel technologies and emerging biomarkers for personalized cancer immunotherapy", JOURNAL FOR IMMUNOTHERAPY OF CANCER, BIOMED CENTRAL LTD, LONDON, UK, vol. 4, no. 1, 19 January 2016 (2016-01-19), pages 1 - 25, XP021233391, DOI: 10.1186/S40425-016-0107-3
- GUILHEM RICHARD ET AL: "Selection of Class I and Class II T cell neo-epitopes and removal of Treg epitopes", 1 July 2018 (2018-07-01), XP055727117, Retrieved from the Internet <URL:https://cancerres.aacrjournals.org/content/78/13_Supplement/5311> [retrieved on 20200902], DOI: 10.1158/1538-7445.AM2018-5311
- A. CORTHAY: "How do Regulatory T Cells Work?", SCANDINAVIAN JOURNAL OF IMMUNOLOGY, vol. 70, no. 4, 1 October 2009 (2009-10-01), pages 326 - 336, XP055197142, ISSN: 0300-9475, DOI: 10.1111/j.1365-3083.2009.02308.x

(52) Cooperative Patent Classification (CPC): (Cont.)
G01N 2800/7028; Y02A 90/10

**Description**

RELATED APPLICATIONS

This application claims priority to US provisional application Serial No. 62/842,800, filed May 3, 2019, US provisional application Serial No. 62/880,965, filed July 31, 2019, US provisional application Serial No. 62/932,651, filed November 8, 2019, US provisional application Serial No. 62/932,654, filed November 8, 2019, and US provisional application Serial No. 62/936,654, filed November 18, 2019.

Reference is made to international application Serial No. PCT/US2020/020089, filed February 27, 2020 and to US provisional application Serial No. 62/811,207, filed February 27, 2019.

FIELD OF THE INVENTION

The technical field of the present invention relates to improved strategies, prognostic indicators, compositions, and methods for producing and using personalized neoplasia vaccines. More particularly, the technical field of the present invention relates to the identification of neoplasia-specific neoantigens to identify and design subject-specific neo-epitopes, further assessing the identified neo-epitopes encoded by said mutations to identify neo-epitopes that are known or determined (e.g. predicted) to engage regulatory T cells and/or other detrimental T cells (including T cells with potential host crossreactivity and/or anergic T cells), and excluding such identified neo-epitopes that are known or determined (e.g. predicted) to engage regulatory T cells and/or other detrimental T cells (including T cells with potential host cross-reactivity and/or anergic T cells), from the subject-specific neo-epitopes that are to be used in personalized neoplasia vaccines.

BACKGROUND OF THE INVENTION

There are many existing cancer therapies, including ablation techniques (e.g., surgical procedures, cryogenic/heat treatment, ultrasound, radiofrequency, and radiation) and chemical techniques (e.g., pharmaceutical agents, cytotoxic/chemotherapeutic agents, monoclonal antibodies, and various combinations thereof). However, these therapies are frequently associated with serious risk, toxic side effects, and extremely high costs, as well as uncertain efficacy. More recently, clinical studies have highlighted the potential of precision cancer immunotherapy to effectively control the cancer of patients by harnessing a patient's own immune system. Such precision cancer immunotherapies include the identification and use of a patient-specific pool of neoplasia-specific neoantigens in a personalized vaccine. However, while several different methodologies for preparing personalized neoplasia vaccines have been employed, recent studies showcase the difficulty of establishing robust CD8$^+$ and CD4$^+$ effector T cell responses to effectively treat the targeted neoplasia. This difficulty may be due to the inadvertent inclusion of suppressive T cell neo-epitopes in neoantigen-based vaccines that may be recognized by, and thus activate, regulatory T cells, which may abrogate effective immune responses against tumor cells. Further, T cells that recognize antigen-derived epitopes sharing TCR contacts with epitopes derived from self may be deleted or rendered anergic during thymic selection before they can be released to the periphery. As such, vaccine components targeting these T cells may be ineffective. On the other hand, vaccine-induced immune response targeting cross-reactive epitopes may induce unwanted autoimmune responses targeting the homologs of the cross-reactive epitopes identified by homology search. As a result, vaccine safety may be reduced. Thus, the inadvertent inclusion of other detrimental T cell-neo-epitopes in neoantigen-based vaccines that may be recognized by, and thus activate, other detrimental T cells (including T cells with potential host cross-reactivity that may lead to autoimmune responses, as well as anergic T cells) may also lead to ineffective immune responses against tumor cells.

Immune tolerance is regulated by a complex interplay between antigen presenting cells (APC), T cells, B cells, cytokines, chemokines, and surface receptors. Initial self/non-self discrimination occurs in the thymus during neonatal development where medullary epithelial cells express specific self protein epitopes to immature T cells. T cells recognizing self antigens with high affinity are deleted, but autoreactive T cells with moderate affinity sometimes avoid deletion and can be converted to so called 'natural' regulatory T cells. These natural regulatory T cells are exported to the periphery and help to control latent autoimmune response.

A second form of tolerance develops in the periphery. In this case activated T cells are converted to an 'adaptive' regulatory T cells phenotype through the action of certain immune suppressive cytokines and chemokines such as IL-10, TGF-β and CCL19. The possible roles for these 'adaptive' regulatory T cells include dampening immune response following the successful clearance of an invading pathogen, controlling excessive inflammation caused by an allergic reaction, controlling excessive inflammation caused by low level or chronic infection, or possibly controlling inflammatory response targeting beneficial symbiotic bacteria.

Naturally occurring regulatory T cells (including both natural regulatory T cells and adaptive regulatory T cells) are a critical component of immune regulation in the periphery. For example, upon activation of natural regulatory T cells through their TCR, natural regulatory T cells express immune modulating cytokines and chemokines. Activated natural regulatory T cells may suppress nearby effector T cells through contact dependent and independent mechanisms. In addition, the cytokines released by these cells including, but not limited to, IL-10 and TGF-β, are capable of inducing antigen-specific adaptive regulatory T cells. However, although regulatory T cells activity is essential for prevention of autoimmunity, excessive regulatory T cells function may abrogate effective immune responses against tumor cells (Nishikawa et al., "Regulatory T Cells in Tumor Immunity," Int. J. Cancer 127:759-767 (2010)). Indeed, down-regulation of regulatory T cell activity has been used as an effective tool to improve anticancer therapies (Grauer et al., "Elimination of Regulatory T Cells is Essential for an Effective Vaccination with Tumor Lysate-Pulsed Dendritic Cells in a Murine Glioma Model," Int. J. Cancer 122:1794-1802 (2008); Zhou et al., "Depletion of Endogenous Tumor-Associated Regulatory T Cells Improves the Efficacy of Adoptive Cytotoxic T-Cell Immunotherapy in Murine Acute Myeloid Leukemia," Blood 114:3793-3802 (2009)). Thus, inadvertent inclusion of suppressive T cell neo-epitopes in neoantigen-based vaccines that may be recognized by, and thus activate, regulatory T cells, must be avoided to prevent the abrogation of an effective immune response against tumor cells.

As such, there is an ongoing need for improved strategies, compositions, and methods for producing personalized neoplasia vaccines. More particularly, there remains an ongoing need for strategies, compositions, and methods for producing personalized neoplasia vaccines that include identifying neo-epitopes that are known or determined (e.g. predicted) to engage and/or induce regulatory T cells and/or other detrimental T cells (including T cells with potential host cross-reactivity and/or anergic T cells), and excluding such identified neo-epitopes that are known or determined (e.g. predicted) to engage regulatory T cells and/or other detrimental T cells (including T cells with potential host cross-reactivity and/or anergic T cells) from the subject-specific neo-epitopes that are to be used in the personalized neoplasia vaccines. There also remains an ongoing need for improved ranking systems to determine the optimal subject-specific neo-epitopes that are to be used in personalized neoplasia vaccines.

Brown et al., Genome Research, Vol. 24, No. 5, pp. 743-750 (2014) reports about the result that neo-antigens predicted by tumor gemome meta-analysis correlate with increased patient survival.

Masucci et al., Journal for ImmunoTherapy of Cancer, Vol. 4, No. 1, pp. 1-25 (2016) describes the validation of biomarkers to predict response to immunotherapy in cancer.

Richard et al., AACR Annual Meeting 2018, Abstract 5311 describes integrated approaches for design of precision cancer immunotherapies. They are based on the selection of class I and class II T cell neo-epitopes and removal of Treg epitopes.

SUMMARY OF THE INVENTION

The present invention concerns a prognostic method as defined in claim 1. Preferred embodiments of said method are defined in the dependent claims. Particularly, the invention provides a prognostic method for determining risk of death of a human subject with a neoplasia, which comprises identifying a population of neoplasia-specific mutations in a neoplasia specimen of a subject, assessing the neoplasia-specific mutations identified to classify Class I and Class II MHC-binding neoantigens encoded by said mutations, analyzing the neoantigens encoded by said mutations to identify and classify neoantigens that engage effector T cells and neo-epitopes that engage regulatory T cells, and computing a prognostic score from the immunogenicity of the population, wherein assessing neoplasia-specific mutations comprises:

  a) determining a binding score for a mutated peptide to one or more MHC molecules, wherein said mutated peptide is encoded by at least one of said neoplasia-specific mutations;
  b) determining a binding score for a non-mutated peptide to the one or more MHC molecules, wherein the non-mutated peptide is identical to the mutated peptide except for the encoded at least one of said neoplasia-specific mutations;
  c) determining the percentile rank of the binding scores of both the mutated peptide of step (a) and the non-mutated peptide of step (b) as compared to an expected distribution of binding scores for at least 10,000 randomly generated peptides using naturally observed amino acid frequencies;
  d) determining the TCR facing amino acid residues of said mutated peptide and said non-mutated peptide; and
  e) using it to calculate a prognostic score when:

    1) the mutated peptide has a determined binding score in the top 5 percentile of the expected distribution and the non-mutated peptide has a determined binding score below the top 10 percentile of the expected distribution; or

    2) the mutated peptide has a determined binding score in the top 5 percentile of the expected distribution, the non-mutated peptide has a determined binding score in the top 10 percentile of the expected distribution, and there is at least one mismatched TCR facing amino acid between the mutated peptide the non-mutated peptide.

In an aspect, the prognostic method is used to distinguish subjects with neoplasias that are best suited to respond to a neoantigen vaccine. In another aspect, the prognostic method is used to identify subjects that would benefit improved outcomes by coadministration of a neoantigen vaccine and an agent that selectively inhibits Treg function.

The prognostic method of the present invention comprises identifying a population of neoplasia-specific mutations in a neoplasia specimen of a subject; assessing the neoplasia-specific mutations identified to classify Class I and Class II neoantigens encoded by said mutations; analyzing the neoantigens encoded by said mutations to identify and classify neoantigens that engage effector T cells and neo-epitopes that engage regulatory T cells, and determining a prognostic score from the immunogenicity of the neoantigen population, wherein assessing neoplasia-specific mutations comprises steps (a) to (e) as outlined above. In certain embodiments, the method is implemented on a computer.

The method of the invention is designed to classify, order, or otherwise recognize Class I and Class II neoantigens as effector neoantigens or tolerogenic antigens, or classify any neoantigen in between, for any given patient. The invention is further designed to recognize neoantigens that are crossreactive, for example having Class II effector and Class I tolerogenic activity, or Class I effector and Class II tolerogenic activity, or other combinations that are detrimental to prognosis or treatment outcomes.

Thus, in an embodiment of the invention, the method comprises classification of Class I neoantigens of a subject as effector neoantigens or tolerogenic neoantigens. In another embodiment, the method comprises classification of the Class I neoantigens of a subject as effector neoantigens, tolerated neoantigens, or tolerogenic neoantigens. In another embodiment, the method comprises classification of the Class I neoantigens of a subject on a graded scale from a strong effector neoantigen to a strong tolerogenic neoantigen.

In an embodiment of the invention, the method comprises classification of the Class II neoantigens of a subject as effector neoantigens or tolerogenic neoantigens. In another embodiment, the method comprises classification of the Class II neoantigens of a subject as effector neoantigens, tolerated neoantigens, or tolerogenic neoantigens. In another embodiment, the method comprises classification of the Class II neoantigens of a subject on a graded scale from a strong effector neoantigen to a strong regulatory neoantigen.

In certain embodiments, the method comprises classification of the Class I neoantigens and classification of the Class II neoantigens of the subject and can include identification of subject neoantigens that are cross-reactive between Class I and Class II.

Such embodiments of the invention may exclude neoantigens from prognostic or diagnostic methods if the neoantigen is a Class I effector neoantigen and a Class II tolerogenic neoantigen or a Class II effector neoantigen and a Class I tolerogenic neoantigen, or a tolerated Class I neoantigen and a tolerated Class II antigen, or an effector Class II neoantigen and a tolerated Class I neoantigen.

In certain embodiments, the prognostic score is calculated based on the top 50% of the effector neoantigens and the top 50% of the tolerogenic neoantigens, or the top 40% of effector neoantigens and the top 40% of the tolerogenic neoantigens, or the top 30% of effector neoantigens and the top 30% of the tolerogenic neoantigens, or the top 20% of the effector neoantigens and the top 20% of the tolerogenic neoantigens, or the top 10% of the effector neoantigens and the top 10% of the tolerogenic neoantigens, or the top 5% of the effector neoantigens and the top 5% of the tolerogenic neoantigens, or the top 2% of the effector neoantigens and the top 2% of the tolerogenic neoantigens, or the top 1% of the effector neoantigens and the top 1% of the tolerogenic neoantigens. In certain embodiments, the prognostic score is calculated based on at least the top 10 of the effector neoantigens and at least the top 10 of the tolerogenic neoantigens, or at least the top 25 of the effector neoantigens and at least the top 25 of the tolerogenic neoantigens, or at least on the top 50 of the effector neoantigens and at least the top 50 of the tolerogenic neoantigens, or at least on the top 100 of the effector neoantigens and at least the top 100 of the tolerogenic neoantigens.

In certain embodiments, the method comprises determining binding strength of a neoantigen to Teff and/or Treg cells. The strength of binding to Teff and/or Treg cells may be known, measured, predicted or calculated. In certain embodiments, the method comprises determining immunogenicity of a neoantigen by comparision to physicochemical methods and/or by comparison to a panel of antigens of known immunogenicity. In certain embodiments, the activity of a neoantigen in a subject is determined by its capacity to activate or inhibit an immune cell, such as, for example, to inhibit IFN$\gamma$ production by itself or mixed with other antigens or neoantigens. In some embodiments, strength of binding to a Teff cell or a Treg cell is determined by comparison to a panel of neoantigens having predetermined Teff and/or Treg activity.

Accordingly, the invention provides prognostic biomarkers. The method involves identifying a plurality of neoplasia-specific mutations (neo-epitopes) in a subject and determining the extent to which a population of neoantigens containing the the neo-epitopes is immunogenic. Higher overall immunogenicity, i.e. higher capability to stimulate effector cells and lower capability to stimulate regulatory cells indicates a better prognosis.

Furthermore, the present application further describes a method of identifying subject-specific neo-epitopes for a personalized neoplasia vaccine, wherein said method is not mentioned in the present claims. The method of identifying subject-specific neo-epitopes for a personalized neoplasia vaccine includes: i) identifying neoplasia-specific mutations in a neoplasia specimen of a subject diagnosed as having a neoplasia; ii) to identify Class I and Class II neo-epitopes encoded by said mutations for use in the personalized neoplasia vaccine, wherein said neo-epitopes are known, determined, or predicted to bind to a MHC protein of the subject; and iii) classifying the identified neo-epitopes encoded by said mutations from step (ii) to identify Class I and Class II neo-epitopes that are that are tolerated or tolerogenic, and excluding such Class II neo-epitopes that are tolerated or tolerogenic, and excluding such Class I neo-epitopes that are tolerogenic, from the subject-specific neo-epitopes for use in the personalized neoplasia vaccine.

Described is further the identification of sequence differences between the full or partial genome, exome, and/or transcriptome of a neoplasia specimen from the subject diagnosed as having a neoplasia and a non-neoplasia specimen. In aspects, a non-neoplasia specimen is derived from the subject diagnosed as having a neoplasia. In further aspects, identifying neoplasia-specific mutations or identifying sequence differences comprises Next Generation Sequencing (NGS). In aspects, identifying neoplasia-specific mutations in step (i) comprises selecting from the neoplasia a plurality of nucleic acid sequences, each comprising mutations, which are not present in a non-neoplasia sample. In aspects, identifying neoplasia-specific mutations or identifying sequence differences comprises sequencing genomic DNA and/or RNA of the neoplasia specimen.

In aspects of the invention, the neoplasia-specific mutations are neoplasia-specific somatic mutations. In aspects, the neoplasia-specific mutations are single nucleotide variations (SNVs), insertions and deletions (which can generate both in-frame and frameshift mutations), and other large-scale rearrangements such as but not limited to chromosomal inversions, duplications, insertions, deletions, or translocations. In aspects, neoplasia specific mutations, including SNVs, insertions, and deletions, are non-synonymous mutations. In aspects, neoplasia-specific mutations, including SNVs, insertions and deletions (which can be non-synonymous mutations), and other large-scale rearrangements, are mutations of proteins encoded in the neoplasia specimen of the subject diagnosed as having a neoplasia. In aspects, neoplasia specific mutations, including SNVs, are non-synonymous mutations. In aspects, neoplasia-specific mutations, including SNVs (which can be non-synonymous mutations), indels, and frameshifts, are mutations of proteins expressed in the neoplasia specimen of the subject diagnosed as having a neoplasia.

In aspects of the invention, assessing the neoplasia-specific mutations to identify known or determined (e.g. predicted) neo-epitopes encoded by said mutations includes: a) determining a binding score for a mutated peptide to one or more MHC molecules, wherein said mutated peptide is encoded by at least one of said neoplasia-specific mutations; b) determining a binding score for a non-mutated peptide to the one or more MHC molecules, wherein the non-mutated peptide is identical to the mutated peptide except for the encoded at least one of said neoplasia-specific mutations; c) determining the percentile rank of the binding scores of both the mutated peptide of step (a) and the non-mutated peptide of step (b) as compared to an expected distribution of binding scores for a sufficiently large enough group randomly generated peptides (e.g., at least 10,000) using naturally observed amino acid frequencies; d) determining the TCR facing amino acid residues of said mutated peptide and said non-mutated peptide; and e) identifying the mutated peptide as a neo-epitope when: 1) the mutated peptide has a determined binding score in the top 5 percentile of the expected distribution and the non-mutated peptide has a determined binding score below the top 10 percentile of the expected distribution; or 2) the mutated peptide has a determined binding score in the top 5 percentile of the expected distribution, the non-mutated peptide has a determined binding score in the top 10 percentile of the expected distribution, and there is at least one mismatched TCR facing amino acid between the mutated peptide and the non-mutated peptide. In further aspects, the one or more MHC molecules are MHC class I molecules and/or MHC class II molecules.

The mutated peptide and non-mutated peptide are both 9 amino acids in length or the mutated peptide and non-mutated peptide are both 10 amino acids in length. In aspects, the TCR facing amino acid residues for a 9-mer mutated peptide and a 9-mer non-mutated peptide that bind to a MHC class II molecule are at position 2, 3, 5, 7, and 8 of the mutated and non-mutated peptide as counted from the amino terminal, wherein the TCR facing amino acid residues for a 9-mer mutated peptide and a 9-mer non-mutated peptide that bind to a MHC class I molecule are at position 4, 5, 6, 7, and 8 of the mutated and non-mutated peptide as counted from the amino terminal, and wherein the TCR facing amino acid residues for a 10-mer mutated peptide and 10-mer non- mutated peptide that bind to a MHC class I molecule are at position 4, 5, 6, 7, 8, and 9 of the mutated and non-mutated peptide as counted from the amino terminal. In further aspects, TCR contacts for a 9-mer identified neo-epitope that binds to a MHC class II molecule are at any combination of residues at positions 2, 3, 5, 7, and 8 (e.g., but not limited to, positions 3, 5, 7 and 8; positions 2, 5, 7, and 8; positions 2, 3, 5, and 7, etc.) as counted from the amino terminal. In aspects, the TCR contacts for a 9-mer identified neo-epitope that binds to a MHC class I molecule are at positions 4, 5, 6, 7, and 8; 1, 4, 5, 6, 7 and 8; or 1, 3, 4, 5, 6, 7, and 8 of the identified neo-epitope as counted from the amino terminal. In further aspects, the TCR contacts for a 9-mer identified neo-epitope that binds to a MHC class I molecule are at any combination of residues at positions 1, 3, 4, 5, 6, 7, and 8 as counted from the amino terminal. In aspects, the TCR contacts for a 10-mer identified neo-epitope that bind to a MHC class I molecule are at position 4, 5, 6, 7, 8, and 9; 1, 4, 5, 6, 7, 8, and 9; or 1, 3, 4, 5, 6, 7, 8, and 9 of the identified neo-epitope as counted from the amino terminal. In further aspects, the TCR contacts for a 10- mer identified neo-epitope that binds to a MHC class I molecule are at any combination of residues at positions 1, 3, 4, 5, 6, 7, 8, and 9 as counted from the amino terminal.

In aspects of the invention, assessing the neoplasia-specific mutations to identify known or determined (e.g.

predicted) neo-epitopes encoded by said mutations comprises *in silico* testing. In certain aspects, said *in silico* testing to identify known or determined (e.g. predicted) neo-epitopes encoded by said mutations in step (ii) comprises using an algorithm to screen protein sequences for putative T cell epitopes. For example, the algorithm may comprise the Epi-Matrix® algorithm.

In aspects of the invention, assessing the identified neo-epitopes encoded by said mutations to identify neo-epitopes that are known or determined (e.g. predicted) to engage regulatory T cells and/or other detrimental T cells (including T cells with potential host cross-reactivity and/or anergic T cells) comprises determining whether said identified neo-epitopes encoded by said mutations share TCR contacts with proteins derived from either the human proteome or the human microbiome, wherein said identified neo-epitopes encoded by said mutations that are determined to share TCR contacts with proteins derived from either the human proteome or the human microbiome may be identified as neo-epitopes that are known or determined (e.g. predicted) to engage regulatory T cells and/or other detrimental T cells (including T cells with potential host cross-reactivity and/or anergic T cells). In aspects, TCR contacts for a 9-mer identified neo-epitope that binds to a MHC class II molecule are at position 2, 3, 5, 7, and 8 of the identified neo-epitope as counted from the amino terminal, wherein the TCR contacts for a 9- mer identified neo-epitope that binds to a MHC class I molecule are at position 4, 5, 6, 7, and 8 of the identified neo-epitope as counted from the amino terminal, and wherein the TCR contacts for a 10-mer identified neo-epitope that bind to a MHC class I molecule are at position 4, 5, 6, 7, 8, and 9 of the identified neo-epitope as counted from the amino terminal. In further aspects, TCR contacts for a 9-mer identified neo-epitope that binds to a MHC class II molecule are at any combination of residues at positions 2, 3, 5, 7, and 8 (e.g., but not limited to, positions 3, 5, 7 and 8; positions 2, 5, 7, and 8; positions 2, 3, 5, and 7, etc.) as counted from the amino terminal. In aspects, the TCR contacts for a 9-mer identified neo-epitope that binds to a MHC class I molecule are at positions 4, 5, 6, 7, and 8; 1, 4, 5, 6, 7 and 8; or 1, 3, 4, 5, 6, 7, and 8 of the identified neo-epitope as counted from the amino terminal. In further aspects, the TCR contacts for a 9-mer identified neo-epitope that binds to a MHC class I molecule are at any combination of residues at positions 1, 3, 4, 5, 6, 7, and 8 as counted from the amino terminal. In aspects, the TCR contacts for a 10-mer identified neo-epitope that bind to a MHC class I molecule are at position 4, 5, 6, 7, 8, and 9; 1, 4, 5, 6, 7, 8, and 9; or 1, 3, 4, 5, 6, 7, 8, and 9 of the identified neo-epitope as counted from the amino terminal. In further aspects, the TCR contacts for a 10-mer identified neo-epitope that binds to a MHC class I molecule are at any combination of residues at positions 1, 3, 4, 5, 6, 7, 8, and 9 as counted from the amino terminal.

In aspects of the invention, assessing the identified neo-epitopes encoded by said mutations to identify neo-epitopes that are known or determined (e.g. predicted) to engage regulatory T cells and/or other detrimental T cells (including T cells with potential host cross-reactivity and/or anergic T cells) comprises *in silico* testing. In aspects, *in silico* testing comprises analyzing whether the identified neo-epitopes are predicted to engage regulatory T cells and/or other detrimental T cells (including T cells with potential host cross-reactivity and/or anergic T cells) using the JANUSMATRIX™ algorithm. In further aspects, an identified neo-epitope is predicted to engage regulatory T cells and/or other detrimental T cells (including T cells with potential host cross-reactivity and/or anergic T cells) if the JANUSMATRIX™ score for the neo-epitope is greater than or equal to 2 (and in further aspects, greater than or equal to 3). In aspects, the method further comprises determining whether the identified neo-epitopes engage regulatory T cells and/or other detrimental T cells (including T cells with potential host cross-reactivity and/or anergic T cells) *in vitro.* In aspects, a neo-epitope is determined to engage regulatory T cells when said neo-epitope results in regulatory T cell activation, proliferation, and/or IL-10 or TGF-β production.

In aspects of the invention, assessing the identified neo-epitopes encoded by said mutations to identify neo-epitopes that are known or determined (e.g. predicted) to engage regulatory T cells and/or other detrimental T cells (including T cells with potential host cross-reactivity and/or anergic T cells) may comprise determining whether the identified neo-epitopes engage regulatory T cells and/or other detrimental T cells (including T cells with potential host cross-reactivity and/or anergic T cells) *in vitro.* In aspects, a neo-epitope is determined to engage regulatory T cells when said neo-epitope results in regulatory T cell activation, proliferation, and/or IL-10 or TGF-β production.

In aspects of the non-claimed method of identifying subject-specific neo-epitopes for a personalized neoplasia vaccine, the method further includes: designing at least one subject-specific peptide or polypeptide, said peptide or polypeptide comprising at least one identified neo- epitope encoded by said mutations, provided said neo-epitope is not identified as being known or determined (e.g. predicted) to engage regulatory T cells and/or other detrimental T cells (including T cells with potential host cross-reactivity and/or anergic T cells). In aspects, the non-claimed method further includes providing the at least one peptide or polypeptide designed in step (iv) or a nucleic acid encoding said peptides or polypeptides. In even further aspects, the method further includes vi) providing a vaccine comprising the at least one peptide or polypeptide or nucleic acid provided in step (v).

The invention provides a prognostic method for determining risk of death of a human subject with a neoplasia, which comprises identifying a population of neoplasia-specific mutations in a neoplasia specimen of a subject; assessing the neoplasia-specific mutations identified in step (i) to identify Class I and Class II neo-epitopes encoded by said mutations, analyzing the neo-epitopes encoded by said mutations to identify and quantify neo-epitopes that engage effector T cells and neo-epitopes that engage regulatory T cells, and computing a prognostic score from the immunogenicity of the population, wherein assessing neoplasia-specific mutations comprises steps (a) to (e) as outlined above.

BRIEF DESCRIPTION OF THE DRAWINGS

The following detailed description, given by way of example, but not intended to limit the invention solely to the specific embodiments described, may best be understood in conjunction with the accompanying drawings.

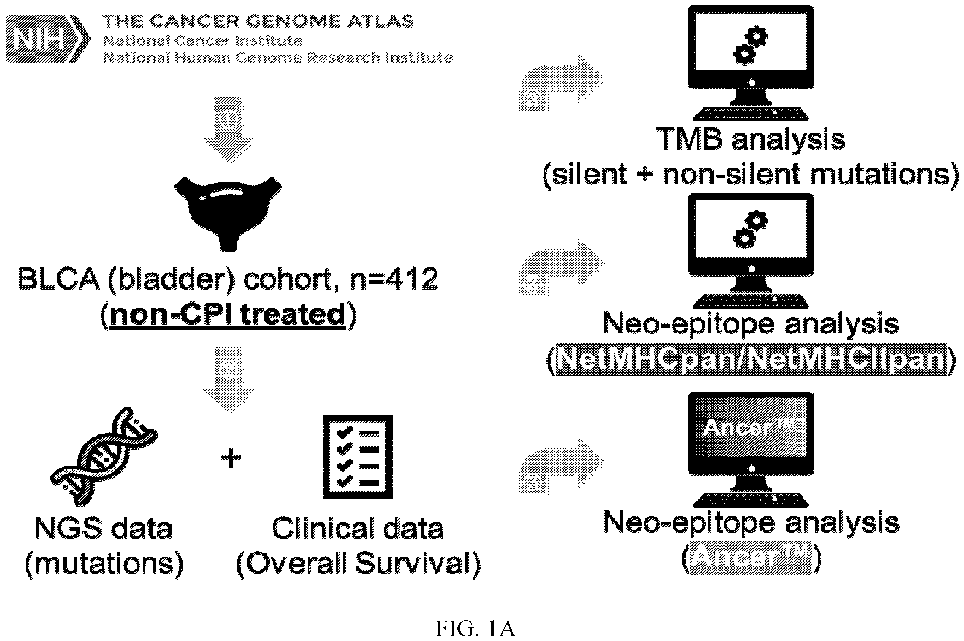

Figure 1A-1C. - Prediction of neo-epitopes. (A) Bladder cancer 5-year survival predictions by three different analysis methods: tumor mutational burden (TMB): neo-epitope analysis of Class I and Class II neo-epitopes (NetMHCpan/NetMHCIIpan), and ANCER™ neoepitope analysis. and were compared using mutation data from The Cancer Genome Atlas (TCGA). (B) Accuracy analysis. (C) Predictive values of Class I and Class II epitopes and filtering out self-like neo-epitopes.

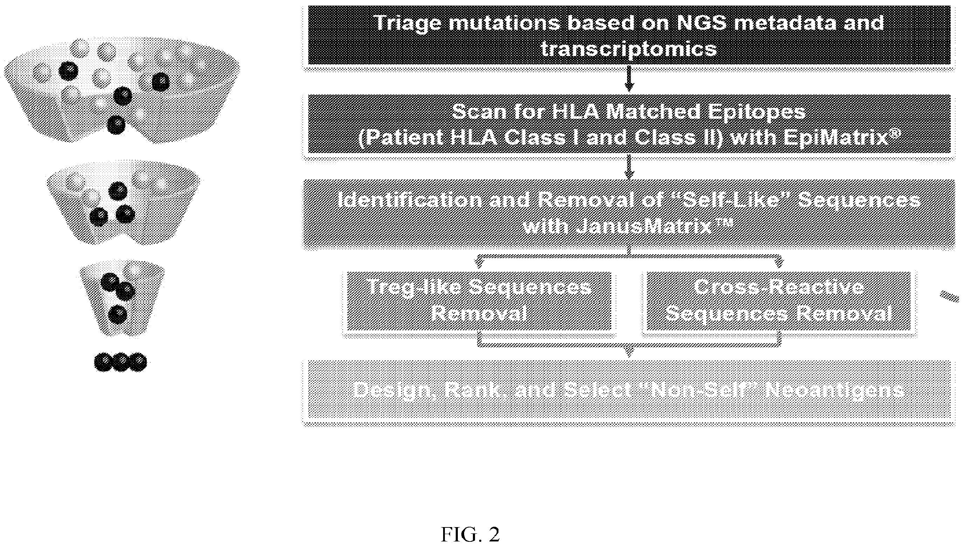

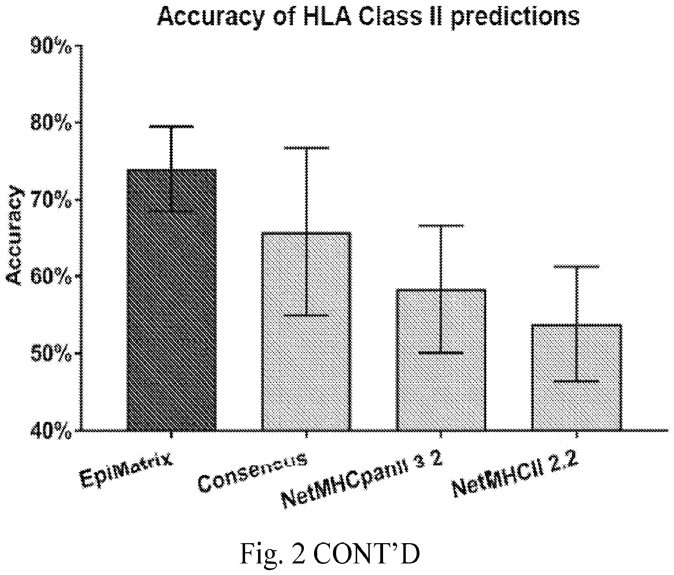

Figure 2. - Accurate and identification of CD4 and CD8 epitopes using EPIMATRIX®. CD4 T cell epitopes - Class II predictions are 74% accurate when prospectively tested in in vitro HLA binding assays. IEDB predictions are 54-66% accurate when tested against the same set of peptides.

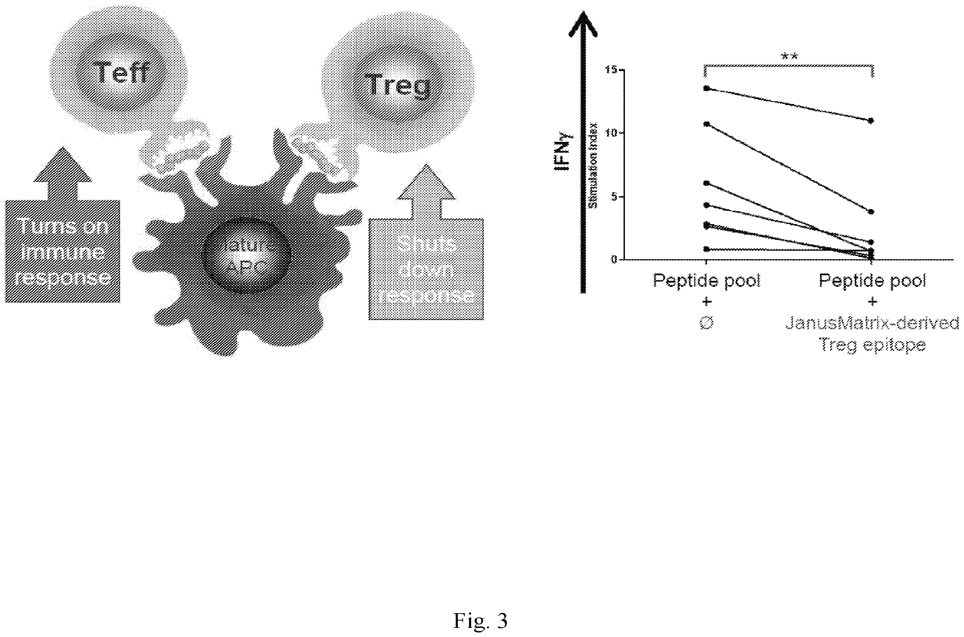

Figure 3. - Epitopes can be either effector or regulatory. JANUSMATRIX™ is an immunoinformatics tool developed to compare T cell epitopes and evaluate neo-epitope peptide sequences as self-like (immunosuppressive; Treg) and non-self. (Moise L. et al., Hum Vaccin Immunother. 2015;11(9):2312-21). As shown in the right panel, in silico-derived influenza (H7N9) Treg epitope reduces IFNγ responses to effector peptides similarly to a JANUSMATRIX™-derived Treg epitope (Liu R. et al., Hum Vaccin Immunother. 2015 11:9, 2241-2252).

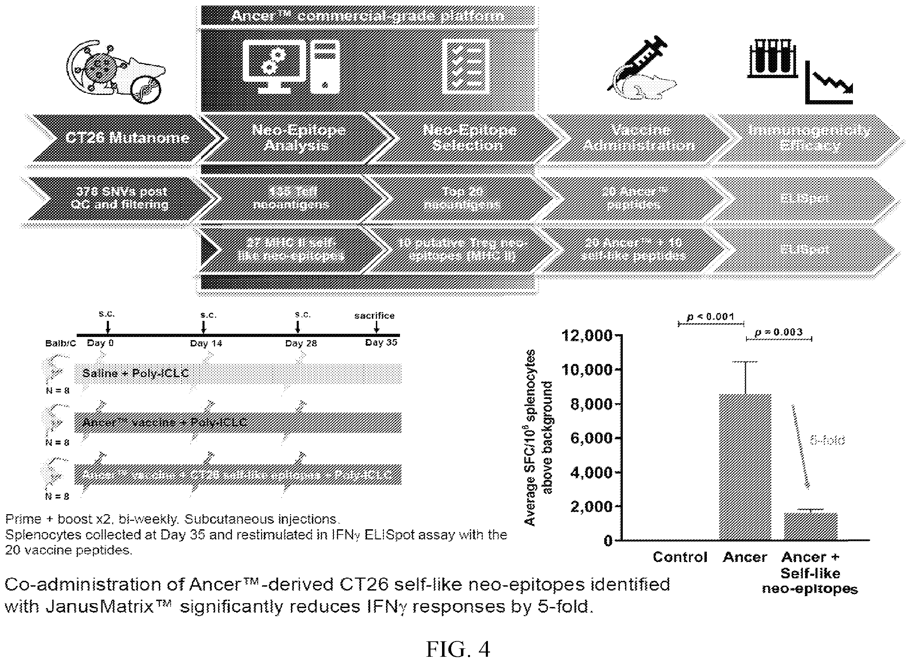

Figure 4. - Immunosuppresion of IFNγ responses by CT26 self-like neo-epitopes. ANCER™-derived Teff neo-epitopes induce an IFNγ response, while self-like neo-epitopes identified by JANUSMATRIX™ suppress the response 5-fold.

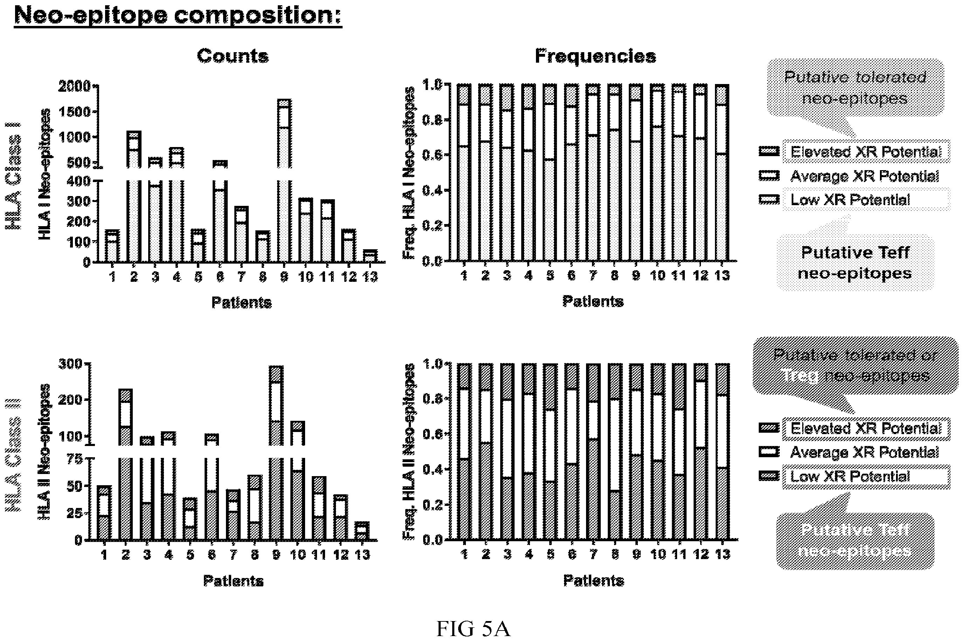

Figure 5A-5B. - Analysis of Class I and Class II neo-epitopes in 13 GVAX-treated pancreatic patients. (A) Neo-epitope composition. Number and frequency of Class I and Class II neo-epitopes with low, average, and high cross-reactivity (XR) potential across pancreatic cancer patients. (B) The ratio of MHC Class II effector (Teff) vs. regulatory (Treg) neo-epitopes found in tumor is associated with tumor outcomes. Patients that are (Teff/Treg)$^{hi}$ have a higher disease free survival (DFS) time than (Teff/Treg)$^{lo}$ patients and the ratio is a more sensitive predictor than tumor mutational burden.

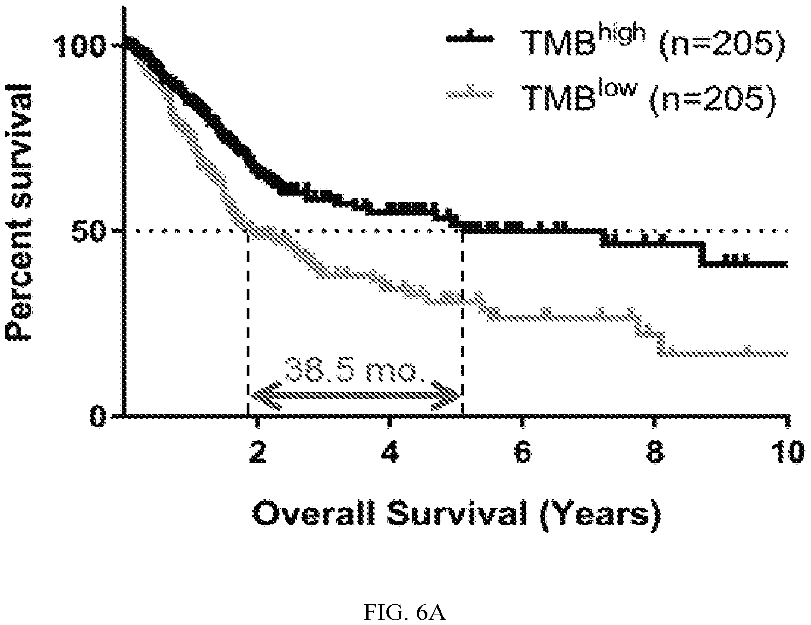

Figure 6A-6E. - TCGA marker analysis. (A) Patients separated on the basis of total mutational burden. (B) Patients separated on the basis of raw CD8 neo-epitope content. (C) Patients separated on basis of CD8 neo-epitope content with "tolerated" neo-epitopes removed. (D) Patients separated on basis of CD8 neo-epitope content with "tolerated" CD8 neo-epitopes removed and raw CD4 neo-epitope content. (E) Patients separated on basis of CD8 neo-epitope content with "tolerated" CD8 neo-epitopes removed and CD4 neo-epitope content with "tolerated" CD4 neo-epitopes removed, using higher cutoff for CD4 tolerated epitope removal.

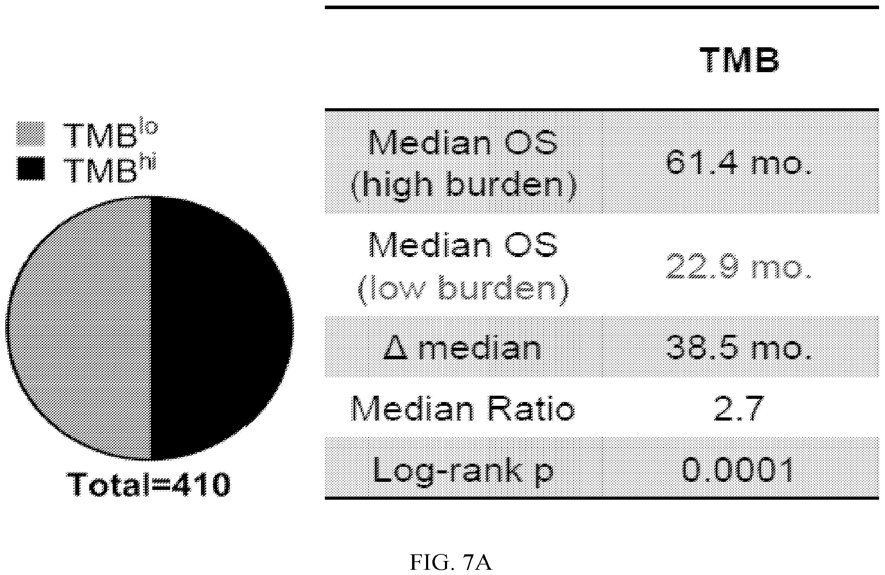

Figure 7A-7B. Patient stratification. (A) Patient TMB significantly stratifies short- and long-term survivors. (B) Evaluating CD8 neo-epitope burden with ANCER™ helps stratify bladder cancer patients. (C) Inclusion of CD4 neo-epitope burden helps ANCER™ further stratify cancer patients. (E) Patient stratification is further enhanced when evaluating the phenotype of CD4 neo-epitopes.

The following detailed description, given by way of example, but not intended to limit the invention solely to the specific embodiments described, may best be understood in conjunction with the accompanying drawings.

DETAILED DESCRIPTION OF THE INVENTION

Particular details of various embodiments of the invention are set forth to illustrate certain aspects and not to limit the scope of the invention.Unless defined otherwise, all technical and scientific terms used herein have the same meaning as commonly understood by one of ordinary skill in the art to which the presently- disclosed subject matter belongs.

While clinical studies have highlighted the potential of personalized cancer immunotherapy to effectively control the cancer of patients across cancer indications, recent studies showcase the difficulty of establishing robust CD8+ and CD4+ T cell responses against the patient's cancer. We hypothesized that poor cancer vaccine performance may be due in large part to the inadvertent inclusion of suppressive T cell neo-epitopes in neoantigen vaccines that may be recognized by, and thus activate, regulatory T cells (Tregs), and/or the inadvertent inclusion of other detrimental T cell-neo-epitopes in neoantigen-based vaccines that may be recognized by, and thus activate, other detrimental T cells (including T cells with potential host cross-reactivity that may lead to autoimmune responses, as well as anergic T cells). To test this hypothesis, we used the instantly-disclosed strategies, compositions, and methods for producing personalized neoplasia vaccines to identify and select neo-epitopes from the CT26 syngeneic mouse model. Distinctive features of the instantly-disclosed strategies, compositions, and methods for producing personalized neoplasia vaccines over other personalized vaccine pipelines are the ability to predict CD4+ and CD8+ T cell neo-epitopes and to identify, and subsequently remove, neo-epitopes that may be recognized by and activate regulatory T cells, and/or the inadvertent inclusion of other detrimental T cells (including T cells with potential host cross-reactivity that may lead to autoimmune responses, as well as anergic T cells).

In a first set of experiments, optimally selected CT26 neo-epitope vaccine candidates encoding CD4+ and CD8+ neo-epitopes were designed and ranked with the instantly-disclosed strategies and methods. Self-like, putative regulatory T cell epitopes and epitopes that may be recognized by, and thus activate, other detrimental T cells (including T cells with potential host cross-reactivity that may lead to autoimmune responses, as well as anergic T cells) were removed in this process.

To gain further insight into the role of Treg cells and components of neo-epitope vaccine candidates, a retrospective analysis was performed to identify correlations between cancer patient treatment outcomes and repertoires of neo-epitopes in the cancer patients. An initial analysis focused on Class II neo-epitopes and was extended to include Class I neo-epitopes.

Thus, the Class I and Class II neo-epitopes are now more precisely classified as to immunogenic activity. Class I and Class II neo-epitopes are advantageously ranked as immunogenic, tolerated, or tolerogenic. Further, a retrospective analysis of Class I and Class II neoepitopes in bladder cancer patients comparing proportions of immunogenic, tolerated, or tolerogenic neoepitopes with overall survival reveals distinctions between Class I and Class II that provide for more effective neoantigen vaccines.

Neo-epitopes may be recognized by regulatory T cells due to their high degree of similarity with self. Previously, co-administration of the CT26 self-like neo-epitopes (e.g. which were predicted to be recognized by and activate regulatory T cells) with our optimally designed neo- epitope vaccine in naïve Balb/c mice were shown to diminish IFNγ ELISpot responses by 5-fold compared to vaccination without the self-like neo-epitopes (p = 0.003). Thus, it was indicated that poor cancer vaccine performance may be due in large part to the inadvertent inclusion of suppressive T cell neo-epitopes in neo-epitope vaccines that may be recognized by, and thus activate, regulatory T cells, and/or the inadvertent inclusion of other detrimental T cell- neo-epitopes in neoantigen-based vaccines that may be recognized by, and thus activate, other detrimental T cells (including T cells with potential host cross-reactivity that may lead to autoimmune responses, as well as anergic T cells).

While it has been well known that regulatory T cells are present in tumors, these results suggest the possibility that tumor-derived neo-epitopes may be recruiting regulatory T cells to the tumor. More importantly, the inadvertent inclusion of regulatory T cell-driving neo- epitopes, and/or the inadvertent inclusion of other detrimental T cell-neo-epitopes that may be recognized by, and thus activate, other detrimental T cells (including T cells with potential host cross-reactivity that may lead to autoimmune responses, as well as anergic T cells), in vaccine formulations may hinder efforts to induce strong T cell-mediated tumor control while also leading to possible autoimmune responses. Screening of neoantigen sequences to identify and remove potential regulatory T cell inducing neo-epitopes and/or other detrimental T cell-neo- epitopes that may be recognized by, and thus activate, other detrimental T cells (including T cells with potential host cross-reactivity that may lead to autoimmune responses, as well as anergic T cells) (e.g., using specialized tools, including *in silico* screening tools) offers the possibility of enriching and designing new vaccines with higher quality candidates while minimizing costs and turnaround times.

We now show by classification of neoantigens and retrospective analysis of bladder cancer patients the advantages and improved effects of combinations of Class I and Class II neoantigens. Further, cross-reactive neoantigens that bind to both Class I and Class II MHC may have immunogenicity with respect to one while tolerated or tolerogenic with respect to the other. Our results allow improved discrimination of Class I and Class II neoantigens that cross-react. Thus, the invention provides a prognostic method for determining risk of death of a human subject with a neoplasia. In certain embodiments, the method of the invention comprises identification of neoantigens that are cross-reactive between Class I and Class II MHC.

The instant invention provides a prognostic method for example to assess risk of death, or to adjust treatment. More particularly, embodiments of the present disclosure relate to the identification of neoplasia-specific neoantigens to identify and design subject-specific neo-epitopes, further assessing the identified neo-epitopes encoded by said mutations to identify neo-epitopes that are known or determined (e.g. predicted) to engage regulatory T cells and/or other detrimental T cells (including T cells with potential host cross-reactivity and/or anergic T cells), and excluding such identified neo-epitopes that are known or determined (e.g. predicted) to engage regulatory T cells and/or other detrimental T cells (including T cells with potential host cross-reactivity and/or anergic T cells) from the subject- specific neo-epitopes that are to be used in personalized neoplasia vaccines. Further, the instant disclosure relates to a novel ranking system for determining the optimal peptides or polypeptides comprising subject-specific neo-epitopes that are to be used in personalized neoplasia vaccines..

*Definitions*

As used herein, the singular forms "a," "an," and "the" are intended to include the plural forms, including "at least one," unless the content clearly indicates otherwise. "Or" means "and/or." As used herein, the term "and/or" and "one or more" includes any and all combinations of the associated listed items.

As used herein, the term "about" is understood as within a range of normal tolerance in the art, for example within 2 standard deviations of the mean. About can be understood as within 50%, 45%, 40%, 35%, 30%, 25%, 20%, 15%, 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

As used herein, the term "antigen" refers to any substance that will elicit an immune response. In aspects, an antigen relates to any substance, preferably a peptide or protein, that reacts specifically with antibodies or T-lymphocytes (T cells). According to the present invention, the term "antigen" comprises any molecule which comprises at least one epitope. Preferably, an antigen is a molecule which, optionally after processing, induces an immune reaction, which is preferably specific for the antigen (including cells expressing the antigen). An antigen is preferably presented by a cell, preferably by an antigen presenting cell which includes a diseased cell, in particular a cancer cell, in the context of MHC molecules, which results in an immune reaction against the antigen. An antigen is preferably a product which corresponds to or is derived from a naturally occurring antigen. Such naturally occurring antigens include tumor antigens, e.g., a part of a tumor cell such as a protein or peptide expressed in a tumor cell which may be derived from the cytoplasm, the cell surface, or the cell nucleus, in particular those which primarily occur intracellularly or as surface antigens of tumor cells.

As used herein, the term "biological sample" as refers to any sample of tissue, cells, or secretions from an organism.

As used herein, the terms "comprises," "comprising," "containing" and "having" and the like can mean "includes," "including," and the like; "consisting essentially of" or "consists essentially" likewise is open-ended, allowing for the presence of more than that which is recited so long as basic or novel characteristics of that which is recited is not changed by the presence of more than that which is recited, but excludes prior art embodiments.

As used herein, the term "control" is meant a standard or reference condition.

As used herein, the term "disease" is meant any condition or disorder that damages or interferes with the normal function of a cell, tissue, or organ.

As used herein, the term "effective amount" is meant the amount required to ameliorate the symptoms of a disease (e.g., a neoplasia/tumor) relative to an untreated patient. The effective amount of active compound(s) for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount.

As used herein, "fragment" refers to a portion of a polypeptide or nucleic acid molecule. This portion contains, preferably, at least 5%, 10%, 20%, 30%, 40%, 50%, 60%, 70%, 80%, or 90% of the entire length of the reference nucleic acid molecule or polypeptide. A fragment may contain 5, 10, 20, 30, 40, 50, 60, 70, 80, 90, or 100, 200, 300, 400, 500, 600, 700, 800, 900, 1000 or more nucleotides or amino acids.

As used herein, the term "immune response" refers to the concerted action of lymphocytes, antigen presenting cells, phagocytic cells, granulocytes, and soluble macromolecules produced by the above cells or the liver (including antibodies, cytokines, and complement) that results in selective damage to, destruction of, or elimination from the human body of cancerous cells, metastatic tumor cells, malignant melanoma, invading pathogens, cells or tissues infected with pathogens, or, in cases of autoimmunity or pathological inflammation, normal human cells or tissues.

As used herein, the term "immune synapse" means the protein complex formed by the simultaneous engagement of a given T cell epitope to both a cell surface MHC complex and TCR.

As used herein, the term "isolated" means that the polynucleotide or polypeptide or fragment, variant, or derivative thereof has been essentially removed from other biological materials with which it is naturally associated, or essentially free from other biological materials derived, e.g., from a recombinant host cell that has been genetically engineered to express the polypeptide as presently disclosed.

As used herein, the terms "the major histocompatibility complex (MHC)", "MHC molecules", "MHC proteins" or "HLA proteins" are to be understood as meaning, in particular, proteins capable of binding peptides resulting from the proteolytic cleavage of protein antigens and representing potential T-cell epitopes, transporting them to the cell surface and presenting them there to specific cells, in particular cytotoxic T-lymphocytes or T-helper cells. The major histocompatibility complex in the genome comprises the genetic region whose gene products expressed on the cell surface are important for binding and presenting endogenous and/or foreign antigens and thus for regulating immunological processes. The major histocompatibility complex is classified into two gene groups coding for different proteins, namely molecules of MHC class I and molecules of MHC class II. The molecules of the two MHC classes are specialized for different antigen sources. The molecules of MHC class I present endogenously synthesized antigens, for example viral proteins and tumor antigens. The molecules of MHC class II present protein antigens originating from exogenous sources, for example bacterial products. The cellular biology and the expression patterns of the two MHC classes are adapted to these different roles. MHC molecules of class I consist of a heavy chain and a light chain and are capable of binding a peptide of about 8 to 11 amino acids, but usually 9 or 10 amino acids, if this peptide has suitable binding motifs, and presenting it to cytotoxic T-lymphocytes. The peptide bound by the MHC molecules of class I originates from an endogenous protein antigen. The heavy chain of the MHCmolecules of class I is preferably an HLA-A, HLA-B or HLA-C monomer, and the light chain is β-2-microglobulin. MHC molecules of class II consist of an α- chain and a β-chain and are capable of binding a peptide of about 15 to 24 amino acids if this peptide has suitable binding motifs, and presenting it to T-helper cells. The peptide bound by the MHC molecules of class II usually originates from an extracellular of exogenous protein antigen. The α-chain and the β-chain are in particular HLA-DR, HLA-DQ andHLA-DP monomers.

As used herein, the term "MHC Binding Motif" refers to a pattern of amino acids in a protein sequence that predicts binding to a particular MHC allele.

As used herein, the term "MHC Ligand" means a polypeptide capable of binding to one or more specific MHC alleles. The term "HLA ligand" is interchangeable with the term "MHC Ligand". Cells expressing MHC/Ligand complexes on their surface are referred to as "Antigen Presenting Cells" (APCs). Similarly, as used herein, the term "MHC binding peptide" relates to a peptide which binds to an MHC class I and/or an MHC class II molecule. In the case of MHC class I/peptide complexes, the binding peptides are typically 8-10 amino acids long although longer or shorter peptides may be effective. In the case of MHC class II/peptide complexes, the binding peptides are typically 10-25 amino acids long and are in particular 13-18 amino acids long, whereas longer and shorter peptides may also be effective.

As used herein, the term "epitope" refers to an antigenic determinant in a molecule such as an antigen, i.e., to a part in or fragment of the molecule that is recognized by the immune system, for example, that is recognized by a T cell, in particular when presented in the context of MHC molecules. An epitope of a protein such as a tumor antigen preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 9, 10, 11, 12, 13, 14, 15, 16, 17, 18, 19, 20, 21, 22, 23, 24, or 25 amino acids in length. It is particularly preferred that the epitope in the context of the present invention is a T cell epitope.

As used herein, the term "polypeptide" refers to a polymer of amino acids, and not to a specific length; thus, peptides, oligopeptides and proteins are included within the definition of a polypeptide. As used herein, a polypeptide is said to be "isolated" or "purified" when it is substantially free of cellular material when it is isolated from recombinant and non-recombinant cells, or free of chemical precursors or other chemicals when it is chemically synthesized. A polypeptide of the present disclosure, however, can be joined to, linked to, or inserted into another polypeptide (e.g., a heterologous polypeptide) with which it is not normally associated in a cell and still be "isolated" or "purified." When a polypeptide is recombinantly produced, it can also be substantially free of culture medium, for example, culture medium represents less than about 20%, less than about 10%, or less than about 5% of the volume of the polypeptide preparation.

As used herein, the term "neo-epitope" refers to a T cell epitope that is not present in a reference such as a normal non-cancerous or germline cell but is found in cancer cells. This includes, in particular, situations wherein in a normal non-cancerous or germline cell a corresponding epitope is found, however, due to one or more mutations in a cancer cell the sequence of the epitope is changed so as to result in the neo-epitope. This also includes situations wherein in a normal non-cancerous or germline cell no T cell epitope is found, however, due to one or more mutations in a cancer cell the sequence is changed so as to create a new neo-epitope. In aspects, a "neo-epitope" of the present disclosure may be encoded by a neoplasia-specific mutation that is unique to the neoplasia patient/subject (e.g., epitope that is specific to both the cancer cell and subject from which it is found), and my be referred to herein as a "subject-specific neo-epitope." In aspects, a "neo-epitope" of the present disclosure may be encoded by a neoplasia-specific mutation that is present in a neoplasia (e.g., cancer) cell in at least 1%, 2%, 3%, 4%, 5%, or more than 5% of subjects in a population of subjects suffering from the neoplasia (e.g., bladder cancer), and may be referred to herein as a "shared neo-epitope." In aspects, a "shared neo-epitope" may be present in two or more, three or more, four or more, five or more, etc. subjects in a population of subjects suffering from the neoplasia (e.g., bladder cancer).

As used herein, the terms "neoantigen" or "neo-antigenic" means a class of tumor antigens that arises from a neoplasia-specific mutation(s) which alters the amino acid sequence of genome encoded proteins. "Neoantigens" can include one or more neo-epitopes, including subject-specific or shared neo-epitopes. A "subject-specific neo-epitope" means a neoplasia- specific mutation that is unique to the neoplasia patient/subject (e.g., a mutation that is specific to both the cancer cell and subject from which it is found). A "shared neoplasia-specific mutation" means a neoplasia-specific mutation that is present in a neoplasia (e.g., cancer) cell in at least 1%, 2%, 3%, 4%, 5%, or more than 5% of subjects in a population of subjects suffering from the neoplasia, e.g., the specific type of neoplasia, such as bladder cancer. In aspects, a "shared neoplasia-specific mutation" means a neoplasia-specific mutation that is present in a neoplasia (e.g., cancer) cell in two or more, three or more, four or more, five or more, etc. subjects in a population of subjects suffering from the neoplasia, e.g., the specific type of neoplasia, such as bladder cancer.

As used herein, the term "neoplasia" refers to any disease that is caused by or results in the abnormal proliferation of cells, inappropriately low levels of apoptosis, or both. Neoplasia can be benign, pre-malignant, or malignant. Cancer is an example of a neoplasia. Non-limiting examples of cancer include leukemia (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (e.g., Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, nile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, elanoma, neuroblastoma, and retinoblastoma). Lymphoproliferative disorders are also considered to be proliferative diseases.

As used herein, the term "pharmaceutically acceptable" refers to approved or approvable by a regulatory agency of the Federal or a state government or listed in the U.S. Pharmacopeia or other generally recognized pharmacopeia for use in animals, including humans.

As used herein, the term "pharmaceutically acceptable excipient, carrier or diluent" or the like refer to an excipient, carrier or diluent that can be administered to a subject, together with an agent, and which does not destroy the pharmacological activity thereof and is nontoxic when administered in doses sufficient to deliver a therapeutic amount of the agent.

Ranges provided herein are understood to be shorthand for all of the values within the range. For example, a range of 1 to 25 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, or 25, as well as all intervening decimal values between the aforementioned integers such as, for example, 1.1, 1.2, 1.3, 1.4, 1.5, 1.6, 1.7, 1.8, and 1.9. With respect to sub-ranges, "nested sub-ranges" that extend from either end point of the range are specifically contemplated. For example, a nested sub-range of an exemplary range of 1 to 25 may comprise 1 to 5, 1 to 10, 1 to 15, and 1 to 20 in one direction, or 25 to 20, 25 to 15, 25 to 10, and 25 to 5 in the other direction.

As used herein, the term "regulatory T cell", "Treg" or the like, means a subpopulation of T cells that suppress immune effector function, including the suppression or down regulation of CD4+ and/or CD8+ effector T cell (Teff) induction, proliferation, and/or cytokine production, through a variety of different mechanisms including cell-cell contact and suppressive cytokine production. In aspects, CD4+ Tregs are characterized by the presence of certain cell surface markers including but not limited to CD4, CD25, and FoxP3. In aspects, upon activation, CD4+ regulatory T cells secrete immune suppressive cytokines and chemokines including but not limited to IL-10 and/or TGFβ. CD4+ Tregs may also exert immune suppressive effects through direct killing of target cells, characterized by the expression upon activation of effector molecules including but not limited to granzyme B and perforin. In aspects, CD8+ Tregs are characterized by the presence of certain cell surface markers including but not limited to CD8, CD25, and, upon activation, FoxP3. In aspects, upon activation, regulatory CD8+ T cells secrete immune suppressive cytokines and chemokines including but not limited to IFNy, IL-10, and/or TGFβ. In aspects, CD8+ Tregs may also exert immune suppressive effects through direct killing of target cells, characterized by the expression upon activation of effector molecules including but not limited to granzyme B and/orperforin.

As used herein, the term "regulatory T cell epitope" ("Tregitope") refers to a "T cell epitope" that causes a tolerogenic response (Weber CA et al., (2009), Adv Drug Deliv, 61(11):965-76) and is capable of binding to MHC molecules and engaging (i.e.interacting with and activating) circulating naturally occurring Tregs (in aspects, including natural Tregs and/or adaptive Tregs). In aspects, upon activation, CD4+ regulatory T cells secrete immune suppressive cytokines and chemokines including but not limited to IL-10 and/or TGFβ. CD4+ Tregs may also exert immune suppressive effects through direct killing of target cells, characterized by the expression upon activation of effector molecules including but not limited to granzyme B and perforin. In aspects, CD8+ Tregs are characterized by the presence of certain cell surface markers including but not limited to CD8, CD25, and, upon activation, FoxP3. In aspects, upon activation, regulatory CD8+ T cells secrete immune suppressive cytokines and chemokines including but not limited to IFNγ, IL-10, and/or TGFβ. In aspects, CD8+ Tregs may also exert immune suppressive effects through direct killing of target cells, characterized by the expression upon activation of effector molecules including but not limited to granzyme B and/or perforin.

As used herein, the term "T cell epitope" means an MHC ligand or protein determinant, 7 to 30 amino acids in length, and capable of specific binding to MHC molecules (e.g. human leukocyte antigen (HLA) molecules) and interacting with specific T cell receptors (TCRs). As used herein, in the context of a T cell epitope (e.g., a neo-epitope, Tregitope, etc.) that is known or determined (e.g. predicted) to engage a T cell (e.g., regulatory T cells and/or other detrimental T cells, such as T cells with potential host cross-reactivity and/or anergic T cells), the terms "engage", "engagement" or the like means that when bound to a MHC molecule (e.g. human leukocyte antigen (HLA) molecules), the T cell epitope is capable of interacting with the TCR of the T cell and activating the T cell (which in the case of an anergic T cell, includes functional inactivation). Generally, T cell epitopes are linear and do not express specific three-dimensional characteristics. T cell epitopes are not affected by the presence of denaturing solvents. The ability to interact with T cell epitopes can be predicted by in silico methods (De Groot AS et al., (1997), AIDS Res Hum Retroviruses, 13(7):539-41; Schafer JR et al., (1998), Vaccine, 16(19):1880-4; De Groot AS et al., (2001), Vaccine, 19(31):4385-95; De Groot AR et al.,(2003), Vaccine, 21(27-30):4486-504.

As used herein, the term "T Cell Receptor" or "TCR" refers to a protein complex expressed by T cells that is capable of engaging a specific repertoire of MHC/Ligand complexes as presented on the surface of APCs.

As used herein, the term "vaccine" refers to a pharmaceutical preparation (pharmaceutical composition) or product that upon administration induces an immune response, in particular a cellular immune response, which recognizes and attacks a pathogen or a diseased cell such as a neoplasia (e.g., a cancer cell). A vaccine may be used for the prevention or treatment of a disease. Accordingly, vaccines are medicaments which include antigens and are used in humans or animals for generating specific defense and protective substance by vaccination. The term "personalized neoplasia vaccine" or the like concerns a particular neoplasia patient and means that a neoplasia (e.g. cancer) vaccine is adapted to the needs or special circumstances of an individual neoplasia patient.

*HLA/MHC binding*

In aspects of the invention, neoantigens and self antigens are evaluated for immune presentation. The major histocompatibility complex (MHC) is a large locus on vertebrate's DNA containing a set of closely linked polymorphic genes that code for cell surface proteins essential for the adaptive immune system. The human MHC is also called the HLA (human leukocyte antigen) HLA. Neoantigens may be individually evaluated for binding *in vitro* or to panels of mono-allelic cells. MHC binding can be determined by identifying peptides associated with MHC by liquid chromatography-tandem mass spectrometry (LC MS/MS) which allows identification of a large number of sequences that have undergone the entire peptide presentation pathway. (Creech et al., 2018, The Role of Mass Spectrometry and Proteogenomics in the Advancement of HLA Epitope Prediction. Proteomics 18(12):e 1700259). The LC MS/MS dataset published by Abelin et al. represents over 26,000 Class I peptides eluted across 16 HLA-A or HLA-B monoallelic cell lines. (Abelin et al., 2017, Mass Spectrometry Profiling of HLA-Associated Peptidomes in Mono-allelic Cells Enables More Accurate Epitope Prediction. Immunity 46(2):315-326). Such eluted peptides databases are useful to identify peptides that bind to MHC, to evaluate in silico predictions, to screen or rank peptides identified by in silico predictions, and to improve in silico prediction methods, binding to HLA/MHC may be evaluated.

Several public *in silico* tools have been developed to predict MHC binding. NetMHCpan-2.0 is an *in silico* tool that generates quantitative predictions of the affinity of any peptide-MHC class I interaction. NetMHCpan-2.0 was trained on a large set of quantitative MHC binding data, covering human HLA-A and HLA-B. (Hoof I, Peters B, Sidney J, et al. NetMHCpan, a method for MHC class I binding prediction beyond humans. Immunogenetics. 2009;61:1-13). NetMHCpan-3.0 captures differences in the length profile of binders to different MHC molecules leading to improved accuracy for ligand identification due to more uniform sampling of the MHC. (Nielsen M, Andreatta M. NetMHCpan-3.0; improved prediction of binding to MHC class I molecules integrating information from multiple receptor and peptide length datasets. Genome Med. 2016;8:33). NetMHCpan-4.0 is further iteration of the in-silico tool and is trained on binding affinity and eluted ligand data. (Jurtz V, Paul S, Andreatta M, et al. NetMHCpan-4.0: Improved Peptide-MHC Class I Interaction Predictions Integrating Eluted Ligand and Peptide Binding Affinity Data. J. Immunol. 2017;199:3360 LP-3368).

*T cells*

T-cells are the most important effector cell types that mediate various immune responses and therefore, have been preferred targets for immunomodulation. T cells can be broadly classified as T-effector (Teff) cells and T-regulatory (Treg) cells based on the paradoxical nature of their function. (Kumar, P. et al., 2018, A Comprehensive Review on the Role of Co-signaling Receptors and Treg Homeostasis in Autoimmunity and Tumor Immunity. J. Autoimmun. 95:77). Foxp3 is the lineage-specific transcription factor exclusively expressed in Tregs and not in Teff cells (Josefowicz, S.Z. et al., 2012, Regulatory T cells: mechanisms of differentiation and function.Annu Rev Immunol 30:531). The major discriminating factor between Treg and Teff cells is their affinity for self-antigens. (Jordan, M.S. et al., 2001, Thymic selection of CD4(+)CD25(+) regulatory T cells induced by an agonist self-peptide. Nat Immunol 2:468). During thymic selection, T-cell clones expressing high-affinity T-cell receptors (TCRs) for self-antigens are either deleted by negative selection or rendered anergic. However, thymic negative selection is imperfect in that self-reactive T-cell clones often escape negative selection, migrate to the periphery and contribute to autoimmunity. However, T-cells expressing TCRs with an intermediate affinity for self-antigens gain Foxp3 expression and become Tregs are positively selected and migrate to the periphery where they help maintain peripheral self-tolerance (Jordan, 2001).

*T cell receptor (TCR) repertoire*

The T cell receptor repertoire is diverse and differs according to T cell function. Bentzen describes a method for determining interactions that govern TCR recognition of peptide-major histocompatibility complex (pMHC) by measuring relative affinities of TCRs to libraries of barcoded peptide-MHC variants and develop a TCR fingerprint. (Bentzen et al., 2018, T cell receptor fingerprinting enables in-depth characterization of the interactions governing recognition of peptide-MHC complexes. Nature Biotechnology 36:1191). Jurtz et al. developed a method to predict T cell receptor fingerprinting enables in-depth characterization of the interactions governing recognition of peptide-MHC complexes (Jurtz et al., 2018, NetTCR: sequence-based prediction of TCR binding to peptide-MHC complexes using convolutional neural networks. doi:10.1101/433706). In another example, a machine learning approach, called NetTCR, has been develop and was trained on 8,920 TCRβ CDR3 sequences and 91 cognate peptide targets obtained from IEDB and from immune assay data. (Klinger et al., 2015, Multiplex identification of antigen-specific T cell receptors using a combination of immune assays and immune receptor sequencing. PloS One 10, e0141561). Ogishi and Yotsuyanagi have taken advantage of immunodominant epitopes, which would be expected to exhibit some prominent features that make them especially prone to be recognized by T cells to model repertoire-wide TCR-epitope contact potential (Ogishi and Yotsuyanagi, 2019, Quantitative prediction of the landscape of T cell epitope immunogenicity in sequence space. Front. Immunol. 10, 827).

*Identifying Neoplasia-Specific Mutations*

In aspects, the step of identifying neoplasia-specific mutations (e.g., cancer-specific mutations) comprises sequencing genomic DNA and/or RNA of a neoplasia specimen (e.g., a neoplasia specimen of the patient). In aspects, a neoplasia specimen relates to any sample, such as a bodily sample derived from a patient, containing or being expected of containing neoplasia cells (e.g. tumor or cancer cells). In aspects, the bodily sample may be any tissue sample such as blood, a tissue sample obtained from a neoplasia sample (e.g., a primary tumor or from tumor metastases/circulating tumor cells), or any other sample containing neoplasia cells (e.g., tumor or cancer cells).

In aspects, the step of identifying of neoplasia-specific mutations comprises comparing the sequence information obtained from the neoplasia specimen with a reference sample, such as sequence information obtained from sequencing nucleic acid (e.g., such as DNA or RNA) of normal, non-neoplasia cells (e.g., non-cancerous cells) cells, such as somatic or germline tissue/cells. In aspects, a reference sample may be obtained from the neoplasia patient or a different individual. In aspects, a reference sample may be any tissue sample such as blood or a sample from a non-neoplasia tissue. In aspects, normal genomic germline DNA may be obtained from peripheral blood mononuclear cells (PBMCs).

In aspects, neoplasia-specific mutations may include all neoplasia-specific (e.g. cancer-specific) mutations present in one or more neoplasia cells (e.g., cancer or tumor cells) of a patient, or it may refer to only a portion of the neoplasia-specific mutations present in one or more neoplasia cells of a patient. Accordingly, the present disclosure may involve the identification of all neoplasia-specific mutations present in one or more neoplasia cells of a patient, or it may involve the identification of only a portion of the neoplasia-specific mutations present in one or more neoplasia cells of a patient. In aspects, the methods of identifying subject-specific neo-epitopes for a personalized neoplasia vaccine of the present disclosure provide for the identification of a number of neoplasia-specific mutations which will provide a sufficient number of neo-epitopes to be included in the instantly-disclosed strategies, methods, and compositions.

In aspects, the mutations are neoplasia-specific mutations (e.g., somatic mutations) in a neoplasia specimen (e.g. a tumor specimen) of a neoplasia patient (e.g. a cancer patient), which may be determined by identifying sequence differences between the genome, exome and/or transcriptome of a neoplasia specimen and the genome, exome and/or transcriptome of a non-neoplasia specimen. In aspects, neoplasia-specific mutations, including somatic mutations, are determined in the genome, preferably the entire genome, of a neoplasia specimen. As such, the instant disclosure may include identifying all or a portion of neoplasia-specific mutations of the genome, preferably the entire genome, of one or more neoplasia cells. In aspects, neoplasia-specific mutations, including somatic mutations, are determined in the exome, preferably the entire exome, of a neoplasia specimen. As such, the instant disclosure may include identifying all or a portion of neoplasia-specific mutations of the exome, preferably the entire exome of one or more neoplasia cells. In aspects, optionally, neoplasia-specific mutations, including somatic mutations, are determined in the transcriptome, preferably the entire transcriptome, of a neoplasia specimen. As such, the instant disclosure may include identifying all or a portion of the neoplasia-specific transcriptome, preferably the entire transcriptome, of one or more neoplasia cells.

In aspects, any suitable sequencing method as is known in the art can be used according to the instant disclosure for determining neoplasia-specific mutations is step (i), including but not limited to "conventional" sequencing methodology and Next Generation Sequencing (NGS) technologies. "Next Generation Sequencing" or "NGS" refers to all high throughput sequencing technologies which, in contrast to the "conventional" sequencing methodology known as Sanger chemistry, read nucleic acid templates randomly in parallel along the entire genome by breaking the entire genome into small pieces. As is known in the art, such NGS technologies (also known as massively parallel sequencing technologies) are able to deliver nucleic acid sequence information of a whole genome, exome, transcriptome (all transcribed sequences of a genome) or methylome (all methylated sequences of a genome) in very short time periods, e.g. within 1-2 weeks, preferably within 1-7 days or most preferably within less than 24 hours and allow, in principle, single cell sequencing approaches. Multiple NGS platforms which are commercially available, or which are known in the art can be used. Non-limiting examples of such NGS technologies/platforms include but are not limited to sequencing-by-ligation approaches, ion semiconductor sequencing, pyrosequencing, single-molecule sequencing technologies, nano-technologies for single-molecule sequencing, and electron microscopy based technologies for single-molecule sequencing. Further, in aspects, "Third Generation Sequencing" methods, as are known in the art, could be used for determining neoplasia-specific mutations. In aspects, neoplasia-specific mutations may be determined by direct protein sequencing techniques, as are known in the art. Further, in aspects, neoplasia-specific mutations can be determined by using MHC multimers, as is known in the art.

As such, in aspects of the method of identifying subject-specific neo-epitopes for a personalized neoplasia vaccine, the step of identifying neoplasia-specific mutations includes identifying sequence differences between the full or partial genome, exome, and/or transcriptome of a neoplasia specimen from the subject diagnosed as having a neoplasia and a non-neoplasia specimen. In aspects, a non-neoplasia specimen is derived from the subject diagnosed as having a neoplasia. In further aspects, identifying neoplasia-specific mutations or identifying sequence differences comprises Next Generation Sequencing (NGS). In aspects, the step of identifying neoplasia-specific mutations comprises selecting from the neoplasia a plurality of nucleic acid sequences, each comprising mutations, which are not present in a non-neoplasia sample. In aspects, identifying neoplasia-specific mutations comprises sequencing genomic DNA and/or RNA of the neoplasia specimen.

In aspects of the method of identifying subject-specific neo-epitopes for a personalized neoplasia vaccine, the neoplasia-specific mutations are neoplasia-specific somatic mutations. In aspects, the neoplasia-specific mutations are single nucleotide variations (SNVs), insertions and deletions (which can generate both in-frame and frameshift mutations), and other large-scale rearrangements such as but not limited to chromosomal inversions, duplications, insertions, deletions, or translocations. In aspects, neoplasia specific mutations, including SNVs, insertions, and deletions, are non-synonymous mutations. In aspects, neoplasia-specific mutations, including SNVs, insertions and deletions (which can be non-synonymous mutations), and other large-scale rearrangements, are mutations of proteins encoded in the neoplasia specimen of the subject diagnosed as having a neoplasia. In aspects, neoplasia specific mutations, including SNVs, are non-synonymous mutations. In aspects, neoplasia-specific mutations, including SNVs (which can be non-synonymous mutations), indels, and frameshifts, are mutations of proteins encoded in the neoplasia specimen of the subject diagnosed as having a neoplasia.

*Identifying Subject-Specific Neo-Epitopes*

In aspects of the method of identifying subject-specific neo-epitopes for a personalized neoplasia vaccine, the step of assessing the neoplasia-specific mutations to identify known or determined (e.g. predicted) neo-epitopes encoded by said mutations comprises *in silico* testing. In aspects, *in silico* testing includes using validated algorithms (e.g., but not limited to, EpiMatrix®, netMHCpan, NetMHC, netMHCcons, SYFPEITHI, HLA_BIND) to predict which neoplasia-specific mutations create neo-epitopes, particularly neo-epitopes that can bind to the MHC allotypes of the patient. For example, using validated algorithms, bioinformatic analysis of the identified neoplasia-specific mutations and their respective cognate native antigens can be performed to predict which identified neoplasia-specific mutations create neo-epitopes that can bind to the patient's MHC allotype, and in aspects to predict which identified neoplasia-specific mutations create neo-epitopes that could bind to the patient's MHC allotype more effectively than the cognate native antigen. Thus, in aspects, assessing identified neoplasia-specific mutations from a neoplasia specimen of a subject diagnosed as having a neoplasia identified to identify known or determined (e.g. predicted) neo-epitopes encoded by said mutations for use in the personalized neoplasia vaccine, wherein said neo-epitopes are known or determined (e.g. predicted) to bind to a MHC protein of the subject comprises the use of well-validated algorithms.

In aspects, said *in silico* testing to identify known or determined (e.g. predicted) neo-epitopes encoded by said mutations comprises using the EpiMatrix® algorithm. EpiMatrix® is a proprietary computer algorithm developed by EpiVax, which is used to screen protein sequences for the presence of putative T cell epitopes. The algorithm uses matrices for prediction of 9- and 10-mer peptides binding to MHC molecules. Each matrix is based on position-specific coefficients related to amino acid binding affinities that are elucidated by a method similar to, but not identical to, the pocket profile method (Sturniolo, T. et al., Nat. Biotechnol., 17:555-561, 1999). Input sequences are parsed into overlapping 9-mer or 10-mer frames where each frame overlaps the last by 8 or 9 amino acids, respectively. Thus, in aspects, input sequences of the mutated peptide from step (a) and the non-mutated peptide from step (b) are parsed into overlapping 9-mer or 10-mer frames where each frame overlaps the last by 8 or 9 amino acids. Each of the resulting frames form the mutated peptide from step (a) and the non-mutated peptide from step (b) are then scored for predicted binding affinity with respect to MHC class I alleles (e.g., but not limited to, HLA-A and HLA-B alleles) and MHC class II alleles (e.g., but not limited to HLA-DRB1 alleles). EpiMatrix® raw scores are normalized against the scores of a large sample of randomly generated peptides (e.g., but not limited to 10,000 randomly generated peptides). The resulting "Z" scores are normally distributed and directly comparable across alleles. The resulting "Z" score is reported. In aspects, any 9-mer or 10-mer peptide with an allele-specific EpiMatrix® Z-score that is theoretically the top 5% of any given sample (e.g., having an EpiMatrix® Z-score above 1.64), is considered a putative T cell epitope. In aspects, EpiMatrix® identifies the mutated peptide as a neo-epitope when: 1) the mutated peptide has a determined binding score in the top 5 percentile of the expected distribution and the non- mutated peptide has a determined binding score below the top 10 percentile of the expected distribution; or 2) the mutated peptide has a determined binding score in the top 5 percentile of the expected distribution, the non-mutated peptide has a determined binding score in the top 10 percentile of the expected distribution, and there is at least one mismatched TCR facing amino acid between the mutated peptide the non-mutated peptide. Previous studies have also demonstrated that EpiMatrix® accurately predicts published MHC ligands and T cell epitopes.

In aspects of the prognostic methods and methods of identifying subject-specific neo-epitopes for a personalized neoplasia vaccine, assessing the neoplasia-specific mutations to identify known or determined (e.g. predicted) neo-epitopes encoded by said mutations includes one or more of the following steps:

a) determining a binding score for a mutated peptide to one or more MHC molecules, wherein said mutated peptide is encoded by at least one of said neoplasia-specific mutations;
b) determining a binding score for a non-mutated peptide to the one or more MHC molecules, wherein the non-mutated peptide is identical to the mutated peptide except for the encoded at least one of said neoplasia-specific mutations;
c) determining the percentile rank of the binding scores of both the mutated peptide of step (a) and the non-mutated peptide of step (b) as compared to an expected distribution of binding scores for sufficiently large enough set (e.g., at least 10,000) randomly generated peptides using naturally observed amino acid frequencies;
d) determining the TCR facing amino acid residues of said mutated peptide and said non-mutated peptide; and
e) identifying the mutated peptide as a neo-epitope when: 1) the mutated peptide has a determined binding score in the top 5 percentile of the expected distribution and the non- mutated peptide has a determined binding score below the top 10 percentile of the expected distribution; or 2) the mutated peptide has a determined binding score in the top 5 percentile of the expected distribution, the non-mutated peptide has a determined binding score in the top 10 percentile of the expected distribution, and there is at least one mismatched TCR facing amino acid between the mutated peptide the non-mutated peptide. In further aspects, the one or more MHC molecules are MHC class I molecules and/or MHC class II molecules.

In aspects of the invention, the step of assessing the neoplasia-specific mutations to identify known or determined (e.g. predicted) neo-epitopes encoded by said mutations comprises *in vitro* testing. More particularly, determining the binding score of both the mutated peptide of step (a) and the non-mutated peptide of step (b) may comprise *in vitro* MHC binding assays (as are known in the art) to determine a binding score for the mutated peptide to one or more MHC molecules and to determine a binding score for the non-mutated peptide to the one or more MHC molecules. In aspects of the *in vitro* MHC binding assays, and similar to an *in silico* analysis, input sequences are parsed into overlapping 9-mer or 10-mer frames where each frame overlaps the last by 8 or 9 amino acids, respectively. Thus, in aspects of the *in vitro* MHC binding assays, input sequences of the mutated peptide from step (a) and the non-mutated peptide from step (b) are parsed into overlapping 9-mer or 10-mer frames where each frame overlaps the last by 8 or 9 amino acids. Each of the resulting frames from the mutated peptide from step (a) and the non-mutated peptide from step (b) are then scored for binding affinity with respect to MHC class I alleles (e.g., but not limited to, HLA-A and HLA-B alleles) in *in vitro* binding assays, with such binding assays as are known in the art. In the case of testing for epitopes that bind MHC class II alleles (e.g., but not limited to HLA-DRB 1 alleles) in *in vitro* binding assays, input sequences are parsed into overlapping 15-mer or 20-mer frames where each frame overlaps the last by 5 or 10 amino acids, respectively. Thus, in aspects, input sequences of the mutated peptide from step (a) and the non-mutated peptide from step (b) are parsed into overlapping 15-mer or 20-mer frames where each frame overlaps the last by 5 or 10 amino acids. Each of the resulting frames from the mutated peptide from step (a) and the non-mutated peptide from step (b) are then scored for binding affinity with respect to MHC class II alleles (e.g., but not limited to HLA-DRB 1 alleles) in *in vitro* binding assays, with such binding assays as are known in the art.

In aspects of the invention, the step of determining the percentile rank of the binding scores of both the mutated peptide of step (a) and the non-mutated peptide of step (b) as compared to an expected distribution of binding scores for a sufficiently large enough set (e.g., at least 10,000) randomly generated peptides using naturally observed amino acid frequencies, the raw binding scores, whether determined by *in silico* methods or *in vitro* methods, are adjusted to

EP 3 963 335 B1

fit a normal, or Z-distribution. Raw binding scores are normalized based on the average ($\mu$) binding score and standard deviation ($\sigma$) of a set of a large number (e.g., 10,000) random 9- or 10-mer amino acid sequences, following the naturally observed amino acid frequencies from UniProtKB/Swiss-Prot, as follows:

$$\text{Normalized binding score} = \frac{Raw\ binding\ score - \mu}{\sigma}$$

Normalized binding scores, which may be referred to as binding scores or likelihood of binding, within the top 5% of this normal distribution are defined as "hits"; which are potentially immunogenic and worthy of further consideration. These peptides have a significant chance of binding to MHC molecules with moderate to high affinity and, therefore, have a significant chance of being presented on the surface of both professional antigen presenting cells (APC) such as dendritic cells or macrophages, as well as non-professional APC, where they may be interrogated and potentially bound by passing CD8+ and CD4+ T cells.

In aspects, the mutated peptide and non-mutated peptide are both 9 amino acids in length or the mutated peptide and non-mutated peptide are both 10 amino acids in length. In aspects, the step of determining the TCR facing amino acid residues of said mutated peptide and said non-mutated peptide the TCR facing amino acid residues for a 9-mer mutated peptide and a 9-mer non-mutated peptide that bind to a MHC class II molecule comprises identifying the amino acid residues which are at position 2, 3, 5, 7, and 8 of the mutated and non-mutated peptide as counted from the amino terminal. In aspects, the step of determining the TCR facing amino acid residues of said mutated peptide and said non-mutated peptide for a 9-mer mutated peptide and a 9-mer non-mutated peptide that bind to a MHC class I molecule comprises identifying the amino acid residues which are at position 4, 5, 6, 7, and 8 of the mutated and non-mutated peptide as counted from the amino terminal. In aspects, the step of determining the TCR facing amino acid residues of said mutated peptide and said non-mutated peptide for a 10-mer mutated peptide and 10-mer non-mutated peptide that bind to a MHC class I molecule comprises identifying the amino acid residues which are at position 4, 5, 6, 7, 8, and 9 of the mutated and non-mutated peptide as counted from the amino terminal. In aspects, the step of determining the TCR facing amino acid residues of said mutated peptide and said non-mutated peptide the TCR facing amino acid residues for a 9-mer mutated peptide and a 9-mer non-mutated peptide that bind to a MHC class II molecule comprises identifying the amino acid residues which are at any combination of residues at positions 2, 3, 5, 7, and 8 (e.g., but not limited to, positions 3, 5, 7 and 8; positions 2, 5, 7, and 8; positions 2, 3, 5, and 7, etc. of the mutated and non-mutated peptide as counted from the amino terminal). In aspects, the step of determining the TCR facing amino acid residues of said mutated peptide and said non-mutated peptide for a 9-mer mutated peptide and a 9-mer non-mutated peptide that bind to a MHC class I molecule comprises identifying the amino acid residues which are at positions 4, 5, 6, 7, and 8; 1, 4, 5, 6, 7 and 8; or 1, 3, 4, 5, 6, 7, and 8 of the mutated and non-mutated peptide as counted from the amino terminal. In aspects, the step of determining the TCR facing amino acid residues of said mutated peptide and said non-mutated peptide for a 10-mer mutated peptide and 10-mer non-mutated peptide that bind to a MHC class I molecule comprises identifying the amino acid residues which are at any combination of residues at positions 1, 3, 4, 5, 6, 7, 8, and 9 of the mutated and non-mutated peptide as counted from the amino terminal.

In aspects of the method of identifying subject-specific neo-epitopes for a personalized neoplasia vaccine, particularly assessing the neoplasia-specific mutations to identify known or determined (e.g. predicted) neo-epitopes, the identified neo-epitopes may be optionally further confirmed by experimental validation for peptide-MHC binding, activation of CD8+ and/or CD4+ T cells, and/or by confirmation of gene expression at the RNA level. Such experimental validation may comprise *in vitro* and/or *in vivo* techniques, as are known in the art.

*Identification and Removal of Neo-Epitopes that are Known or Determined (e.g. Predicted) to Engage Regulatory T cells and/or Other Detrimental T cells*

As previously described, the instantly-disclosed data suggest the possibility that tumor-derived neo-epitopes may be recruiting regulatory T cells and/or other detrimental T cells (including T cells with potential host cross-reactivity and/or anergic T cells) to the tumor. As such, the inadvertent inclusion of regulatory T cell-driving neo-epitopes and/or other detrimental T cell-driving neo-epitopes (including T cells with potential host cross-reactivity and/or anergic T cells) in vaccine formulations may hinder efforts to induce strong T cell-mediated tumor control. Screening of neoantigen sequences to identify and remove potential regulatory T cell inducing neo-epitopes and/or other detrimental T cell inducing neo-epitopes (e.g., using specialized tools, including *in silico* screening tools) may be critical to designing new vaccines with higher quality candidates.

As such, in aspects of the method of identifying subject-specific neo-epitopes for a personalized neoplasia vaccine, the step of assessing the identified neo-epitopes encoded by said mutations to identify neo-epitopes that are

17

known or determined (e.g. predicted) to engage regulatory T cells and/or other detrimental T cells (including T cells with potential host cross-reactivity and/or anergic T cells) comprises determining whether said identified neo-epitopes encoded by said mutations share TCR contacts with proteins derived from either the human proteome or the human microbiome, wherein said identified neo-epitopes encoded by said mutations that are determined to share TCR contacts with proteins derived from either the human proteome or the human microbiome are identified as neo-epitopes that are known or determined (e.g. predicted) to engage regulatory T cells and/or other detrimental T cells (including T cells with potential host cross-reactivity and/or anergic T cells). In aspects, TCR contacts for a 9-mer identified neo-epitope that bind to a MHC class II molecule are at position 2, 3, 5, 7, and 8 of the identified neo-epitope as counted from the amino terminal, wherein the TCR contacts for a 9-mer identified neo-epitope that binds to a MHC class I molecule are at position 4, 5, 6, 7, and 8 of the identified neo-epitope as counted from the amino terminal, and wherein the TCR contacts for a 10-mer identified neo-epitope that bind to a MHC class I molecule are at position 4, 5, 6, 7, 8, and 9 of the identified neo-epitope as counted from the amino terminal. In aspects, the step of determining the TCR facing amino acid residues of said mutated peptide and said non-mutated peptide the TCR facing amino acid residues for a 9-mer mutated peptide and a 9-mer non-mutated peptide that bind to a MHC class II molecule comprises identifying the amino acid residues which are at any combination of residues at positions 2, 3, 5, 7, and 8 (e.g., but not limited to, positions 3, 5, 7 and 8; positions 2, 5, 7, and 8; positions 2, 3, 5, and 7, etc. of the mutated and non-mutated peptide as counted from the amino terminal). In aspects, the step of determining the TCR facing amino acid residues of said mutated peptide and said non-mutated peptide for a 9-mer mutated peptide and a 9-mer non- mutated peptide that bind to a MHC class I molecule comprises identifying the amino acid residues which are at positions 4, 5, 6, 7, and 8; 1, 4, 5, 6, 7 and 8; or 1, 3, 4, 5, 6, 7, and 8 of the mutated and non-mutated peptide as counted from the amino terminal. In aspects, the step of determining the TCR facing amino acid residues of said mutated peptide and said non-mutated peptide for a 10-mer mutated peptide and 10-mer non-mutated peptide that bind to a MHC class I molecule comprises identifying the amino acid residues which are at any combination of residues at positions 1, 3, 4, 5, 6, 7, 8, and 9 of the mutated and non-mutated peptide as counted from the amino terminal.

In aspects, the step of assessing the identified neo-epitopes encoded by said mutations to identify neo-epitopes that are known or determined (e.g. predicted) to engage regulatory T cells comprises conducting a homology screen on each identified neo-epitope or epitope sequence presenting a high likelihood of binding to MHC in order to characterize the degree of similarity with self of each of the encoded MHC class I- and MHC class II-restricted identified neo- epitopes and their corresponding non-mutated epitopes. MHC class I or MHC class II neo- epitopes and MHC class I or MHC class II corresponding non-mutated epitopes with two or more (and in further aspects, three or more) cross-reactive matches in the reference proteome are categorized as exhibiting a high degree of similarity with self and are considered to have a higher likelihood of being tolerated or to engage regulatory T cells and/or other detrimental T cells (including T cells with potential host cross-reactivity and/or anergic T cells).

In aspects, a homology screen is used remove epitopes containing combinations of TCR- facing residues that are commonly found in a reference proteome. In aspects, a homology screen comprises analysis of all the predicted epitopes contained within a given protein sequence and dividing each predicted epitope into its constituent amino acid content of both the MHC-binding agretope and the TCR-binding epitope. In aspects, the TCR-binding epitope (which canbe referred to as TCR binding residues, TCR facing epitope, TCR facing residues, or TCR contacts) for a 9-mer identified neo-epitope or epitope that bind to a MHC class II molecule are at position 2, 3, 5, 7, and 8 of the identified neo-epitope, while the MHC-binding agretope (which can be referred to as MHC contacts, MHC facing residues, MHC-binding residues, or MHC-binding face) for a 9-mer identified neo-epitope or epitope that bind to a MHC class II molecule are at position 1, 4, 6, and 9, both as counted from the amino terminal. In aspects, the TCR binding epitope for a 9-mer identified neo-epitope that binds to a MHC class I molecule are at position 4, 5, 6, 7, and 8 of the identified neo-epitope or epitope, while the MHC binding agretope for a 9-mer identified neo-epitope or epitope that bind to a MHC class I molecule are at position 1, 2, 3, and 9, both as counted from the amino terminal. In aspects, the TCR binding epitope for a 10-mer identified neo-epitope or epitope that bind to a MHC class I molecule are at position 4, 5, 6, 7, 8, and 9 of the identified neo-epitope, while the MHC binding agretope for a 10-mer identified neo-epitope or epitope that bind to a MHC class I molecule are at position 1, 2, 3, 9, and 10, both as counted from the amino terminal. In aspects, the TCR-binding epitope for a 9-mer identified neo-epitope or epitope that bind to a MHC class II molecule are at any combination of residues at positions 2, 3, 5, 7, and 8 (e.g., but not limited to, positions 3, 5, 7 and 8; positions 2, 5, 7, and 8; positions 2, 3, 5, and 7, etc.) of the identified neo-epitope or epitope, while the MHC binding agretope for a 9-mer identified neo-epitope or epitope is the complementary face to the TCR facing residues, both as counted from the amino terminal. In aspects, the TCR binding epitope for 9-mer identified neo-epitope or epitope that bind to a MHC class I molecule are at positions 4, 5, 6, 7, and 8; 1, 4, 5, 6, 7 and 8; or 1, 3, 4, 5, 6, 7, and 8 of the identified neo-epitope or epitope, while the MHC binding agretope for a 9-mer identified neo-epitope or epitope is the complementary face to the TCR facing residues, both as counted from the amino terminal. In aspects, the TCR-binding epitope for a 10-mer identified neo- epitope or epitope that bind to a MHC class I molecule are at any combination of residues at positions 1, 3, 4, 5, 6, 7, 8, and 9 of the identified neo-epitope or epitope, while the MHC binding agretope for a 10-mer identified neo-epitope or epitope is the complementary face to the TCR facing residues, both as counted from the amino terminal.

Each sequence is then screened against a database of proteins (e.g., a database of human proteins derived from the UniProt database (UniProt Proteome ID UP000005640, Reviewed/Swiss-Prot set)). Cross-conserved epitopes, or peptides derived from the reference proteome with a compatible MHC binding agretope (i.e. the agretopes of both the input (mutated) peptide and its reference non-mutated counterpart are predicted to bind to the same MHC allele) and exactly the same TCR facing epitope, are returned. The Homology Score of an epitope corresponds to the number of matching cross-conserved MHC binding peptides within the reference proteome. In other words, the Homology Score $H_e$ of an epitope $e$ is calculated as follows:

$$H_e = |X_e|$$

where:

$X_e$ corresponds to the set of MHC binding peptides derived from the reference proteome that are restricted to the same MHC class I or MHC class II as epitope $e$ and presenting a TCR facing epitope identical to the epitope $e$.

By extension, the Homology Score of a given peptide or protein corresponds to the average Homology Score of each individual epitope contained with the peptide or protein. In other words, the Homology Score $H_p$ of a peptide $p$ is calculated as follows:

$$H_p = \frac{\sum_{e \in E} H_e}{|E|}$$

where:

- $E$ corresponds to the set of MHC class I- or MHC class II-restricted epitopes within peptide p;
- $H_e$ corresponds to the Homology Score of epitope e as defined above.

In aspects, an analysis procedure is then run on each mutated sequence to determine if a substring within the amino acid sequence can be found, such that:

- at least one MHC class I- or MHC class II-restricted epitope is encoded in the substring, and;
- all MHC class I- or MHC class II-restricted neo-epitopes encoded in the substring have no more than two cross-reactive matches in the reference proteome, and;
- all MHC class I- or MHC class II-restricted epitopes encoded in the substring have no more than two cross-reactive matches in the referenceproteome.

This analysis procedure has the effect of removing amino acid substrings containing putative epitopes that engage regulatory T cells other detrimental T cells (including T cells with potential host cross-reactivity and/or anergic T cells) and other highly cross-conserved epitopes from the identified neo-epitope sequences. The resulting sequences will only contain epitopes or neo-epitopes that exhibit low degree of similarity with self-sequences. Neo-epitope sequences are discarded from consideration for use in a personalized neoplasia-specific vaccine if no substring matching the above criteria can be found. Conversely, the same homology analysis can be performed against a set of known infectious disease-derived epitopes known to be immunogenic, extracted for example from the IEDB database, or against a set of other known immunogenic sequences or common pathogen-derived sequences. This analysis has the purpose of identifying neo-epitope candidates that share a high degree of homology with other known or putative effector T cell epitopes. Neoantigens containing such neo-epitopes can be prioritized in vaccine formulations.

In aspects of the method of identifying subject-specific neo-epitopes for a personalized neoplasia vaccine, the step of assessing the identified neo-epitopes encoded by said mutations to identify neo-epitopes that are known or determined (e.g. predicted) to engage regulatory T cells and/or other detrimental T cells (including T cells with potential host cross-reactivity and/or anergic T cells) comprises *in silico* testing. In aspects, *in silico* testing comprises analyzing whether the identified neo-epitopes are predicted to engage regulatory T cells and/or other detrimental T cells (including T cells with potential host cross-reactivity and/or anergic T cells) using the JANUSMATRIX™ algorithm. JANUSMATRIX™ is a homology analysis tool that compares putative T cell epitopes and their TCR-facing residues across genome sequences rather than linear peptide fragments, and thus considers aspects of antigen recognition that are not captured by raw sequence alignment. In aspects, JANUSMATRIX™ parses the epitopes into 9-mer frames and/or 10-mer frames and divides each 9-mer or 10-mer into the MHC-binding agretope and the TCR-binding epitope. In aspects, the TCR-binding epitope (which can be referred to as TCR binding residues, TCR facing epitope, TCR facing residues, or TCR contacts) for a 9-mer identified neo-epitope or epitope that bind to a MHC class II molecule are at position 2, 3, 5, 7, and 8 of the identified neo-epitope, while the MHC-binding agretope (which can be referred to as MHC contacts, MHC facing residues, MHC-binding residues, or MHC-binding face) for a 9-mer identified neo-epitope or epitope that bind to a MHC class II molecule are at position 1, 4, 6, and 9, both as counted from the amino terminal. In aspects, the TCR binding epitope for a 9- mer identified neo-epitope that binds to a MHC class I molecule are at position 4, 5, 6, 7, and 8 of the identified neo-epitope or epitope, while the MHC binding agretope for a 9-mer identified neo-epitope or epitope that bind to a MHC class I molecule are at position 1, 2, 3, and 9, both as counted from the amino terminal. In aspects, the TCR binding epitope for a 10-mer identified neo-epitope that bind to a MHC class I molecule are at position 4, 5, 6, 7, 8, and 9 of the identified neo-epitope, while the MHC binding agretope for a 10-mer identified neo-epitope or epitope that bind to a MHC class I molecule are at position 1, 2, 3, 9, and 10, both as counted from the amino terminal. In aspects, the TCR-binding epitope for a 9-mer identified neo- epitope or epitope that bind to a MHC class II molecule are at any combination of residues at positions 2, 3, 5, 7, and 8 (e.g., but not limited to, positions 3, 5, 7 and 8; positions 2, 5, 7, and 8; positions 2, 3, 5, and 7, etc.) of the identified neo-epitope or epitope, while the MHC binding agretope for a 9-mer identified neo-epitope or epitope is the complementary face to the TCR facing residues, both as counted from the amino terminal. In aspects, the TCR binding epitope for 9-mer identified neo-epitope or epitope that bind to a MHC class I molecule are at positions 4, 5, 6, 7, and 8; 1, 4, 5, 6, 7 and 8; or 1, 3, 4, 5, 6, 7, and 8 of the identified neo-epitope or epitope, while the MHC binding agretope for a 9-mer identified neo-epitope or epitope is the complementary face to the TCR facing residues, both as counted from the amino terminal. In aspects, the TCR-binding epitope for a 10-mer identified neo-epitope or epitope that bind to a MHC class I molecule are at any combination of residues at positions 1, 3, 4, 5, 6, 7, 8, and 9 of the identified neo-epitope or epitope, while the MHC binding agretope for a 10-mer identified neo-epitope or epitope is the complementary face to the TCR facing residues, both as counted from the amino terminal. JANUSMATRIX™ then searches for potentially cross-reactive TCR-facing epitopes across any number of large sequence databases that have been pre-loaded into the tool, including the protein sequences from bacterial and viral organisms that make up the gut microbiome (e.g., the human gut microbiome), autologous proteins from the genome (e.g., the human genome), and viral and bacterial pathogens (e.g., human viral and human bacterial pathogens). JANUSMATRIX™ focuses in 9-mer and/or 10-mer searches because although peptides of different lengths interact with the MHC, most T cell epitopes can be mapped to a minimum of nine or ten amino acids in any given peptide, even if the peptide is longer. In further aspects an identified neo-epitope is predicted to engage regulatory T cells and/or other detrimental T cells (including T cells with potential host cross-reactivity and/or anergic T cells) if the output JANUSMATRIX™ score for the neo-epitope is greater than or equal to 2 (and in further aspects, greater than or equal to 3).

In aspects, the method further comprises determining whether the identified neo-epitopes engage regulatory T cells and/or other detrimental T cells (including T cells with potential host cross-reactivity and/or anergic T cells) *in vitro*. In aspects, a neo-epitope is determined to engage regulatory T cells when said neo-epitope results in regulatory T cell activation, proliferation, and/or IL-10 or TGF-β production. As described previously, in aspects, upon activation, CD4+ regulatory T cells secrete immune suppressive cytokines and chemokines including but not limited to IL-10 and/or TGFβ. CD4+ Tregs may also exert immune suppressive effects through direct killing of target cells, characterized by the expression upon activation of effector molecules including but not limited to granzyme B and perforin. In aspects, CD8+ Tregs are characterized by the presence of certain cell surface markers including but not limited to CD8, CD25, and, upon activation, FoxP3. In aspects, upon activation, regulatory CD8+ T cells secrete immune suppressive cytokines and chemokines including but not limited to IFNy, IL-10, and/or TGFβ. In aspects, upon activation, CD8+ Tregs may also exert immune suppressive effects through direct killing of target cells, characterized by the expression upon activation of effector molecules including but not limited to granzyme B and/or perforin.

In aspects of the method of identifying subject-specific neo-epitopes for a personalized neoplasia vaccine, the step of assessing the identified neo-epitopes encoded by said mutations to identify neo-epitopes that are known or determined (e.g. predicted) to engage regulatory T cells and/or other detrimental T cells (including T cells with potential host cross-reactivity and/or anergic T cells) comprises determining whether the identified neo-epitopes engage regulatory T cells and/or other detrimental T cells (including T cells with potential host cross-reactivity and/or anergic T cells) *in vitro*. In aspects, a neo-epitope is determined to engage regulatory T cells when said neo-epitope results in regulatory T cell activation, proliferation, and/or IL-10 or TGF-β production. As described previously, in aspects, upon activation, CD4+ regulatory T cells secrete immune suppressive cytokines and chemokines including but not limited to IL-10 and/or TGFβ. CD4+ Tregs may also exert immune suppressive effects through direct killing of target cells, characterized by the expression upon activation of effector molecules including but not limited to granzyme B and perforin. In aspects, CD8+ Tregs are characterized by the presence of certain cell surface markers including but not limited to CD8, CD25, and, upon activation, FoxP3. In aspects, upon activation, regulatory CD8+ T cells secrete immune suppressive cytokines and chemokines including but not limited to IFNy, IL-10, and/or TGFβ. In aspects, upon activation, CD8+ Tregs may also exert immune suppressive effects through direct killing of target cells, characterized by the expression upon activation of effector molecules including but not limited to granzyme B and/or perforin. In aspects, cross-reactive or auto-reactive T cell responses will be tested by *in vitro* priming of T cells using neoepitope peptides containing non-synonymous amino acid substitutions and presented by autologous pAPC. This *in vitro* immunogenicity protocol may follow the methodology established by Wullner et al. (Wullner D, Zhou L, Bramhall E, Kuck A, Goletz TJ, Swanson S, Chirmule N, Jawa V. Considerations for Optimization and Validation of an In vitro PBMC Derived T cell Assay for Immunogenicity Prediction of Biotherapeutics. Clin Immunol 2010 Oct; 137(1): 5-14). T cells that expand following *in vitro* priming to the neoepitope peptides will then be tested for reactivity to the corresponding native or wild type (non-mutated) peptide epitopes. Reactivity to native peptide sequences will be determined by measuring cytokine production including, but not limited to, IFNγ, TNFα, IL-2 and/or markers of T cell effector function including, but not limited to, CD107a and granzyme B.

*Ranking Polypeptides Comprising Subject-Specific Neo-Epitopes Encoded by Said Identified Neoplasia-Specific Mutations*

In aspects of the method of identifying subject-specific neo-epitopes for a personalized neoplasia vaccine, the method further comprises ranking peptides or polypeptides comprising said identified subject-specific neo-epitopes, provided said neo-epitope is not identified as being known or determined (e.g. predicted) to engage regulatory T cells and/or other detrimental T cells (including T cells with potential host cross-reactivity and/or anergic T cells), for their usability in an immunogenic composition, such as a personalized neoplasia vaccine. As such, in aspects, the identified subject-specific peptides or polypeptides comprise at least one identified neo-epitope encoded by said identified neoplasia-specific mutations, provided said neo-epitope is not identified as being known or determined (e.g. predicted) to engage regulatory T cells and/or other detrimental T cells (including T cells with potential host cross-reactivity and/or anergic T cells), are ranked for their usability as epitopes in an immunogenic composition, such as a personalized neoplasia vaccine. In aspects, the methods comprise a manual or computer- based analytical process in which peptides or polypeptides comprising the identified subject- specific neo-epitopes are analyzed and selected for their usability in the respective vaccine to be provided. In aspects, said analytical process is a computational algorithm-based process. Preferably, said analytical process comprises determining and/or ranking peptides or polypeptides comprising subject-specific neo-epitopes according to a determination (e.g., prediction) of their capacity of being immunogenic.

In aspects, comprises ranking peptides or polypeptides comprising said identified subject-specific neo-epitopes for their usability as epitopes in an immunogenic composition, such as a personalized neoplasia vaccine comprises determining (e.g., predicting) one or more characteristics associated with the peptides or polypeptides comprising identified subject-specific neo-epitopes, the characteristics including immunogenicity-related features, sequencing-related features, and/or physiochemical-related features.

In aspects, determined immunogenicity-related features of the peptides or polypeptides comprising identified subject-specific neo-epitopes may include one of more of: count of MHC class I neo-epitopes; percentile ranks of the MHC class I neo-epitopes; MHC class I-restricted regulatory T cell and/or other detrimental T cells (including T cells with potential host cross-reactivity and/or anergic T cells) induction potential of the neoantigen (e.g. peptide or polypeptide comprising one or more identified neo-epitopes); Count of MHC class II neo- epitopes; percentile ranks of the MHC class II neo-epitopes; MHC class II-restricted regulatory T cell and/or other detrimental T cells (including T cells with potential host cross-reactivity and/or anergic T cells) induction potential of the neoantigen (e.g. peptide or polypeptide comprising one or more identified neo-epitopes); and/or whether the optimized neoantigen (e.g. peptide or polypeptide comprising one or more identified neo-epitopes) contains both MHC class I and II neo-epitopes.

In aspects, determined sequencing-related features of the peptides or polypeptides comprising identified subject-specific neo-epitopes may include one of more of: expression level of the associated transcript; coverage of the mutation in the tumor DNA, *i.e.*, the number of unique sequencing reads that overlap the genomic position of the mutation; variant allele fraction (VAF) of the mutation in the tumor DNA, *i.e.*, the relative frequency, from 0 to 1, of the observed mutation across sequencing reads; and/or other sequencing metadata, as may be needed.

In aspects, determined physiochemical-related features of the peptides or polypeptides comprising identified subject-specific neo-epitopes may include one of more of: net charge of the optimized neoantigen (e.g. peptide or polypeptide comprising one or more identified neo-epitopes); whether the optimized neoantigen (e.g. peptide or polypeptide comprising one or more identified neo-epitopes) contains at least one charged residue; the count of cysteines (C) within the optimized neoantigen (e.g. peptide or polypeptide comprising one or more identified neo-epitopes); whether the optimized neoantigen (e.g. peptide or polypeptide comprising one or more identified neo-epitopes)contains at least one cysteine (C) and is negatively charged; whether the optimized neoantigen (e.g. peptide or polypeptide comprising one or more identified neo-epitopes) contains a poly-proline motif ('PP'); whether the optimized neoantigen (e.g. peptide or polypeptide comprising one or more identified neo-epitopes)contains at least one methionine (M); whether the optimized neoantigen (e.g. peptide or polypeptide comprising one or more identified neo-epitopes)contains an N-terminal glutamine (Q); whether the optimized neoantigen (e.g. peptide or polypeptide comprising one or more identified neo-epitopes)contains a glycine (G) and/or proline (P) in the last or second to last positions; whether the optimized neoantigen (e.g. peptide or polypeptide comprising one or more identified neo-epitopes)contains a 'DG', DS', 'DA', or 'DN' motif;

and/or the hydropathy index of the optimized neoantigen (e.g. peptide or polypeptide comprising one or more identified neo-epitopes).

In aspects, the method further comprises ranking, based on the determined characteristics, each of the peptides or polypeptides comprising identified subject-specific neo-epitopes. In aspects, the top 5-30, including every value and range therein, ranked peptides or polypeptides comprising identified subject-specific neo-epitopes are included in the personalized neoplasia vaccine. In aspects, the peptides or polypeptides comprising identified subject-specific neo-epitopes are scored and ranked according to the ranking scheme disclosed in Example 1.

*Effect of Inhibitory CD8+ cells*

Suppressor (Treg) CD8+ T cells appear to develop in the periphery and have not been well characterized. CD8+ T cells with suppressive phenotype have been described in human tumors, but data directly correlating control of tumor growth cell phenotype is limited and generation of CD8+ T cells with suppressive phenotype can result from multiple mechanisms. The inventors have characterized CD8+ suppressive effects and developed a score of overall immunogenic activity of variant alleles of a CT26 neoantigen vaccine.

$$Score = Immunogenicity \times VAF$$

where

$$Immunogenicity = (Class\ I\ neo - epitope\ content\ -\ Class\ I\ cross - reactive\ potential) + (Class\ II\ neo - epitope\ content\ -\ Class\ II\ cross - reactive\ potential)$$

and:

*VAF* is the Variant Allele Frequency as it is observed within a tumor sample.

Class I and Class II immunogenicity have also been observed to coincide with effector and regulatory activity, measured for example by cytokine release, or by a predictive algorithm as follows:

$$Class\ I\ Immunogenicity = \beta\ max\left(\sum Z - \left(\sum Z + \frac{JMX}{2}\right)\right)$$

and

$$Class\ II\ Immunogenicity = \alpha\left(0, \sum Z - \left(\sum Z + \frac{JMX}{2}\right)\right)$$

where $Z$ = Neo-epitope Z score (class I or II)
and
JMX = JANUSMATRIX™ score (class I or II)

JANUSMATRIX™ examines cross-reactive T cell epitopes from both HLA binding and TCR-facing sides to allow comparison across large genome sequence databases including common human pathogens. JANUSMATRIX™ score is representative of predicted engagement of regulatory T cells and/or other detrimental T cells.

In general, peptides with more Class II neo-epitopes will have higher Class II immunogenicity scores, and immunogenic potentials can be negated by cross-reactivity potential. Peptides with Class I JMX scores below 3 will retain positive Class II immunogenicity scores, peptides with JMX scores of 3 will have null immunogenicity scores, while peptides with Class II JMX scores of 3 or more will have negative Class II Immunogenicity scores.

In general, peptides with more Class I neo-epitopes will have higher Class I immunogenicity scores, and immunogenic potentials can be negated by cross-reactivity potential. Peptides with Class I JMX scores below 3 will retain positive Class I Immunogenicity scores, while peptides with Class I JMX scores of 3 or more will have null Class I Immunogenicity scores. Said another way, for Class I, high JMX scores had a neutral impact on immunogenicity.

*Analysis of Class II and Class I neo-epitopes in tumors*

Class II and Class I neo-epitopes in tumors were analyzed and correlated with patient outcomes. Neo-epitope expression is a substantially better predictor of disease-free survival (DFS) than mutation burden. Subjects displaying relatively high levels of Teff neo-epitopes compared to Treg epitopes demonstrate longer DFS and the association is improved when both Class II and Class I neo-epitopes are accounted for.

For vaccine design and prognostic applications, analysis of neoepitopes includes consideration of one or more of the following:

Immunogenicity-related features: Count of MHC class I neo-epitopes; Minimal percentile rank of a MHC class I neo-epitope; MHC class I-restricted Treg induction potential of the neoantigen (e.g. peptide or polypeptide comprising one or more identifiedneo-epitopes); Count of MHC class II neo-epitopes (in aspects, which may include one or more of); Minimal percentile rank of a MHC class II neo-epitope; MHC class II-restricted Treg induction potential of the neoantigen (e.g. peptide or polypeptide comprising one or more identified neo-epitopes); Whether the optimized neoantigen (e.g. peptide or polypeptide comprising one or more identified neo-epitopes) contains both MHC class I and II neo-epitopes.

Sequencing-related features (in aspects, which may include one or moreof): Expression level of the associated transcript; Coverage of the mutation in the tumor DNA, i.e. the number of unique sequencing reads that overlap the genomic position of the mutation; Variant allele fraction (VAF) of the mutation in the tumor DNA, i.e. the relative frequency, from 0 to 1, of the observed mutation across sequencing reads; Other sequencing metadata, as needed.

Physicochemical-related features (in aspects, which may include one or more of): Net charge of the optimized neoantigen (e.g. peptide or polypeptide comprising one or more identified neo-epitopes); Whether the optimized neoantigen (e.g. peptide or polypeptide comprising one or more identified neo-epitopes) contains at least one charged residue; The count of cysteines (C) within the optimized neoantigen (e.g. peptide or polypeptide comprising one or more identified neo-epitopes); Whether the optimized neoantigen (e.g. peptide or polypeptide comprising one or more identified neo-epitopes) contains at least one cysteine (C) and is negatively charged; Whether the optimized neoantigen (e.g. peptide or polypeptide comprising one or more identified neo-epitopes) contains a poly-proline motif('PP'); Whether the optimized neoantigen (e.g. peptide or polypeptide comprising one or more identified neo-epitopes) contains at least one methionine(M); Whether the optimized neoantigen (e.g. peptide or polypeptide comprising one or more identified neo-epitopes) contains an N-terminal glutamine (Q); Whether the optimized neoantigen (e.g. peptide or polypeptide comprising one or more identified neo-epitopes) contains a glycine (G) and/or proline (P) in the last or second to last positions; Whether the optimized neoantigen (e.g. peptide or polypeptide comprising one or more identified neo-epitopes) contains a 'DG', DS', 'DA', or 'DN' motif; The hydropathy index of the optimized neoantigen (e.g. peptide or polypeptide comprising one or more identified neo-epitopes).

Scores can be assigned to features of neoantigens. One exemplary scheme follows, including points, percentages, and penalizing steps that may be varied or optimized.

Count of MHC class I neo-epitopes (maximum of *20points*): Neoantigens (e.g. peptide or polypeptide comprising one or more identified neo- epitopes) containing one or less MHC class I neo-epitopes are assigned *0%* of the points (i.e. 0 point); Neoantigens (e.g. peptide or polypeptide comprising one or more identified neo- epitopes) containing two MHC class I neo-epitopes are assigned *80%* of the points (i.e. 16 points); Neoantigens (e.g. peptide or polypeptide comprising one or more identified neo- epitopes) containing three or more MHC class I neo-epitopes are assigned *100%* of the points (i.e. 20 points)

Minimal percentile rank of a MHC class I neo-epitope (maximum of *20 points*): Neoantigens (e.g. peptide or polypeptide comprising one or more identified neo-epitopes) where the minimal percentile rank of a MHC class I neo-epitope falls between 5% (inclusive) and 2.5% (exclusive) are assigned *0%* of the points (i.e. 0 point); Neoantigens (e.g. peptide or polypeptide comprising one or more identified neo- epitopes) where the minimal percentile rank of a MHC class I neo-epitope falls between 1% (exclusive) and 2.5% (inclusive) are assigned *50%* of the points (i.e. 10 points); Neoantigens (e.g. peptide or polypeptide comprising one or more identified neo- epitopes) where the minimal percentile rank of a MHC class I neo-epitope falls within 1% (inclusive) are assigned *100%* of the points (i.e. 20 points)

Count of MHC class II neo-epitopes (maximum of *10points*): Neoantigens (e.g. peptide or polypeptide comprising one or more identified neo- epitopes) containing one or less MHC class II neo-epitopes are assigned *0%* of the points (i.e. 0 point); Neoantigens (e.g. peptide or polypeptide comprising one or more identified neo- epitopes) containing 2 MHC class II neo-epitopes are assigned *80%* of the points (i.e. 8 points); Neoantigens (e.g. peptide or polypeptide comprising one or more identified neo- epitopes) containing three or more MHC class II neo-epitopes are assigned *100%* of the points (i.e. 10 points)

Minimal percentile rank of a MHC class II neo-epitope (maximum of *5 points*): Neoantigens (e.g. peptide or polypeptide comprising one or more identified neo-epitopes) where the minimal percentile rank of a MHC class II neo-epitope falls between 5% (inclusive) and 2.5 (exclusive) are assigned *0%* of the points (i.e. 0 point); Neoantigens (e.g. peptide or polypeptide comprising one or more identified neo-epitopes) where the minimal percentile rank of a MHCclass II neo-epitope falls between 1% (exclusive) and 2.5% (inclusive) are assigned *50%* of the points (i.e. 2.5 points); Neoantigens (e.g. peptide or polypeptide comprising one or more identified neo-epitopes) where the minimal percentile rank of a MHC class II neo-epitope falls within 1% (inclusive) are assigned *100%* of the points (i.e. 5 points)

Presence of both MHC class I and II neo-epitopes (maximum of *20 points*): Neoantigens (e.g. peptide or polypeptide comprising one or more identified neo-epitopes) containing MHC class I neo-epitopes only or MHC class II neo- epitopes only are assigned *0%* of the points (i.e. 0 point); Neoantigens (e.g. peptide or polypeptide comprising one or more identified neo-epitopes) containing at least one MHC class I neo-epitope and at least one MHC class II neo-epitope are assigned *100%* of the points (i.e. 20points)

MHC class I-restricted Treg induction potential (maximum of *5points*): Neoantigens (e.g. peptide or polypeptide comprising one or more identified neo-epitopes) with a MHC class I-restricted average depth of coverage within the reference proteome, or MHC class I Homology Score (as calculated above), between *0* (inclusive) and *0.25* (exclusive) are assigned *100%* of the points (i.e. 5 points); Neoantigens (e.g. peptide or polypeptide comprising one or more identified neo-epitopes) with a MHC class I-restricted average depth of coverage within the reference proteome, or MHC class I Homology Score, between *0.25* (inclusive) and *0.5* (exclusive) are assigned *50%* of the points (i.e. 2.5 points); Neoantigens (e.g. peptide or polypeptide comprising one or more identified neo-epitopes) with a MHC class I-restricted average depth of coverage within the reference proteome, or MHC class I Homology Score, between *0.5* (inclusive) and *1* (exclusive) are assigned *10%* of the points (i.e. 0.5 points); Neoantigens (e.g. peptide or polypeptide comprising one or more identified neo-epitopes) with a MHC class I-restricted average depth of coverage within the reference proteome, or MHC class I Homology Score, above *1* (inclusive) are assigned *0%* of the points (i.e. 0 point)

MHC class II-restricted Treg induction potential (maximum of *20points*): Neoantigens (e.g. peptide or polypeptide comprising one or more identified neo-epitopes) with a MHC class II-restricted average depth of coverage within the reference proteome, or MHC class II Homology Score, between *0* (inclusive) and *0.25* (exclusive) are assigned *100%* of the points (i.e. 20 points); Neoantigens (e.g. peptide or polypeptide comprising one or more identified neo-epitopes) with a MHC class II-restricted average depth of coverage within the reference proteome, or MHC class II Homology Score, between *0.25* (inclusive) and *0.5* (exclusive) are assigned *50%* of the points (i.e. 10 points); Neoantigens (e.g. peptide or polypeptide comprising one or more identified neo-epitopes) with a MHC class II-restricted average depth of coverage within the reference proteome, or MHC class II Homology Score, between *0.5* (inclusive) and *1* (exclusive) are assigned *10%* of the points (i.e. 2 points); Neoantigens (e.g. peptide or polypeptide comprising one or more identified neo-epitopes) with a MHC class II-restricted average depth of coverage within the reference proteome, or MHC class II Homology Score, above *1* (inclusive) are assigned *0%* of the points (i.e. 0 point)

Transcript expression (in e.g. Transcript Per Million, TPM, which is calculated as is known in the art) (maximum of *30points*): Neoantigens (e.g. peptide or polypeptide comprising one or more identified neo-epitopes) derived from a transcript whose expression lies in the top *10%* of the TPMs are assigned *100%* of the points (i.e. 30 points); Neoantigens (e.g. peptide or polypeptide comprising one or more identified neo-epitopes) derived from a transcript whose expression lies below the top *25%* of the TPMs are assigned *0%* of the points (i.e. 0 points); Neoantigens (e.g. peptide or polypeptide comprising one or more identified neo-epitopes) derived from a transcript whose expression lies between the top *25%* and *10%* of the TPMs are assigned a linearly distributed percent of points.

Coverage, calculated as is known in the art (maximum of *1 point*): Neoantigens (e.g. peptide or polypeptide comprising one or more identified neo-epitopes) containing a mutation with a depth of coverage in the tumor DNA of less than *20* are assigned *0%* of the points (i.e. 0 point); Neoantigens (e.g. peptide or polypeptide comprising one or more identified neo-epitopes) containing a mutation with a coverage in the tumor DNA of between *20* and *50* (strictly below) are assigned *50%* of the points (i.e. 0.5 point); Neoantigens (e.g. peptide or polypeptide comprising one or more identified neo-epitopes) containing a mutation with a coverage in the tumor DNA of *50* or more are assigned *100%* of the points (i.e. 1 point).

Variant allele fraction (VAF), calculated as is known in the art (maximum of *20points*):

For neoantigens (e.g. peptide or polypeptide comprising one or more identified neo-epitopes) derived from the mutanome of syngeneic models: Neoantigens (e.g. peptide or polypeptide comprising one or more identified neo-epitopes) containing a mutation with a VAF below *0.5* are assigned *0%* of the points (i.e. 0 point); Neoantigens (e.g. peptide or polypeptide comprising one ormore identified neo-epitopes) containing a mutation with a VAF between *0.5* and *0.75* (strictly below) are assigned *50%* of the points (i.e. 10 points); Neoantigens (e.g. peptide or polypeptide comprising one or more identified neo-epitopes) containing a mutation with a VAF equal to or more than *0.75* are assigned *100%* of the points (i.e. 20 points)

For neoantigens (e.g. peptide or polypeptide comprising one or more identified neo-epitopes) derived from the mutanome of patients: Neoantigens (e.g. peptide or polypeptide comprising one or more identified neo-epitopes) containing a mutation with a VAF below *0.1* are assigned *0%* of the points (i.e. 0 point); Neoantigens (e.g. peptide or polypeptide comprising one or more identified neo-epitopes) containing a mutation with a VAF between *0.1* and *0.25* (strictly below) are assigned *50%* of the points (i.e. 10points); Neoantigens (e.g. peptide or polypeptide comprising one or more identified neo-epitopes) containing a mutation with a VAF equal to or more than *0.25* are assigned *100%* of the points (i.e. 20points).

Penalties cam be assigned to a candidate neoantige.

Severe penalties (e.g. set to a deduction of 100 points, which may be assigned before or after the 100-point normalization) if: the neoantigen (e.g. peptide or polypeptide comprising one or more identified neo- epitopes) has no charged residues, or the neoantigen (e.g. peptide or polypeptide comprising one or more identified neo- epitopes) has a null net charge, or the neoantigen (e.g. peptide or polypeptide comprising one or more identified neo- epitopes) contains at least two cysteines, or the neoantigen (e.g. peptide or polypeptide comprising one or more identified neo- epitopes) contains at least one cysteine and is negatively charged, or the neoantigen (e.g. peptide or polypeptide comprising one or more identified neo-epitopes) contains an N-terminal glutamine, or the neoantigen (e.g. peptide or polypeptide comprising one or more identified neo- epitopes) contains a poly-proline motif, or the neoantigen (e.g. peptide or polypeptide comprising one or more identified neo- epitopes) has an hydropathy index greater or equal to 2.

Moderate penalties (e.g. set to a deduction of 10 points) can be assigned to a candidate neoantigen (e.g. peptide or polypeptide comprising one or more identified neo- epitopes) if: the neoantigen (e.g. peptide or polypeptide comprising one or more identified neo-epitopes) contains one cysteine

Minor penalties (e.g. set to a deduction of 1 point) can be assigned to a candidate neoantigen (e.g. peptide or polypeptide comprising one or more identified neo-epitopes) if: the neoantigen (e.g. peptide or polypeptide comprising one or more identified neo-epitopes) is negatively charged, or the neoantigen (e.g. peptide or polypeptide comprising one or more identified neo- epitopes) contains at least one methionine, or the neoantigen (e.g. peptide or polypeptide comprising one or more identified neo- epitopes) contains a glycine and/or proline in the last or second to last positions, or the neoantigen (e.g. peptide or polypeptide comprising one or more identified neo- epitopes) contains a 'DG', DS', 'DA', or 'DN' motif.

*Designing of Subject-Specific Peptides Comprising At Least One Identified Neo-Epitope Encoded by Said Identified Neoplasia-Specific Mutations*

In aspects of the method of identifying subject-specific neo-epitopes for a personalized neoplasia vaccine, the method further comprises designing at least one subject-specific peptide or polypeptide, said peptide or polypeptide comprising at least one identified neo-epitope encoded by said mutations, provided said neo-epitope is not identified in as being known or determined (e.g. predicted) to engage regulatory T cells and/or other detrimental T cells (including T cells with potential host cross-reactivity and/or anergic T cells).

In aspects of the method of identifying subject-specific neo-epitopes for a personalized neoplasia vaccine, the method further includes: iv) designing at least one subject-specific peptide or polypeptide, said peptide or polypeptide comprising at least one identified neo- epitope encoded by said mutations, provided said neo-epitope is not identified in step (iii) as being known or determined (e.g. predicted) to engage regulatory T cells and/or other detrimental T cells (including T cells with potential host cross-reactivity and/or anergic T cells). In aspects, the method further includes providing the at least one peptide or polypeptide designed in step (iv) or a nucleic acid encoding said peptides or polypeptides. In even further aspects, the method further includes vi) providing a vaccine comprising the at least one peptide or polypeptide or nucleic acid provided in step (v).

In aspects, the subject-specific peptides or polypeptides comprising the at least one identified neo-epitope can be of a variety of lengths. In aspects, such subject-specific peptides or polypeptides will at least contain an identified neo-epitope that is determined (e.g. predicted) to bind to the MHC molecule of the patient. In aspects, the subject-specific peptides or polypeptides comprise additional adjacent amino acids extending in the N- and/or C-terminal directions. In aspects, the subject-specific peptides or polypeptides comprising the at least one identified neo-epitope comprising, consisting, or consisting essentially of an amino acid sequence of the at least one identified neo-epitope (e.g., a 9-mer identified neo-epitope that bind to a MHC class II molecule and/or a 9-mer or 10-mer identified neo-epitope that bind to a MHC Class I molecule, and/or fragments or variants thereof), and optionally 1 to 12 additional amino acids distributed in any ratio on the N terminus and/or C-terminus of the the at least one identified neo-epitope. In aspects, the instant disclosure is directed to a peptide or polypeptide have a core amino acid sequence comprising, consisting of, or consisting essentially of the at least one identified neo-epitope, and optionally having extensions of 1 to 12 amino acids on the C-terminal and/or the N-terminal of the core amino acid sequence, wherein the overall number of these flanking amino acids is 1 to 12, 1 to 3, 2 to 4, 3 to 6, 1 to 10, 1 to 8, 1 to 6, 2 to 12, 2 to 10, 2 to 8, 2 to 6, 3 to 12, 3 to 10, 3 to 8, 3 to 6, 4 to 12, 4 to 10, 4 to 8, 4 to 6, 5 to 12, 5 to 10, 5 to 8, 5 to 6, 6 to 12, 6 to 10, 6 to 8, 7 to 12, 7 to 10, 7 to 8, 8 to 12, 8 to 10, 9 to 12, 9 to 10, or 10 to 12, wherein the flanking amino acids can be distributed in any ratio to the C-terminus and the N-terminus (for example all flanking amino acids can be added to one terminus, or the amino acids can be added equally to both termini or in any other ratio). In aspects, the instant disclosure is directed to a peptide or polypeptide have a core sequence comprising, consisting of, or consisting essentially of the at least one identified neo-epitope (and/or fragments and variants thereof), optionally with extensions of 1 to 12 amino acids on the C-terminal and/or the N-terminal, wherein the overall number of these flanking amino acids is 1 to 12, 1 to 3, 2 to 4, 3 to 6, 1 to 10, 1 to 8, 1 to 6, 2 to 12, 2 to 10, 2 to 8, 2 to 6, 3 to 12, 3 to 10, 3 to 8, 3 to 6, 4 to 12, 4 to 10, 4 to 8, 4 to 6, 5 to 12, 5 to 10, 5 to 8, 5 to 6, 6 to 12, 6 to 10, 6 to 8, 7 to 12, 7 to 10, 7 to 8, 8 to 12, 8 to 10, 9 to 12, 9 to 10, or 10 to 12, wherein the flanking amino acids can be distributed in any ratio to the C-terminus and the N-terminus (for example all flanking amino acids can be added to one terminus, or the amino acids can be added equally to both termini or in any other ratio), provided that the polypeptide with the flanking amino acids is still able to bind to the same HLA molecule (i.e., retain MHC binding propensity) as said polypeptide core sequence without said flanking amino acids. In aspects, said polypeptide with the flanking amino acids is still able to bind to the same HLA molecule (i.e., retain MHC binding propensity) and retain the same TCR specificity as said polypeptide core sequence without said flanking amino acids. In aspects, said flanking amino acid sequences are those that also flank the the at least one identified neo-epitope in the naturally occurringprotein.

In aspects, the subject-specific peptides or polypeptides can be capped with an N-terminal acetyl and C-terminal amino group. In aspects, the subject-specific peptides or polypeptides can be either in neutral (uncharged) or salt forms and may be either free of or include modifications such as glycosylation, side chain oxidation, or phosphorylation.

In aspects, a subject-specific peptide or polypeptide can be "isolated" or "purified", which means that it is substantially free of cellular material when it is isolated from recombinant and non-recombinant cells, or free of chemical precursors or other chemicals when it is chemically synthesized. A subject-specific peptide or polypeptide of the present disclosure, however, can be joined to, linked to, or inserted into another polypeptide (e.g., a heterologous polypeptide) with which it is not normally associated in a cell and still be "isolated" or "purified."

In aspects, a subject-specific peptide or polypeptide may comprise, but is not limited to, about 9 to about 100 amino acid residues, including any value or range therein. In aspects, a subject-specific peptide or polypeptide may comprise greater than 100 amino acid residues. In aspects, each subject-specific peptide or polypeptide comprising one or more identified neo-epitopes has a length of from 9-40 amino acids, from 9-30 amino acids, from 9-25 amino acids, from 9-23 amino acids, from 9-20 amino acids, or from 9-15 amino acids. In aspects, a subject-specific peptide or polypeptide may comprise at least one identified neo-epitope that is determined (e.g. predicted) to bind to the MHC molecule of the patient, with each at least one neo-epitope including an extension of amino acids (e.g., of a length of 1-12 amino acids, as described above), the extension possibly serving to improve the biochemical properties of the subject-specific peptides or polypeptides (e.g., but not limited to solubility or stability) or to improve the likelihood for efficient proteasomal processing of the peptide.

In aspects a subject-specific peptide or polypeptide may comprise one or more identified neo-epitopes, wherein each one or more identified neo-epitopes may be spaced by linkers, in particular neutral linkers. The term "linker" refers to a peptide added between two peptide domains such as epitopes or vaccine sequences to connect said peptide domains. In aspects, a linker sequence is used to reduce steric hindrance between each one or more identified neo-epitopes, is well translated, and supports or allows processing of the each one or more identified neo-epitopes. The linker should have little or no immunogenic sequence elements. For example, in aspects, the present disclosure is directed to a concatemeric polypeptide or peptide that comprises one or more of the instantly-disclosed subject-specific peptides or polypeptides linked, fused, or joined together (e.g., fused in-frame, chemically-linked, or otherwise bound) to an additional peptide or polypeptide. Such additional peptide or polypeptide may be one or more of the instantly-disclosed subject-specific peptides or polypeptides, or may be an additional peptide or polypeptide of interest, such as traditional tumor-associated antigens (TAAs). In aspects a concatemeric peptide is composed of 2 or more, 3 or more, 4 or more, 5 or more 6 or more 7 or more, 8 or more, 9 or more of the instantly-disclosed subject-specific peptides or polypeptides. In other aspects, the concatemeric peptides or polypeptides include 1000 or more, 1000 or less, 900 or less, 500 or less, 100 or less, 75 or less, 50 or less, 40 or less, 30 or less, 20 or less or 100 or less subject-specific peptides or polypeptides. In yet other embodiments, a concatemeric peptide has 3-100, 5-100, 10-100, 15-100, 20-100, 25-100, 30- 100, 35-100, 40-100, 45-100, 50-100, 55-100, 60-100, 65-100, 70-100, 75-100, 80-100, 90-100, 5-50, 10-50, 15-50, 20-50, 25-50, 30-50, 35-50, 40-50, 45-50, 100-150, 100-200, 100-300, 100-400, 100-500, 50-500, 50-800, 50-1,000, or 100-1,000 of the instantly-disclosed subject-specific peptides or polypeptides linked, fused, or joined together. Each peptide or polypeptide of the concatemeric polypeptide may optionally have one or more linkers, which may optionally be cleavage sensitive sites, adjacent to their N and/or C terminal end. In such a concatemeric peptide, two or more of the peptides (including subject-specific peptides or polypeptides as disclosed herein) may have a cleavage sensitive site between them. Alternatively, two or more of the peptides (including subject-specific peptides or polypeptides as disclosed herein) may be connected directly to one another or through a linker that is not a cleavage sensitive site.

As used herein, two peptide or olypeptides (or a region of the polypeptides) are substantially homologous or identical when the amino acid sequences are at least about 45-55%, typically at least about 70-75%, more typically at least about 80-85%, more typically greater than about 90%, and more typically greater than 95% or more homologous or identical. To determine the percent homology or identity of two amino acid sequences, or of two nucleic acid sequences, the sequences are aligned for optimal comparison purposes (e.g., gaps can be introduced in the sequence of one polypeptide or nucleic acid molecule for optimal alignment with the other polypeptide or nucleic acid molecule). The amino acid residues or nucleotides at corresponding amino acid positions or nucleotide positions are then compared. When a position in one sequence is occupied by the same amino acid residue or nucleotide as the corresponding position in the other sequence, then the molecules are homologous at that position. As used herein, amino acid or nucleic acid "homology" is equivalent to amino acid or nucleic acid "identity". The percent homology between the two sequences is a function of the number of identical positions shared by the sequences (e.g., percent homology equals the number of identical positions/total number of positions × 100).

In aspects, the present disclosure also encompasses a subject-specific peptides or polypeptides comprising at least one identified neo-epitope, with the at least one identified neo-epitope having a lower degree of identity but having sufficient similarity so as to perform one or more of the same functions. Similarity is determined by conserved amino acid substitution. Such substitutions are those that substitute a given amino acid in a polypeptide by another amino acid of like characteristics. Conservative substitutions are likely to be phenotypically silent. Typically seen as conservative substitutions are the replacements, one for another, among the aliphatic amino acids Ala, Val, Leu, Met, and Ile; interchange of the hydroxyl residues Ser and Thr, exchange of the acidic residues Asp and Glu, substitution between the amide residues Asn and Gln, exchange of the basic residues His, Lys and Arg and replacements among the aromatic residues Trp, Phe and Tyr. Guidance concerning which amino acid changes are likely to be phenotypically silent are found (Bowie JU et al., (1990), Science, 247(4948):130610).

In aspects, a variant of the at the least one identified neo-epitope of the subject specific peptides of polypeptides can differ in amino acid sequence by one or more substitutions, deletions, insertions, inversions, fusions, and truncations or a combination of any of these. In aspects, variant of the at least one identified neo-epitope of the subject specific peptides of polypeptides can be fully functional (e.g., retain MHC binding propensity and TCR specificity) or can lack function in one or more activities. Fully functional variants typically contain only conservative variation or variation in non-critical residues or in non-critical regions; in this case, typically MHC contact residues provided MHC binding is preserved. In aspects, functional variants can also contain substitution of similar amino acids that result in no change or an insignificant change in function (e.g., retain MHC binding propensity and TCR specificity). Alternatively, such substitutions can positively or negatively affect function to some degree. Non-functional variants typically contain one or more non-conservative amino acid substitutions, deletions, insertions, inversions, or truncation or a substitution, insertion, inversion, or deletion in a critical residue or critical region; in this case, typically TCR contact residues.

In aspects, the present disclosure also includes fragments of the instantly-disclosed at least one identified neo-epitope of the subject specific peptides of polypeptides. In aspects, the present disclosure also encompasses fragments of the variants of the identified neo-epitopes described herein. In aspects, as used herein, a fragment comprises at least about nine contiguous amino acids. Useful fragments (and fragments of the variants of the identified neo-epitopes described herein) include those that retain one or more of the biological activities of the identified neo-epitope, particularly MHC binding propensity and TCR specificity. Biologically active fragments are, for example, about 9, 12, 15, 16, 20 or 30 or more amino acids in length, including any value or range therebetween. In aspects, fragments can be discrete (not fused to other amino acids or polypeptides) or can be within a larger polypeptide. In aspects, several fragments can be comprised within a single larger polypeptide. In aspects, a fragment designed for expression in a host can have heterologous pre- and pro-polypeptide regions fused to the amino terminus of the polypeptide fragment and an additional region fused to the carboxyl terminus of the fragment.

In aspects, the at the least one identified neo-epitope of the subject specific peptides of polypeptides can include allelic or sequence variants ("mutants") or analogs thereof. In aspects, the subject-specific peptides or polypeptides comprising the at least one identified neo-epitope can include chemical modifications (e.g., pegylation, glycosylation). In aspects, a mutant retains the same functions performed by a polypeptide encoded by a nucleic acid molecule of the present disclosure, particularly MHC binding propensity and TCR specificity. In aspects, a mutant can provide for enhanced binding to MHC molecules. In aspects, a mutant can lead to enhanced binding to TCRs. In another instance, a mutant can lead to a decrease in binding to MHC molecules and/or TCRs. Also contemplated is a mutant that binds but does not allow signaling via the TCR.

In aspects, a subject-specific peptide or polypeptide comprising at least one identified neo-epitope can include a pharmaceutically acceptable salt thereof. "Pharmaceutically acceptable salt" of a peptide or polypeptide means a salt that is pharmaceutically acceptable and that possesses the desired pharmacological activity of the parent peptide or polypetide. As used herein, "pharmaceutically acceptable salt" refers to derivative of the instantly-disclosed peptides or polypeptides, wherein such compounds are modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2- acetoxybenzoic, 2-hydroxyethane sulfonic, acetic, ascorbic, benzene sulfonic, benzoic, bicarbonic, carbonic, citric, edetic, ethane disulfonic, 1,2-ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

The subject-specific peptides or polypeptides comprising the at least one identified neo-epitope may be produced by any known methods of producing peptides or polypeptides, including known *in vitro* and *in vivo* methods. *In vitro* production may be done by variety of methods known in the art, which include peptide or polypeptide chemical synthesis techniques, the expression of proteins, polypeptides or peptides through standard molecular biological techniques, the isolation of proteins or peptides from natural sources, *in vitro* translation, followed by any necessary purification of the expressed peptide/polypeptide. Alternatively, the subject-specific peptides or polypeptides comprising the at least one identified neo-epitope may be produced *in vivo* by introducing molecules (e.g., DNA, RNA, viral expression systems, and the like) that encode tumor specific neoantigens into a subject, whereupon the encoded tumor specific neoantigens are expressed.

In aspects, the present disclosure also provides for nucleic acids (e.g., DNA, RNA, vectors, viruses, or hybrids) that encode in whole or in part one or more peptides or polypeptides or concatemeric peptides of the present disclosure. In aspects, a nucleic acid (e.g., a polynucleotide) encoding a subject-specific peptides or polypeptides or concatemeric peptides comprising the at least one identified neo-epitope may be used to produce the neo-epitope *in vitro* or *in vivo*. The polynucleotide may be, e.g., DNA, cDNA, PNA, CNA, RNA, either single- and/or double-stranded, or native or stabilized forms of polynucleotides as are known in the art. An expression vector capable of expressing a polypeptide can also be prepared. Expression vectors for different cell types are well known in the art and can be selected without undue experimentation. Generally, the DNA is inserted into an expression vector, such as a plasmid, in proper orientation and correct reading frame for expression. If necessary, the DNA may be linked to the appropriate transcriptional and translational regulatory control nucleotide sequences recognized by the desired host (e.g., bacteria), although such controls are generally available in the expression vector. The vector is then introduced into the host bacteria for cloning using standard techniques (see, e.g., Sambrook et al. (1989) Molecular Cloning, A Laboratory Manual, Cold Spring Harbor Laboratory, Cold Spring Harbor,N.Y.).

In aspects, the present disclosure is directed to expression vectors comprising the subject-specific peptides or polypeptides or concatemeric peptides comprising the at least one identified neo-epitope, as well as host cells containing the expression vectors, are also contemplated. The subject-specific peptides or polypeptides or concatemeric peptides comprising the at least one identified neo-epitope may be provided in the form of RNA or cDNA molecules encoding the desired neo-epitopes. One or more peptides or polypeptides or concatemeric peptides of the present disclosure may be encoded by a single expression vector. Such nucleic acid molecules may act as vehicles for delivering neoantigenic peptides/polypeptides/concatemeric peptides to the subject in need thereof, *in vivo,* in the form of, e.g., DNA/RNA vaccines.

In aspects, the subject-specific peptides or polypeptides (including concatemeric peptides) comprising at least one identified neo-epitope can be purified to homogeneity or partially purified. It is understood, however, that preparations in which the subject-specific peptides or polypeptides comprising at least one identified neo-epitope are not purified to homogeneity are useful. The critical feature is that the preparation allows for the desired function of the at least one neo-epitope, even in the presence of considerable amounts of other components. Thus, the present disclosure encompasses various degrees of purity. In one embodiment, the language "substantially free of cellular material" includes preparations of the subject-specific peptides or polypeptides comprising at least one identified neo-epitope having less than about 30% (by dry weight) other proteins (e.g., contaminating protein), less than about 20% other proteins, less than about 10% other proteins, less than about 5% other proteins, less than about 4% other proteins, less than about 3% other proteins, less than about 2% other proteins, less than about 1% other proteins, or any value or range therein. In aspects, when a subject-specific peptide or polypeptide comprising at least one identified neo-epitope of the present disclosure is recombinantly produced, said peptide or polypeptide can also be substantially free of culture medium, for example, culture medium represents less than about 20%, less than about 10%, or less than about 5% of the volume of the peptide or polypeptide or nucleic acid preparation. The language "substantially free of chemical precursors or other chemicals" includes preparations of the peptide or polypeptide or nucleic acid is separated from chemical precursors or other chemicals that are involved in its synthesis. The language "substantially free of chemical precursors or other chemicals" can include, for example, preparations of the peptide or polypeptide having less than about 30% (by dry weight) chemical precursors or other chemicals, less than about 20% chemical precursors or other chemicals, less than about 10% chemical precursors or other chemicals, less than about 5% chemical precursors or other chemicals, less than about 4% chemical precursors or other chemicals, less than about 3% chemical precursors or other chemicals, less than about 2% chemical precursors or other chemicals, or less than about 1% chemical precursors or other chemicals.

**,Examples**

*Example 1*

Tumor growth inhibition post vaccination with a Tregitope-depleted personalized cancer vaccine.

Clinical studies have highlighted the potential of precision cancer immunotherapy to effectively control the tumor of patients across cancer indications. However, recent studies showcase the difficulty of establishing robust CD8+ and CD4+ T cell responses. Poor cancer vaccine performance is due in part to the inadvertent inclusion of suppressive T cell neo-epitopes in neoantigen vaccines that may be recognized by regulatory T cells (Tregs).

To test this hypothesis, we identify and select neo-epitopes and exclude such identified neo-epitopes that are predicted to engage regulatory T cells from the subject-specific neo-epitopes for use in the personalized neoplasia vaccine). State-of-the-art predictive algorithms have been extensively validated in prospective vaccine studies for infectious diseases (Moise et al., Hum. Vaccines Immunother 2015; Wada et al., Sci. Rep. 2017). Distinctive features of the procedure over other *in silico* pipelines include the ability to accurately predict CD4+ and CD8+T cell epitopes and to identify tolerated or Treg epitopes.

Bioinformatics design of a Tregitope-depleted vaccine

Each peptide is first parsed into overlapping 9- and 10-mer frames and evaluated frame for its likelihood to bind to Balb/c MHC class I (H2-Dd and H2-Kd) and MHC class II (I-Ad, I-Ed) alleles. For human analyses, each frame is evaluated for its likelihood to bind to the patient's MHC class I (HLA-A, HLA-B) and MHC class II (HLA-DRB1) alleles. In the event that a patient expresses an MHC allele for which predictions are not readily available, a homology analysis can be performed between the patient allele and our reference alleles. For this analysis, we extract the amino acid sequences from the MHC binding pockets of the patient allele and compare them to the binding pockets from alleles we reliably model. Any non-supported patient allele whose MHC binding pockets are at least 90% homologous with the MHC binding pockets of a reference allele can be included in our analysis, where the model for the homologous reference allele is used to assess a frame's likelihood of binding to the patient allele.

Each frame-by-allele "assessment" is a statement about (i.e., determination of) predicted MHC binding affinity. Raw binding scores are adjusted to fit a normal, or Z-distribution. Raw binding scores are normalized based on the average ($\mu$) binding score and standard deviation ($\sigma$) of a set of 10,000 random 9- or 10-mer amino acid sequences, following the naturally observed amino acid frequencies from UniProtKB/Swiss-Prot (web.expasy.org/docs/relnotes/relstat.html), as follows:

$$Normalized\ binding\ score = \frac{Raw\ binding\ score\ -\ \mu}{\sigma}$$

Normalized binding scores, herein referred to as binding scores or likelihood of binding, within the top 5% of this normal distribution are defined as "hits"; that is to say, potentially immunogenic and worthy of further consideration. These peptides have a significant chance of binding to MHC molecules with moderate to high affinity and, therefore, have a significant chance of being presented on the surface of both professional antigen presenting cells (APC) such as dendritic cells or macrophages, as well as non-professional APC, where they may be interrogated by passing T cells.

T cell epitopes predicted in mutated sequences are compared to normal matched sequences in order to identify neo-epitopes. T cell epitopes from mutated sequences are labeled as neo-epitopes if:

- Their likelihood of binding to MHC falls within the top 5 percentile of our expected distribution and the likelihood of binding to MHC of the normal matched sequence falls below the top 10 percentile of the expected distribution, or;
- Their likelihood of binding to MHC falls within the top 5 percentile of our expected distribution and the likelihood of binding to MHC of the normal matched sequence falls within the top 10 percentile of the expected distribution, and there is a least one mismatched TCR-facing amino acid between the mutated and non-mutated peptides.

TCR facing amino acid residues for a 9-mer mutated peptide and a 9-mer non-mutated peptide that bind to a MHC class II molecule are at position 2, 3, 5, 7, and 8 of the mutated and non-mutated peptide as counted from the amino terminal, wherein the TCR facing amino acid residues for a 9-mer mutated peptide and a 9-mer non-mutated peptide that bind to a MHC class I molecule are at position 4, 5, 6, 7, and 8 of the mutated and non-mutated peptide as counted from the amino terminal, and wherein the TCR facing amino acid residues for a 10-mer mutated peptide and 10-mer non-mutated peptide that bind to a MHC class I molecule are at position 4, 5, 6, 7, 8, and 9 of the mutated and non-mutated peptide as counted from the amino terminal.

Additionally, sequences presenting a high likelihood of binding to MHC are screened using a customized homology search to remove epitopes containing combinations of TCR-facing residues that are commonly found in a reference proteome. This homology screen first considers all the predicted epitopes contained within a given protein sequence and divides each predicted epitope into its constituent agretope and epitope. Each sequence is then screened against a database of murine proteins derived from the UniProt database (UniProt Proteome ID UP000000589, Reviewed/Swiss-Prot set). For human analyses, each sequence would be then screened against a database of human proteins derived from the UniProt database (UniProt Proteome ID UP000005640, Reviewed/Swiss-Prot set).

Cross-conserved epitopes, or peptides derived from the reference proteome with a compatible MHC binding agretope (i.e. the agretopes of both the input (mutated) peptide and its reference non-mutated counterpart are predicted to bind to the same MHC allele) and exactly the same TCR facing epitope, are returned. The Homology Score of an epitope corresponds to the number of matching cross-conserved MHC binding peptides within the reference proteome. In other words, the Homology Score $H_e$ of an epitope e is calculated as follows:

$$H_e = |X_e|$$

where:

$X_e$ corresponds to the set of MHC binding peptides derived from the reference proteome that are restricted to the same MHC class I or MHC class II as epitope e and presenting a TCR facing epitope identical to the epitope e.

By extension, the Homology Score of a given peptide or protein corresponds to the average Homology Score of each individual epitope contained with the peptide or protein. In other words, the Homology Score $H_p$ of a peptide p is calculated as follows:

$$H_p = \frac{\sum_{e \in E} H_e}{|E|}$$

where:

- E corresponds to the set of MHC class I- or MHC class II-restricted epitopes within peptide p;
- $H_e$ corresponds to the Homology Score of epitope e as defined above.

T cells that recognize antigen-derived epitopes sharing TCR contacts with epitopes derived from self may be deleted or rendered anergic during thymic selection before they can be released to the periphery. As such, vaccine components targeting these T cells may be ineffective. On the other hand, vaccine-induced immune response targeting cross-reactive epitopes may induce unwanted autoimmune responses targeting the homologues of the cross- reactive epitopes identified by our homology search. As a result, vaccine safety may be reduced. A review of MHC class II-restricted T cell epitopes contained in the IEDB database (iedb.org) indicates that there is a statistically significant relationship between high Homology Scores and observed production of IL-10 and a statistically significant inverse relationship between high Homology Scores and observed production of IL-4 (see, e.g., Moise et al. iVax: An integrated toolkit for the selection and optimization of antigens and the design of epitope- driven vaccines. Human Vaccin Immunother. 2015; 11(9):2312-21, herein incorporated by reference in its entirety). Conversely, the same homology analysis can be performed against a set of known infectious disease-derived epitopes known to be immunogenic, extracted for example from the IEDB database, or against a set of other known immunogenic sequences or common pathogen-derived sequences. This analysis has the purpose of identifying neo-epitope candidates that share a high degree of homology with other known or putative effector T cell epitopes. Peptides or polypeptides containing such neo-epitopes (or nucleic acid encoding said peptides or polypeptides) can be prioritized in vaccine formulations.

Each mutated sequence undergoes the homology screen described above in order to characterize the degree of similarity with self of each of the encoded MHC class I- and MHC class II-restricted epitopes and neo-epitopes. MHC class I or MHC class II epitopes and MHC class I or MHC class II neo-epitopes with two or more cross-reactive matches in the reference proteome are categorized as exhibiting a high degree of similarity with self and are considered to have a higher likelihood of being tolerated or to engage regulatory T cells and/or other detrimental T cells (including T cells with potential host cross-reactivity and/or anergic T cells). An optimization procedure is then run on each mutated sequence to determine if a substring within the amino acid sequence can be found, such that:

- At least one MHC class I- or MHC class II-restricted epitope is encoded in the substring, and;
- All MHC class I- or MHC class II-restricted neo-epitopes encoded in the substring have no more than two cross-reactive matches in the reference proteome, and;
- All MHC class I- or MHC class II-restricted epitopes encoded in the substring have no more than two cross-reactive matches in the referenceproteome.

This procedure has the effect of removing amino acid substrings containing putative Tregitopes and/or other putative detrimental T cell epitopes (including epitopes that engage T cells with potential host cross-reactivity and/or anergic T cells) and other highly cross-conserved epitopes from mutated sequences. The resulting optimized sequences will only contain epitopes or neo-epitopes that exhibit low degree of similarity with self-sequences. Mutated sequences are discarded from consideration if no substring matching the above criteria can be found.

One hundred thirty-five of the 378 analyzed mutated sequences could be optimized to yield amino acid sequences that contained MHC class I and/or MHC class II restricted neo-epitopes displaying a low degree of self-similarity. These 135 sequences (peptides or polypeptides comprising one or more identified neo-epitopes) were then ranked according to one or more of the following features:

- Immunogenicity-related features:

  ◦ Count of MHC class I neo-epitopes;
  ◦ Minimal percentile rank of a MHC class Ineo-epitope;
  ◦ MHC class I-restricted Treg induction potential of the neoantigen (e.g. peptide or polypeptide comprising one or more identifiedneo-epitopes);
  ◦ Count of MHC class II neo-epitopes (in aspects, which may include one ormore of);
  ◦ Minimal percentile rank of a MHC class II neo-epitope;
  ◦ MHC class II-restricted Treg induction potential of the neoantigen (e.g. peptide or polypeptide comprising one or more identified neo-epitopes);
  ◦ Whether the optimized neoantigen (e.g. peptide or polypeptide comprising one or more identified neo-epitopes) contains both MHC class I and II neo-epitopes.

- Sequencing-related features (in aspects, which may include one or more of):

  ◦ Expression level of the associated transcript;
  ◦ Coverage of the mutation in the tumor DNA, i.e. the number of unique sequencing reads that overlap the genomic position of the mutation;
  ◦ Variant allele fraction (VAF) of the mutation in the tumor DNA, i.e. the relative frequency, from 0 to 1, of the observed mutation across sequencing reads;
  ◦ Other sequencing metadata, as needed.

- Physicochemical-related features (in aspects, which may include one or more of):

  ◦ Net charge of the optimized neoantigen (e.g. peptide or polypeptide comprising one or more identified neo-epitopes);
  ◦ Whether the optimized neoantigen (e.g. peptide or polypeptide comprising one or more identified neo-epitopes) contains at least one charged residue;
  ◦ The count of cysteines (C) within the optimized neoantigen (e.g. peptide or polypeptide comprising one or more identifiedneo-epitopes);
  ◦ Whether the optimized neoantigen (e.g. peptide or polypeptide comprising one or more identified neo-epitopes) contains at least one cysteine (C) and is negatively charged;
  ◦ Whether the optimized neoantigen (e.g. peptide or polypeptide comprising one or more identified neo-epitopes) contains a poly-proline motif('PP');
  ◦ Whether the optimized neoantigen (e.g. peptide or polypeptide comprising one or more identified neo-epitopes) contains at least one methionine(M);
  ◦ Whether the optimized neoantigen (e.g. peptide or polypeptide comprising one or more identified neo-epitopes) contains an N-terminal glutamine (Q);
  ◦ Whether the optimized neoantigen (e.g. peptide or polypeptide comprising one or more identified neo-epitopes) contains a glycine (G) and/or proline (P) in the last or second to last positions;
  ◦ Whether the optimized neoantigen (e.g. peptide or polypeptide comprising one or more identified neo-epitopes) contains a 'DG', DS', 'DA', or 'DN' motif;

◦ The hydropathy index of the optimized neoantigen (e.g. peptide or polypeptide comprising one or more identified neo-epitopes).

Scores can be assigned to neoantigens (e.g. peptide or polypeptide comprising one or more identified neo-epitopes) according to the following scoring scheme (in aspects, scores (e.g., points and/or percentages) that are italicized and bolded may be subject to adjustment; in aspects, the scoring scheme may include one of more of the following scoring steps/penalizing steps:).

- Count of MHC class I neo-epitopes (maximum of *20 points*):

   ◦ Neoantigens (e.g. peptide or polypeptide comprising one or more identified neo-epitopes) containing one or less MHC class I neo-epitopes are assigned *0%* of the points (i.e. 0 point)
   ◦ Neoantigens (e.g. peptide or polypeptide comprising one or more identified neo-epitopes) containing two MHC class I neo-epitopes are assigned *80%* of the points (i.e. 16 points)
   ◦ Neoantigens (e.g. peptide or polypeptide comprising one or more identified neo-epitopes) containing three or more MHC class I neo-epitopes are assigned *100%* of the points (i.e. 20 points)

- Minimal percentile rank of a MHC class I neo-epitope (maximum of *20 points*):

   ◦ Neoantigens (e.g. peptide or polypeptide comprising one or more identified neo-epitopes) where the minimal percentile rank of a MHC class I neo-epitope falls between 5% (inclusive) and 2.5% (exclusive) are assigned *0%* of the points (i.e. 0 point)
   ◦ Neoantigens (e.g. peptide or polypeptide comprising one or more identified neo-epitopes) where the minimal percentile rank of a MHC class I neo-epitope fallsbetween 1% (exclusive) and 2.5% (inclusive) are assigned *50%* of the points (i.e. 10 points)
   ◦ Neoantigens (e.g. peptide or polypeptide comprising one or more identified neo-epitopes) where the minimal percentile rank of a MHC class I neo-epitope falls within 1% (inclusive) are assigned *100%* of the points (i.e. 20 points)

- Count of MHC class II neo-epitopes (maximum of *10 points*):

   ◦ Neoantigens (e.g. peptide or polypeptide comprising one or more identified neo-epitopes) containing one or less MHC class II neo-epitopes are assigned *0%* of the points (i.e. 0 point)
   ◦ Neoantigens (e.g. peptide or polypeptide comprising one or more identified neo-epitopes) containing 2 MHC class II neo-epitopes are assigned *80%* of the points (i.e. 8 points)
   ◦ Neoantigens (e.g. peptide or polypeptide comprising one or more identified neo-epitopes) containing three or more MHC class II neo-epitopes are assigned *100%* of the points (i.e. 10 points)

- Minimal percentile rank of a MHC class II neo-epitope (maximum of *5 points*):

   ◦ Neoantigens (e.g. peptide or polypeptide comprising one or more identified neo-epitopes) where the minimal percentile rank of a MHC class II neo-epitope falls between 5% (inclusive) and 2.5 (exclusive) are assigned *0%* of the points (i.e. 0 point)
   ◦ Neoantigens (e.g. peptide or polypeptide comprising one or more identified neo-epitopes) where the minimal percentile rank of a MHC class II neo-epitope falls between 1% (exclusive) and 2.5% (inclusive) are assigned *50%* of the points (i.e. 2.5 points)
   ◦ Neoantigens (e.g. peptide or polypeptide comprising one or more identified neo-epitopes) where the minimal percentile rank of a MHC class II neo-epitope falls within 1% (inclusive) are assigned *100%* of the points (i.e. 5 points)

- Presence of both MHC class I and II neo-epitopes (maximum of *20 points*):

   ◦ Neoantigens (e.g. peptide or polypeptide comprising one or more identified neo-epitopes) containing MHC class I neo-epitopes only or MHC class II neo-epitopes only are assigned *0%* of the points (i.e. 0 point)
   ◦ Neoantigens (e.g. peptide or polypeptide comprising one or more identified neo-epitopes) containing at least one MHC class I neo-epitope and at least one MHC class II neo-epitope are assigned *100%* of the points (i.e. 20points)

- MHC class I-restricted Treg induction potential (maximum of *5 points*):

  ◦ Neoantigens (e.g. peptide or polypeptide comprising one or more identified neo-epitopes) with a MHC class I-restricted average depth of coverage within the reference proteome, or MHC class I Homology Score (as calculated above), between *0* (inclusive) and *0.25* (exclusive) are assigned *100%* of the points (i.e. 5 points)
  ◦ Neoantigens (e.g. peptide or polypeptide comprising one or more identified neo-epitopes) with a MHC class I-restricted average depth of coverage within the reference proteome, or MHC class I Homology Score, between *0.25* (inclusive) and *0.5* (exclusive) are assigned *50%* of the points (i.e. 2.5 points)
  ◦ Neoantigens (e.g. peptide or polypeptide comprising one or more identified neo-epitopes) with a MHC class I-restricted average depth of coverage within the reference proteome, or MHC class I Homology Score, between 0.5 (inclusive) and *1* (exclusive) are assigned *10%* of the points (i.e. 0.5 points)
  ◦ Neoantigens (e.g. peptide or polypeptide comprising one or more identified neo-epitopes) with a MHC class I-restricted average depth of coverage within the reference proteome, or MHC class I Homology Score, above *1* (inclusive) are assigned *0%* of the points (i.e. 0 point)

- MHC class II-restricted Treg induction potential (maximum of *20 points*):

  ◦ Neoantigens (e.g. peptide or polypeptide comprising one or more identified neo-epitopes) with a MHC class II-restricted average depth of coverage within the reference proteome, or MHC class II Homology Score, between *0* (inclusive) and *0.25* (exclusive) are assigned *100%* of the points (i.e. 20 points)
  ◦ Neoantigens (e.g. peptide or polypeptide comprising one or more identified neo-epitopes) with a MHC class II-restricted average depth of coverage within the reference proteome, or MHC class II Homology Score, between *0.25* (inclusive) and *0.5* (exclusive) are assigned *50%* of the points (i.e. 10points)
  ◦ Neoantigens (e.g. peptide or polypeptide comprising one or more identified neo-epitopes) with a MHC class II-restricted average depth of coverage within the reference proteome, or MHC class II Homology Score, between *0.5* (inclusive) and *1* (exclusive) are assigned *10%* of the points (i.e. 2points)
  ◦ Neoantigens (e.g. peptide or polypeptide comprising one or more identified neo-epitopes) with a MHC class II-restricted average depth of coverage within the reference proteome, or MHC class II Homology Score, above *1* (inclusive) are assigned *0%* of the points (i.e. 0 point)

- Transcript expression (in e.g. Transcript Per Million, TPM, which is calculatedas is known in the art) (maximum of *30 points*):

  ◦ Neoantigens (e.g. peptide or polypeptide comprising one or more identified neo-epitopes) derived from a transcript whose expression lies in the top *10%* of the TPMs are assigned *100%* of the points (i.e. 30 points)
  ◦ Neoantigens (e.g. peptide or polypeptide comprising one or more identified neo-epitopes) derived from a transcript whose expression lies below the top *25%* of the TPMs are assigned *0%* of the points (i.e. 0 points)
  ◦ Neoantigens (e.g. peptide or polypeptide comprising one or more identified neo-epitopes) derived from a transcript whose expression lies between the top *25%* and *10%* of the TPMs are assigned a linearly distributed percent of points.

- Coverage, calculated as is known in the art (maximum of *1 point*):

  ◦ Neoantigens (e.g. peptide or polypeptide comprising one or more identified neo-epitopes) containing a mutation with a depth of coverage in the tumor DNA of less than *20* are assigned *0%* of the points (i.e. 0 point)
  ◦ Neoantigens (e.g. peptide or polypeptide comprising one or more identified neo-epitopes) containing a mutation with a coverage in the tumor DNA of between *20* and *50* (strictly below) are assigned *50%* of the points (i.e. 0.5 point)
  ◦ Neoantigens (e.g. peptide or polypeptide comprising one or more identified neo-epitopes) containing a mutation with a coverage in the tumor DNA of *50* or more are assigned *100%* of the points (i.e. 1 point)

- Variant allele fraction (VAF), calculated as is known in the art (maximum of *20 points*):

  ◦ For neoantigens (e.g. peptide or polypeptide comprising one or more identified neo-epitopes) derived from the mutanome of syngeneic models:

    • Neoantigens (e.g. peptide or polypeptide comprising one or more identified neo-epitopes) containing a mutation with a VAF below 0.5 are assigned *0%* of the points (i.e. 0 point)

- Neoantigens (e.g. peptide or polypeptide comprising one ormore identified neo-epitopes) containing a mutation with a VAF between *0.5* and *0.75* (strictly below) are assigned *50%* of the points (i.e. 10 points)
  - Neoantigens (e.g. peptide or polypeptide comprising one or more identified neo-epitopes) containing a mutation with a VAF equal to or more than *0.75* are assigned *100%* of the points (i.e. 20points)

  ○ For neoantigens (e.g. peptide or polypeptide comprising one or more identified neo-epitopes) derived from the mutanome of patients:

  - Neoantigens (e.g. peptide or polypeptide comprising one or more identified neo-epitopes) containing a mutation with a VAF below *0.1* are assigned *0%* of the points (i.e. 0 point)
  - Neoantigens (e.g. peptide or polypeptide comprising one or more identified neo-epitopes) containing a mutation with a VAF between*0.1* and *0.25* (strictly below) are assigned *50%* of the points (i.e. 10 points)
  - Neoantigens (e.g. peptide or polypeptide comprising one or more identified neo-epitopes) containing a mutation with a VAF equal to or more than *0.25* are assigned *100%* of the points (i.e. 20 points)

Points are then summed and normalized to a 100-point scale, where a perfect neoantigen (e.g. peptide or polypeptide comprising one or more identified neo-epitopes), in other words, a neoantigen that is assigned the maximum number of points, would score 100.

Severe penalties (currently set to a deduction of 100 points, which may be assigned before or after the 100-point normalization) can be assigned to a candidate neoantigen (e.g. peptide or polypeptide comprising one or more identified neo-epitopes) if:

- The neoantigen (e.g. peptide or polypeptide comprising one or more identified neo-epitopes) has no charged residues, or
- The neoantigen (e.g. peptide or polypeptide comprising one or more identified neo-epitopes) has a null net charge, or
- The neoantigen (e.g. peptide or polypeptide comprising one or more identified neo-epitopes) contains at least two cysteines, or
- The neoantigen (e.g. peptide or polypeptide comprising one or more identified neo-epitopes) contains at least one cysteine and is negatively charged, or
- The neoantigen (e.g. peptide or polypeptide comprising one or more identified neo-epitopes) contains an N-terminal glutamine, or
- The neoantigen (e.g. peptide or polypeptide comprising one or more identified neo-epitopes) contains a poly-proline motif, or
- The neoantigen (e.g. peptide or polypeptide comprising one or more identified neo-epitopes) has an hydropathy index greater or equal to 2.

Moderate penalties (currently set to a deduction of 10 points) can be assigned to a candidate neoantigen (e.g. peptide or polypeptide comprising one or more identified neo- epitopes) if:

- The neoantigen (e.g. peptide or polypeptide comprising one or more identified neo-epitopes) contains one cysteine

Minor penalties (currently set to a deduction of 1 point) can be assigned to a candidate neoantigen (e.g. peptide or polypeptide comprising one or more identified neo-epitopes) if:

- The neoantigen (e.g. peptide or polypeptide comprising one or more identified neo-epitopes) is negatively charged, or
- The neoantigen (e.g. peptide or polypeptide comprising one or more identified neo-epitopes) contains at least one methionine, or
- The neoantigen (e.g. peptide or polypeptide comprising one or more identified neo-epitopes) contains a glycine and/or proline in the last or second to last positions, or
- The neoantigen (e.g. peptide or polypeptide comprising one or more identified neo-epitopes) contains a 'DG', DS', 'DA', or 'DN' motif.

Alternatively, scores can be assigned according to following scoring scheme:

$$S_p = \left(C1_p + C2_p\right) \times F_p \times E_p$$

or

$$S_p = \left(C1_p + C2_p\right) \times F_p$$

where:

- $S_p$ corresponds to the score of peptide p;
- $C1p$ corresponds to the MHC class I-restricted immunogenic potential of peptide $p$;
- $C2_p$ corresponds to the MHC class II-restricted immunogenic potential of peptide $p$;
- $F_p$ corresponds to the observed frequency of the mutation encoded by peptide $p$ in the tumor biopsy;
- $E_p$ corresponds to expression of the mutation encoded by peptide $p$ in the tumor biopsy (which in aspects is expression of the gene containing the mutation), expressed as a percentile rank of the observed expression distribution.

The MHC class I-restricted immunogenic potential $C1_p$ of peptide $p$ is calculated as follows:

$$C1_p = \alpha_1 \left( Z1_p - \left( Z1_p \times \frac{H1_p}{\beta_1} \right) \right)$$

or as

$$C1_p = \alpha_1 \sum_{e \in E1_p} \left( Z1_e - \left( Z1_e \times \frac{H1_e}{\beta_1} \right) \right)$$

where:

- $\alpha_1$ (e.g., usually set to 1) and $\beta_1$ (e.g., usually set to 2, which corresponds to situations in the above-defined methods wherein "epitopes with two or more cross-reactive matches in the reference proteome are categorized as exhibiting a high degree of similarity with self") are predefined constants;
- $E1_p$ corresponds to the set of MHC class I-restricted neo-epitopes within peptide $p$;
- $Z1_p$ corresponds to the sum of the percentile ranks of each MHC class I-restricted neo-epitope within peptide $p$, expressed using standard Z-Scores;
- $Z1_e$ corresponds to the percentile rank of the MHC class I-restricted neo-epitope $e$, expressed using standard Z-Scores;
- $H1_p$ is the MHC class I-restricted Homology Score of peptide $p$, as defined above;
- $H1_e$ is the MHC class I-restricted Homology Score of neo-epitope $e$, as defined above.

The MHC class II-restricted immunogenic potential C2p of peptide p is calculated as follows:

$$C2_p = \alpha_2 \left( Z2_p - \left( Z2_p \times \frac{H2_p}{\beta_2} \right) \right)$$

or as

$$C2_p = \alpha_2 \sum_{e \in E2_p} \left( Z2_e - \left( Z2_e \times \frac{H2_e}{\beta_2} \right) \right)$$

where:

- $\alpha_2$ (e.g., usually set to 1) and $\beta_2$ (e.g., usually set to 2, which corresponds to situations in the above-defined methods wherein "epitopes with two or more cross-reactive matches in the reference proteome are categorized as exhibiting a high degree of similarity with self') are predefined constants;
- $E2_p$ corresponds to the set of MHC class II-restricted neo-epitopes within peptide $p$;
- $Z2_p$ corresponds to the sum of the percentile ranks of each MHC class II-restricted neo- epitope within peptide $p$, expressed using standard Z-Scores;
- $Z2_e$ corresponds to the percentile rank of the MHC class II-restricted neo-epitope $e$, expressed using standard Z-Scores;
- $H2_p$ is the MHC class II-restricted Homology Score of peptide $p$, as defined above;
- $H2_e$ is the MHC class II-restricted Homology Score of neo-epitope $e$, as defined above.

Candidate neoantigens (e.g. peptide or polypeptide comprising one or more identified neo-epitopes) are then ranked according to their score, from high to low. The highest ranking neoantigens (e.g. peptide or polypeptide comprising one or more identified neo-epitopes) are selected.

The following tables provide exemplary peptides from a CL-26 cancer model.

| Table 1: Neoantigens comprising one or more identified neo-epitopes | | | | | | | |
|---|---|---|---|---|---|---|---|
| Pep ID | Locus | Sequence *MHC class I neo-epitopes* (restriction) MHC class II neo-epitopes (restriction) | Gene | Ref AA | AA Pos | Alt AA | SEQ ID NO: |
| EO_ CT26_01 | CHR4: 86583172 | Ac-LQARLTSYETLK-NH2 | Haus6 | Ala | 821 | Thr | 1 |
| | | *ARLTSYETL* (Dd) | | | | | 2 |
| | | *ARLTSYETL* (Kd) | | | | | 3 |
| | | ARLTSYETL (Ad) | | | | | 4 |
| EO_ CT26_02 | CHR11: 69649178 | Ac-ETPEACRQARNYLEFSE-NH2 | Fxr2 | Ser | 287 | Asn | 5 |
| | | EACRQARNY (Ad) | | | | | 6 |
| | | *ACRQARNYL* (Kd) | | | | | 7 |
| | | RQARNYLEF (Ad) | | | | | 8 |
| EO_ CT26_03 | CHR2: 128676212 | Ac-SSRVQYVVNPAVKIVF-NH2 | Anapc1 | Asp | 241 | Asn | 9 |
| | | RVQYVVNPA (Ad) | | | | | 10 |
| | | *QYVVNPAVKI* (Kd) | | | | | 11 |
| EO_ CT26_04 | CHR2: 158851764 | Ac-TSKYYMRDVIAIESA-NH2 | Dhx35 | Thr | 646 | Ile | 12 |
| | | *SKYYMRDVI* (Kd) | | | | | 13 |
| | | *KYYMRDVIAI* (Kd) | | | | | 14 |
| | | *YYMRDVIAI* (Dd) | | | | | 15 |
| | | *YYMRDVIAI* (Kd) | | | | | 16 |
| | | YYMRDVIAI (Ad) | | | | | 17 |
| EO_ CT26_05 | CHR6: 3377051 | Ac-PALLIKHMYNKLIS-NH2 | Samd9l | Arg | 70 | His | 18 |
| | | PALLIKHMY (Ad) | | | | | 19 |
| | | LLIKHMYNK (Ed) | | | | | 20 |
| | | *LIKHMYNKL* (Kd) | | | | | 21 |
| | | KHMYNKLIS (Ad) | | | | | 22 |

(continued)

| Pep ID | Locus | Sequence *MHC class I neo-epitopes* (restriction) MHC class II neo-epitopes (restriction) | Gene | Ref AA | AA Pos | Alt AA | SEQ ID NO: |
|---|---|---|---|---|---|---|---|
| EO_ CT26_06 | CHR5: 100037938 | Ac-LSWDTSKKNLTEYLSRF-NH2 | Hnrnpd l | Asp | 163 | Asn | 23 |
| | | *SWDTSKKNL* (Kd) | | | | | 24 |
| | | *TSKKNLTEYL* (Kd) | | | | | 25 |
| | | *SKKNLTEYL* (Kd) | | | | | 26 |
| EO_ CT26_07 | CHR12: 98815985 | NNVHYLNDGDAIIYHTAS-NH2 | Eml5 | Asp | 1396 | Ala | 27 |
| | | *HYLNDGDAI* (Kd) | | | | | 28 |
| | | *HYLNDGDAII* (Kd) | | | | | 29 |
| | | GDAIIYHTA (Ad) | | | | | 30 |
| EO_ CT26_08 | CHRX: 60293650 | Ac-PQPDLYRFVRRISI-NH2 | Atp11c | Gly | 223 | Arg | 31 |
| | | DLYRFVRRI (Ed) | | | | | 32 |
| | | *LYRFVRRISI* (Kd) | | | | | 33 |
| EO_ CT26_09 | CHR12: 98785005 | Ac-DTKCTKADCLFTHMSR-NH2 | Zc3h14 | Pro | 653 | Leu | 34 |
| | | TKCTKADCL (Ad) | | | | | 35 |
| | | *KADCLFTHM* (Kd) | | | | | 36 |
| EO_ CT26_10 | CHR15: 3275728 | EEDGIAVWTLLNGN-NH2 | Sepp1 | Asp | 122 | Ala | 37 |
| | | *DGIAVWTLL* (Dd) | | | | | 38 |
| | | *DGIAVWTLL* (Kd) | | | | | 39 |
| EO_ CT26_11 | CHR7: 55873449 | Ac-ATVHSSMNKMLEE-NH2 | Cyfip1 | Glu | 71 | Lys | 40 |
| | | *TVHSSMNKM* (Kd) | | | | | 41 |
| | | VHSSMNKML (Ed) | | | | | 42 |
| EO_ CT26_12 | CHR12: 91825363 | ILGYRYWTGIGVLQSC-NH2 | Sell | Ala | 299 | Thr | 43 |
| | | *GYRYWTGIGV* (Kd) | | | | | 44 |
| | | *RYWTGIGVL* (Dd) | | | | | 45 |
| | | *RYWTGIGVL* (Kd) | | | | | 46 |
| | | *RYWTGIGVLQ* (Kd) | | | | | 47 |
| EO_ CT26_13 | CHR6: 52729334 | Ac-FCYVTYKGEIRGAS-NH2 | Taxlbp 1 | His | 107 | Tyr | 48 |
| | | *CYVTYKGEI* (Kd) | | | | | 49 |
| EO_ CT26_14 | CHR2: 109298148 | Ac-VKICNMQKAAIL-NH2 | Kifl8a | Glu | 383 | Ala | 50 |
| | | KICNMQKAA (Ad) | | | | | 51 |
| | | *KICNMQKAAI* (Kd) | | | | | 52 |
| EO_ CT26_15 | CHR10: 122089020 | Ac-RQFPVVEANWTMLHDE-NH2 | Tmem5 | Ser | 259 | Asn | 53 |
| | | *WEANWTML* (Dd) | | | | | 54 |
| EO_ CT26_16 | CHR2: 180713221 | Ac-MSYAEKSDEITKD-NH2 | Gid8 | Pro | 7 | Ser | 55 |
| | | *SYAEKSDEI* (Kd) | | | | | 56 |

(continued)

| Pep ID | Locus | Sequence *MHC class I neo-epitopes (restriction) MHC class II neo-epitopes (restriction)* | Gene | Ref AA | AA Pos | Alt AA | SEQ ID NO: |
|---|---|---|---|---|---|---|---|
| EO_CT26_17 | CHR7: 45442527 | Ac-RIQEFVRSHFY-NH2 | Gys1 | Gly | 310 | Ser | 57 |
| | | *RIQEFVRSH* (Kd) | | | | | 58 |
| EO_CT26_18 | CHR5: 129697821 | Ac-KVGLTVKTYEFLERNIP-NH2 | Sept14 | Leu | 97 | Phe | 59 |
| | | *LTVKTYEFL* (Kd) | | | | | 60 |
| | | <u>KTYEFLERN</u> (Ed) | | | | | 61 |
| | | *KTYEFLERNI* (Kd) | | | | | 62 |
| | | *TYEFLERNI* (Dd) | | | | | 63 |
| | | *TYEFLERNI* (Kd) | | | | | 64 |
| EO_CT26_19 | CHR7: 65663891 | Ac-NSSTYWKGNPEMETLQ-NH2 | Tarsl2 | Glu | 353 | Lys | 65 |
| | | *TYWKGNPEM* (Kd) | | | | | 66 |
| | | *TYWKGNPEME* (Kd) | | | | | 67 |
| | | *KGNPEMETL* (Kd) | | | | | 68 |
| EO_CT26_20 | CHR11: 58188928 | Ac-RKSYYMQKYFLDTV | Gm 122 50 | Asn | 390 | Lys | 69 |
| | | *KSYYMQKYFL* (Kd) | | | | | 70 |
| | | *SYYMQKYFL* (Kd) | | | | | 71 |
| | | *SYYMQKYFLD* (Kd) | | | | | 72 |
| | | *YYMQKYFLDT* (Kd) | | | | | 73 |

Table 1: Neoantigens comprising one or more identified neo-epitopes

| Table 2: Highly cross-conserved MHC class II-restricted neo-epitopes | | | | | | | |
|---|---|---|---|---|---|---|---|
| Pep ID | Locus | Sequence <u>MHC class II tolerated/tolerogenic epitopes</u> (restriction) | Gene | Ref AA | AA Pos | Alt AA | SEQ ID NO |
| EO_CT26_Treg_01 | CHR2:109894549 | Ac-PQKLQALQRALQSE-NH2 | Lin7 | Val | 41 | Ala | 74 |
| | | <u>KLQALQRAL</u> (Ad) | | | | | 75 |
| EO_CT26_Treg_02 | CHR2:156167568 | Ac-PHSIKLSRRRSRSKNPFRKDKSPVR-NH2 | Rbm39 | Ser | 129 | Asn | 76 |
| | | <u>IKLSRRRSR</u> (Ed) | | | | | 77 |
| | | <u>KLSRRRSRS</u> (Ed) | | | | | 78 |
| | | <u>LSRRRSRSK</u> (Ed) | | | | | 79 |
| | | <u>SRRRSRSKN</u> (Ed) | | | | | 80 |
| | | <u>SKNPFRKDK</u> (Ed) | | | | | 81 |
| EO_CT26_Treg_03 | CHR2:76712742 | Ac-HPWLKQRIDKVSTK-NH2 | Ttn | Arg | 31554 | Lys | 82 |
| | | <u>WLKQRIDKV</u> (Ed) | | | | | 83 |
| EO_CT26_Treg_04 | CHR3:40805884 | Ac-HSVEMLLKPRRSLDEN-NH2 | Plk4 | Ser | 405 | Leu | 84 |
| | | <u>MLLKPRRSL</u> (Ad, Fd) | | | | | 85 |
| EO_CT26_Treg_05 | CHR3:90025194 | Ac-KTPSSLENDSSNLD-NH2 | Ubap21 | Met | 295 | Leu | 87 |
| | | <u>SSLENDSSN</u> (Ed) | | | | | 87 |
| EO_CT26_Treg_06 | CHR5:106983158 | Ac-SRVQAAQAQHSKDSLYKRDND-NH2 | Cdc7 | Glu | 500 | Lys | 88 |
| | | <u>QAAQAQHSK</u> (Ad) | | | | | 89 |
| | | <u>HSKDSLYKR</u> (Ed) | | | | | 90 |
| EO_CT26_Treg_07 | CHR9:85719917 | Ac-LEGVRLENEKSNVIAKKTGNK-NH2 | Ibtk | Ile | 747 | Ser | 91 |
| | | <u>VRLENEKSN</u> (Ed) | | | | | 92 |
| | | <u>EKSNVIAKK</u> (Ed) | | | | | 93 |
| | | <u>KSNVIAKKT</u> (Ed) | | | | | 94 |
| EO_CT26_Treg_08 | CHR11:40728456 | Ac-DTTLLASTKKAKKSVSKK-NH2 | Hmmr | Ala | 66 | Thr | 95 |
| | | <u>LLASTKKAK</u> (Ed) | | | | | 96 |
| | | <u>STKKAKKSV</u> (Fd) | | | | | 97 |

(continued)

| Table 2: Highly cross-conserved MHC class II-restricted neo-epitopes | | | | | | | |
|---|---|---|---|---|---|---|---|
| Pep ID | Locus | Sequence MHC class II tolerated/tolerogenic epitopes (restriction) | Gene | Ref AA | AA Pos | Alt AA | SEQ ID NO |
| EO_CT26_Treg_09 | CHR12:98794154 | Ac-LLVSSLKIWRKKRDRRCAIH-NH2 | EmI5 | Gly | 44 | Arg | 98 |
| | | SSLKIWRKK (Ed) | | | | | 99 |
| | | SLKIWRKKR (Ed) | | | | | 100 |
| | | LKIWRKKRD (Ed) | | | | | 101 |
| | | WRKKRDRRC (Ed) | | | | | 102 |
| EO_CT26_Treg_10 | CHR16:57513281 | Ac-PMNELDKVVKKHKES-NH2 | Filip11 | Glu | 146 | Lys | 103 |
| | | ELDKVVKKH (Ed) | | | | | 104 |

| Table 3: Highly cross-conserved MHC class I-restricted neo-epitopes | | | | | | | |
|---|---|---|---|---|---|---|---|
| Pep ID | Locus | Sequence MHC class I tolerated/tolerogenic epitopes (restriction) | Gene | Ref AA | AA Pos | Alt AA | SEQ ID NO |
| EO_CT26_Treg_MHCI_01 | CHR1:163996661 | Ac-VLDFGCGSGLLGITA-NH2 | Mettl18 | Leu | 184 | Phe | 105 |
| | | FGCGSGLLG (Dd) | | | | | 106 |
| | | CGSGLLGIT (Dd) | | | | | 107 |
| | | GSGLLGITA (Dd) | | | | | 108 |
| EO_CT26_Treg_MHCI_02 | CHR2:109894549 | Ac-PQKLQALQRALQSE-NH2 | Lin7c | Val | 41 | Ala | 74 |
| | | KLQALQRAL (Dd) | | | | | 75 |
| EO_CT26_Treg_MHCI_03 | CHR3 29979653 | Ac-NMDTRPSSDSSLQHA-NH2 | Mecom | Gly | 301 | Ser | 109 |
| | | TRPSSDSSL (Dd) | | | | | 110 |
| EO_CT26_Treg_MHCI_04 | CHR3:138148475 | Ac-RRDVARSSLRLIIDC-NH2 | Trmt10a | Val | 105 | Ile | 111 |
| | | VARSSLRLI (Dd) | | | | | 112 |
| EO_CT26_Treg_MHCI_05 | CHR5:44192134 | Ac-MSNNFVEIKESVFKK-NH2 | Tapt1 | Gly | 285 | Glu | 113 |
| | | NFVEIKESV (Kd) | | | | | 114 |
| EO_CT26_Treg_MHCI_06 | CHR5:137327443 | Ac-PSRAIPLGIIIAVAY-NH2 | Slc12a9 | Thr | 302 | Ile | 115 |
| | | RAIPLGIII (Dd) | | | | | 116 |
| | | AIPLGIIIA (Dd) | | | | | 117 |
| EO_CT26_Treg_MHCI_07 | CHR7_55873449 | Ac-VHSSMNKMLEEGQEY-NH2 | Cyfip1 | Glu | 71 | Lys | 118 |
| | | SMNKMLEEG (Kd) | | | | | 119 |
| EO_CT26_Treg_MHCI_08 | CHR13_12409236 | Ac-QHFGVEKTVSSLLN-NH2 | Heatr1 | Glu | 552 | Lys | 120 |
| | | GVEKTVSSL (Kd) | | | | | 121 |
| EO_CT26_Treg_MHCI_09 | CHR17_84429178 | Ac-IKCLMENLKDEISQA-NH2 | Thada | Glu | 892 | Lys | 122 |
| | | LMENLKDEI (Kd) | | | | | 123 |
| EO_CT26_Treg_MHCI_10 | CHR19_53635752 | Ac-QQRKFKASRASILSEM-NH2 | Smc3 | Asp | 733 | Ala | 124 |
| | | KFKASRASI (Kd) | | | | | 125 |
| | | FKASRASIL (Dd) | | | | | 126 |

*In vivo Study Design*

The effectiveness of the vaccine was tested in Balb/c mice. Mice are separated into three groups: 1) PBS control; 2) poly-ICLC (vehicle); 3) selected Neoantigen Peptides +poly-ICLC.

- Group 1: PBS, sc injections at days 5, 8, 12, 15, 19, 22, and 26.
- Group 2: poly-ICLC, 50 μg, sc injections at days 5, 8, 12, 15, 19, 22, and 26.
- Group 3: 5 μg / selected Neoantigen Peptide (100 μg total selected Neoantigen Peptides) + 50 μg poly-ICLC, sc injections at days 5, 8, 12, 15, 19, 22, and 26.

All mice (N=10 mice per group) were injected $3 \times 10^5$ tumor cells in 0% Matrigel at day 0. Mice are sacrificed once the tumor volume reached 2,000 mm$^3$ or at day 45, whichever comes first.

Poly-ICLC Fails to Control Tumor Burden

Group 2 (mice immunized with poly-ICLC) showed no reduction in tumor burden compared to Group 1 (mice immunized with PBS). These results are consistent with previously reported experiments by Charles River. Less than 50% of mice from Group 1 survived past day 28, with seven out often mice (70%) reaching a tumor volume of at least 2,000 mm$^3$ by that day.

Selected Neoantigen Peptide Vaccine Reduces Tumor Burden

Group 3 (mice immunized with the selected Neoantigen Peptide vaccine + poly-ICLC) showed a prolonged survival compared to Group 1. In contrast with Group 1 fewer mice from Group 3 reached endpoint by that day.

*Example 2*

Suppression of IFNγ Responses When Co-Administrating Self-Like Neo-Epitopes Along With a Peptide Vaccine

Using the Ancer™ platform, CT26 variants were identified and ranked as potential vaccine candidate peptides. From this this list, 20 neoantigens were selected to be utilized in the development of a peptide-based vaccine developed for the CT26 colorectal cancer syngeneic mouse model (see above).

To demonstrate the importance to identify and eliminate putative "self-like" neo-epitopes due to their potential to dampen immunogenicity responses to vaccine candidates, we have identified CT26 neo-epitopes exhibiting high degree of self-similarity based on JANUSMATRIX™ and tested how their inclusion in vaccine formulations alter their immunogenicity.

Selection of CT26 Self-like Sequences

The 378 variants extracted from the private and public CT26 mutanomes were screened with the JANUSMATRIX™ algorithm to identify neoantigen sequences that displayed a high degree of similarity with murine sequences. Thirty-five and 24 out of these 378 SNVs generated putative "self-like" regulatory T cell neo-epitopes restricted to MHC class II and MHC class I, respectively. Ten of these MHC class-II-restricted sequences were filtered out due to potential manufacturability issues or due to the presence of putative Treg neo-epitopes with limited potential of binding to MHC.

The remaining 25 MHC class II-restricted sequences were manually reviewed to prioritize neoantigens containing the most highly cross-conserved neo-epitopes. In other words, neoantigens encoding MHC class II-restricted neo-epitopes with the highest number of homologous matches with compatible TCR faces within the reference murine proteome were prioritized over the remaining neoantigens. Ten MHC class II "self-like" neoantigens were selected from this list to be used in *in vivo* immunogenicity studies.

In addition, ten MHC class I "self-like" neoantigens were selected to be used in follow-up *in vivo* studies.

Study Design

Balb/c mice were separated into three groups: A) Vehicle control; B) Ancer™-selected CT26 Neoantigen Peptides + adjuvant; C) Ancer™-selected CT26 Neoantigen Peptides + JANUSMATRIX™ MHC class II selected peptides + adjuvant. All vaccines were formulated with the addition of the adjuvant Poly-ICLC. Poly-ICLC, also known as Hiltonol, is a synthetic double-stranded RNA (dsRNA) agonist for pattern recognition receptors (PRRs), and TLR3 agonist. Groups A, B, and C received an initial vaccination with subsequent boosts of vaccine at 2- and 4-weeks post initial vaccination. All mice are sacrificed at 7-10 days after the final boost and spleens were harvested for splenocyte isolation and IFNγ ELISpot assay.

> Group A: 50ug Poly-ICLC in 200 uL
> Group B: 20 Ancer™-selected CT26 Neoantigen Peptides at 5 ug/peptide, 100 ug total peptide, 50ug Poly-ICLC, 200 uL
> Group C: 20 Ancer™-selected CT26 Neoantigen Peptides + 10 JANUSMATRIX™ MHC class II self-like peptides at 5 ug/peptide, 150 ug total peptide, 50ug Poly-ICLC, 200 uL

Isolated splenocytes were plated and stimulated with Ancer™-selected CT26 Neoantigen Peptides (CT26_pool), JANUSMATRIX™ selected peptides (CT26-Treg_pool), class I peptide pool, as well as individual Ancer™-selected CT26 Neoantigen peptide CT26-1 (CT26_peptide 1) and CT26-20 (CT26_peptide 1). Plates were incubated overnight and then read. A positive result was defined as spot forming cells >50 SFC/million splenocytes over background, and a Stimulation index >2-fold over background. Statistical significance was determined by Student's T-test; per mouse - antigen vs. no antigen stimulus, as well as group comparisons (p<0.05).

Ancer™-selected CT26 Neoantigen Peptides are Immunogenic.

CT26 neoantigen peptide pool, Class I only pool, and individual peptide CT26-1 elicited a significant epitope-specific IFNγ response in mice who were vaccinated with CT26 peptides. JanuxMatrix selected peptides were not recognized and no positive results were measured in this stimulation condition. Ancer™-selected CT26 Neoantigen Peptides were determined to be immunogenic in mice that were vaccinated with Ancer™-selected CT26 Neoantigen Peptides + Poly-ICLC. Recall responses to the CT26_pool stimulated a significantly increased epitope-specific IFNγ response compared to cells stimulated with media only. No IFNγ response was measured in the cells stimulated with the CT26-Treg peptide pool demonstrating that irrelevant peptides are not recognized in mice vaccinated with only CT26 peptides. In addition, the CT26 neoantigens were able to stimulate a CD8+ specific T cell response as demonstrated by the positive response to the class I pool. Responses to individual epitopes varied. While CT26-1 (class I and II) epitope appears immunogenic, CT26-20 (Class I) was not.

Ancer™-selected CT26 MHC class II "Self-like" Treg NeoantigenPeptides Suppress Neoantigen Immune Responses

The Ancer™-selected CT26 Neoantigen Peptide pool elicited a strong IFNγ response in group B vaccinated mice that was not seen in group A demonstrating epitope specific responses. The CT26 peptide pool and Class I pool were also able to stimulate strong epitope specific IFNγ responses in group B mice when compared to group A, but the addition of JANUSMATRIX™ selected MHC class II epitopes in the vaccines administered to group C significantly reduces IFNγ responses.

Ancer™-selected CT26 Neoantigen Peptide responses are suppressed in group C mice who were immunized with both Ancer™-selected CT26 Neoantigen Peptides and JANUSMATRIX™ selected neoantigens compared to group B who only received Ancer™-selected CT26 Neoantigen Peptides.

*Example 3*

*In vitro* HLA peptide binding assay

HLA binding of individual tumor-specific mutated peptides (e.g., a peptide or polypeptide comprising one or more identified neo-epitopes, as disclosed herein) identified from patient whole exome sequencing data are validated by testing in an *in vitro* competitive binding assay. In this assay, binding affinity of the test peptide is established by measuring inhibition of HLA binding by a control peptide of know binding affinity. Test peptides are incubated at several concentrations with control peptide at a set concentration along with the corresponding HLA molecule. The level of inhibition of control peptide binding to the HLA molecule is measured at each test peptide concentration and these data are used to establish the binding affinity of the test peptide for the specific HLA molecule evaluated in the assay.

An exemplary Class II HLA Binding Assay is adapted from Steere AC et al., 2006, Antibiotic-Refractory Lyme Arthritis is Associated with HLA-DR Molecules that Bind a Borrelia burgdorferi Peptide. J Exp Med 203(4): 961-71. The assay and yields an indirect measure of peptide-MHC affinity. In the binding assay, a biotinylated, HLA-specific high binding control peptide, and soluble HLA molecules are incubated along with an unlabeled experimental peptide. The experimental peptide is mixed with the binding reagents in an aqueous buffering solution at a pH of 5.4, yielding a final range of eight concentrations from 102,500 nM to 0 nM. The mixture is incubated for 24 hours at 37°C to reach equilibrium. The temperature and pH conditions used for the binding reaction were chosen to represent the physiological conditions found in the endosome during peptide processing and loading onto the HLA molecule. The following day the binding reaction is neutralized, and the HLA-peptide complexes are captured on an enzyme-linked immunosorbent assay (ELISA) plate coated with a pan anti-human HLA-DR antibody (clone L243) for 24 hours at 4°C. After wash and incubation with Europium-labeled Streptavidin for one hour at ambient temperature, time-resolved fluorescence measuring bound labeled control peptide is assessed by a SpectraMax M5 plate reader. All assays are performed in triplicate.

*Example 4*

*In vitro* immunogenicity protocol

Upon verification of peptide binding to patient-specific HLA molecules, the ability of patient's T cells to recognize and respond to tumor-specific mutated peptides (e.g., the subject-specific peptides or polypeptides comprising the at least one identified neo-epitope comprising, consisting, or consisting essentially of an amino acid sequence of the at least one identified neo-epitope (and/or fragments or variants thereof) is determined, and optionally 1 to 12 additional amino acids distributed in any ratio on the N terminus and/or C-terminus of the the at least one identified neo-epitope). Mutated peptides that have been confirmed to bind patient HLA molecules are synthesized and used to pulse patient-derived professional antigen presenting cells (pAPC), such as autologous dendritic cells or CD40L-expanded autologous B cells. Weekly *in vitro* re-stimulations with peptide-pulsed autologous pAPC in the presence of IL-2 and IL-7 are used to expand patient-derived T cells. After several weeks of culture, expanded T cells are tested for peptide-HLA specific reactivity by ELISpot assay to measure IFNγ release. Further characterization of peptide-specific T cell responses may be performed using *in vitro* killing assays such as chromium release assays or comparable methods using patient T cell clones. T cell clones are generated by *in vitro* stimulation using peptide-pulsed autologous pAPC and including the additional step of cloning by limiting dilution following standard protocols.

*Example 5*

Evaluation of inhibitory Treg peptide sequences

The ability of peptide sequences to activate inhibitory regulatory T cell (Treg) responses capable of suppressing effector T cell function can be evaluated using an *in vitro* assay referred to as the tetanus toxoid bystander suppression assay (TTBSA). This assay is based on the ability of Tregs to suppress the function of memory T cells specific to tetanus toxoid. Incubation of peripheral blood mononuclear cells (PBMCs) from patients with a history of immunization with tetanus toxoid results in expansion of tetanus toxoid specific CD4$^+$ effector T cells. When peptides recognized by Tregs are added *in vitro* along with tetanus toxoid, activation, and proliferation of the tetanus toxoid specific CD4$^+$ effector T cells is inhibited by the Tregs in a dose dependent manner. This inhibition of effector T cell activation and proliferation is used as a measure of peptide-specific Treg activity.

*Example 6*

Evaluation of inhibitory Treg peptide sequences

Cross-reactive or auto-reactive T cell responses are tested by *in vitro* priming of T cells using neoepitope peptides containing non-synonymous amino acid substitutions and presented by autologous pAPC. This *in* vitro immunogenicity protocol may follow the methodology established by Wullner et al. (Wullner D, Zhou L, Bramhall E, Kuck A, Goletz TJ, Swanson S, Chirmule N, Jawa V. Considerations for Optimization and Validation of an In vitro PBMC Derived T cell Assay for Immunogenicity Prediction of Biotherapeutics. Clin Immunol 2010 Oct; 137(1): 5-14). T cells that expand following *in vitro* priming to the neoepitope peptides are then tested for reactivity to the corresponding native or wild type (non-mutated) peptide epitopes. Reactivity to native peptide sequences is determined by measuring cytokine production including, but not limited to, IFNy, TNFα, IL- 2 and/or markers of T cell effector function including, but not limited to, CD107a and granzyme B.

*Example 7*

Retrospective survival analysis of TCGA bladder cancer patients

Genomic data derived from the muscle-invasive bladder cancer (BLCA) cohort of The Cancer Genome Atlas (TCGA) was analyzed independently by three pipelines (Fig. 1A) to determine whether an advanced *in silico* T cell epitope screening system (ANCER™) would improve prognostic stratification compared to tumor mutational burden (TMB) or T cell epitope analyses performed with publicly available tools (NetMHCpan/NetMHCIIpan).

Methods:

BLCA patient mutanomes (n=412) were retrieved from the TCGA and evaluated with NetMHCpan/NetMHCIIpan or with ANCER™, a screening platform that combines machine learning-based HLA I and HLA II neo-epitope identification tools with removal of inhibitory regulatory T cell epitopes. BLCA patients were stratified based on median TMB (including silent and non-silent mutations) or median neo-epitope burdens based on NetMHCpan/NetMHCIIpan or ANCER™.

A) For each pipeline, overall survival (OS) between patients with "high" and "low" mutational or neo-epitope burden was analyzed using the Kaplan-Meier method and differences analyzed by log-rank testing. The effect of integrating both CD8 and CD4 neo-epitope burdens was investigated as most mutanome pipelines exclusively focus on CD8 neo-epitopes.

B) In addition, we examined the positive (PPV) and negative predictive values (NPV) of 5-year survival predictors based on median mutational or neo-epitope burdens. For each pipeline, patients with "high" or "low" mutational or neo-epitope burden were predicted to survive more than 5 years. Predicted survival status was compared to observed OS. Patients lost to follow-up before the 5-year cutoff were removed from the analysis. Differences between the models were evaluated using McNemar's Test.

Results:

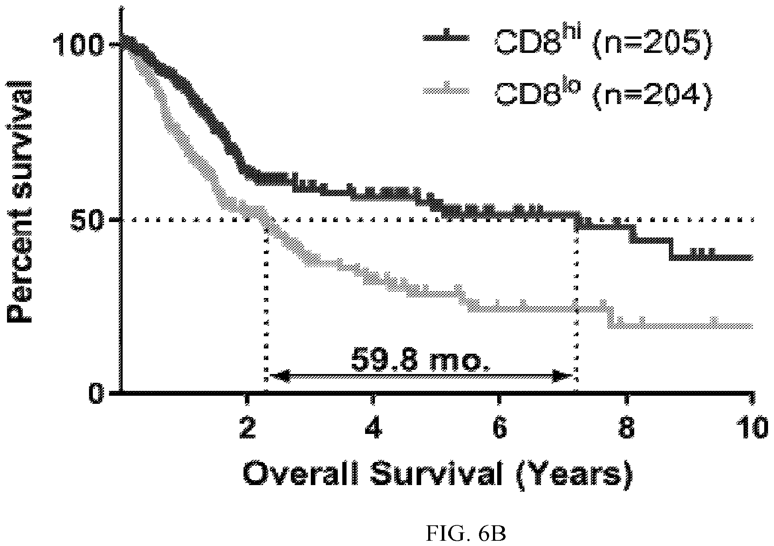

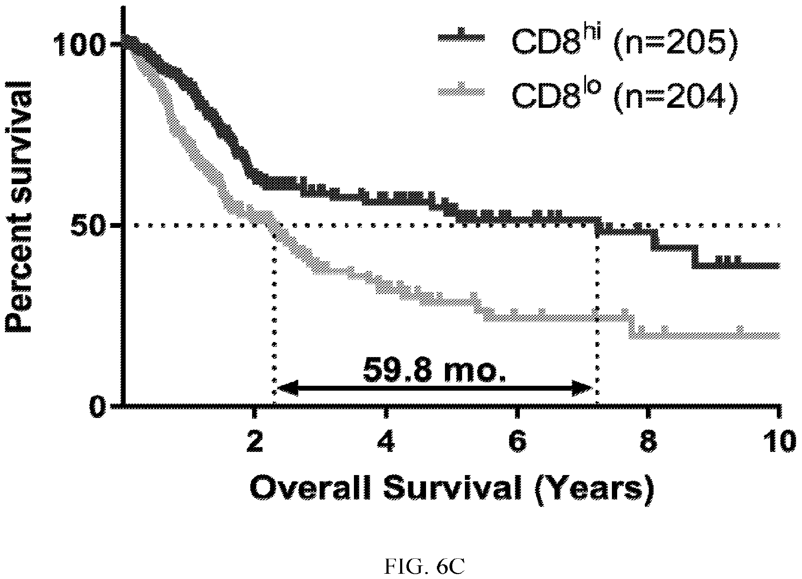

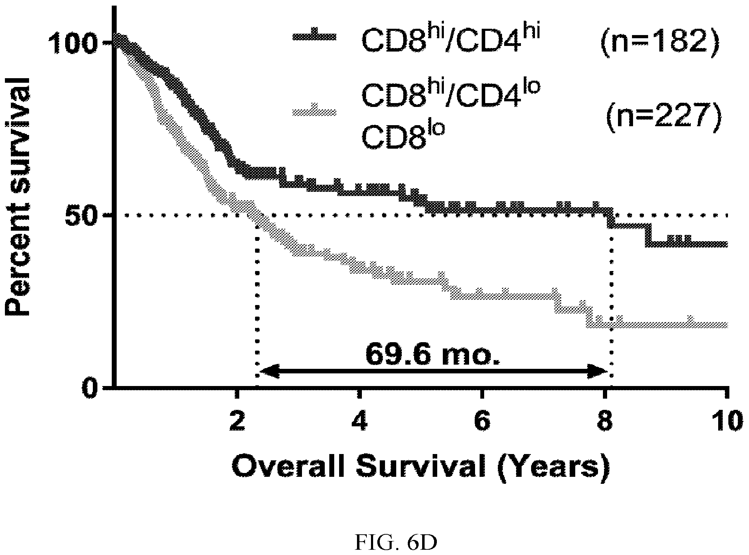

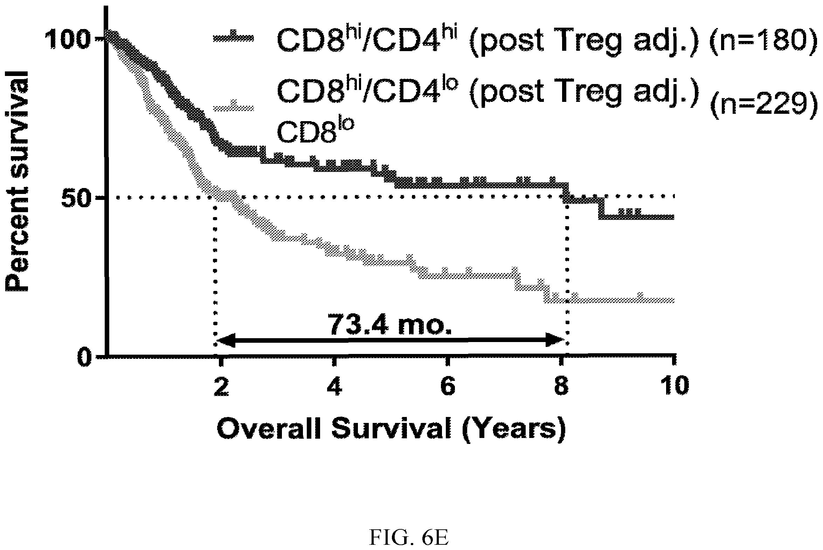

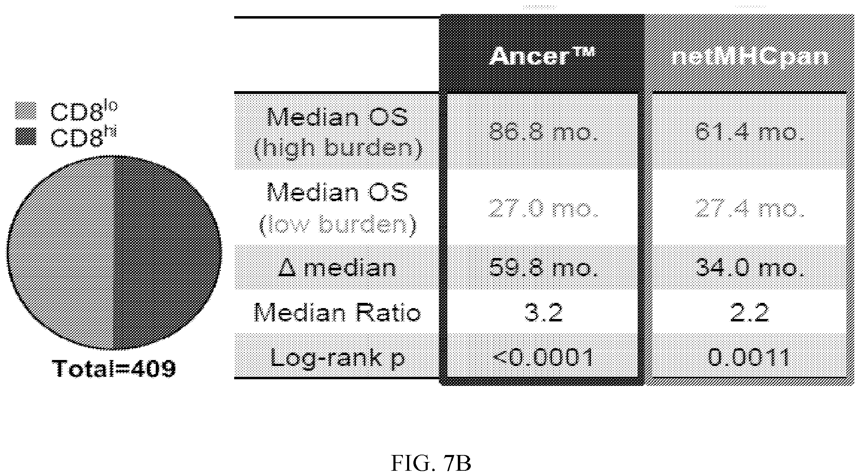

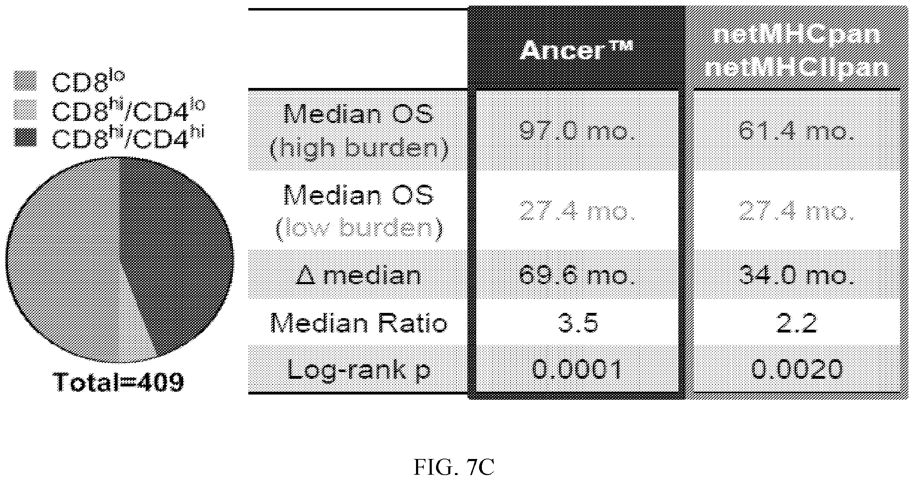

A) Compared to low TMB, high TMB was significantly associated with improved survival (median OS increased by 39 mo., p = 0.0001, Fig. 6A, Fig. 7A). Improved differentiation of median OS was obtained when separating patients based on their total CD8 neo-epitope content, as estimated by ANCER™ (median OS increased by 60 mo., p < 0.0001, Fig. 6B). Removal of CD8 neo-epitopes thought to be "tolerated" did not significantly change differences between median OS (Fig. 6C). However, improved patient stratification was observed when also considering patient total CD4 neo-epitope content in addition to their CD8 neo-epitopes (median OS increased by 70 mo., p = 0.0001, Fig 6D). We further evaluated whether the screening of "tolerated" CD4 sequences could improve our analysis. Upon identifying CD4 neo-epitopes likely to induce T effector (Teff) responses, it was found that the median survival of patients with high CD8 and high CD4 Teff contents was extended to 73.4 months compared to the remainder of the cohort (p < 0.0001, Fig. 6E, 7C). CD8 and CD4 neo-epitope analyses performed with NetMHCpan and NetMHCIIpan did not enhance patient stratification compared to analyses using the TMB or neo-epitopes identified with ANCER™ (median OS increased by 34 mo., p = 0.0020, Fig. 7A, 7B).

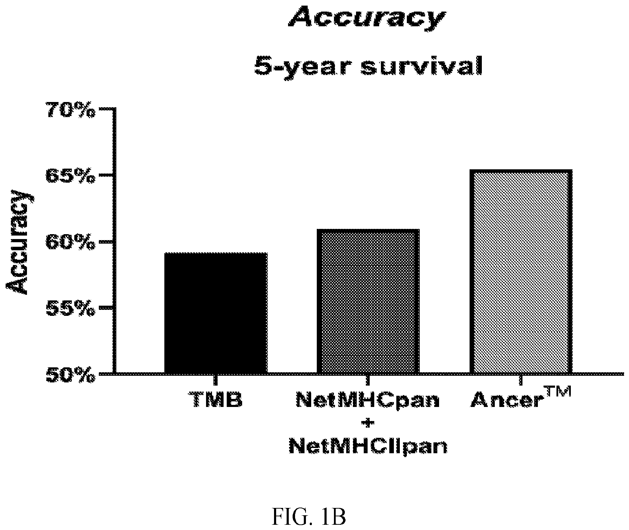

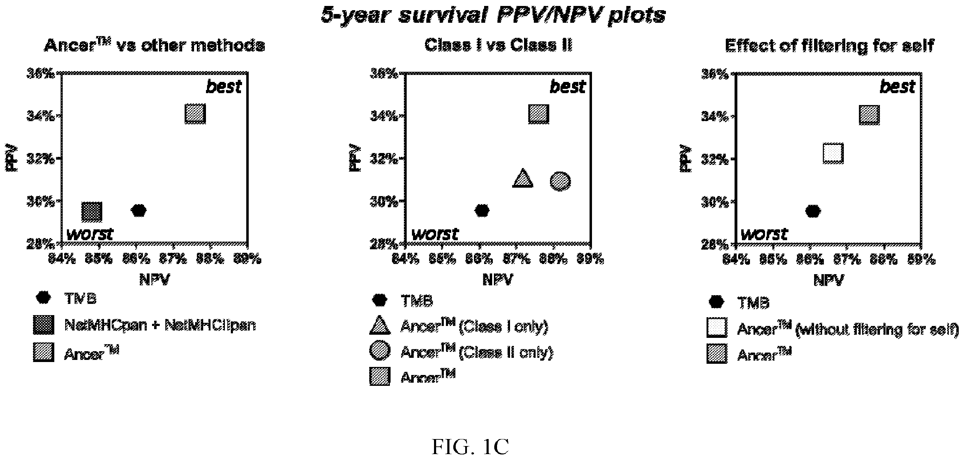

B) Predictive accuracy of 5-year survival status using ANCER™ (median CD8 and CD4 neo-epitope burdens, filtered for "tolerated" neo-epitopes) were significantly more accurate (65% accuracy, Fig. 1B) compared to predictions using median TMB (59% accuracy, ANCER™ vs TMB p = 0.0125) or median neo-epitopes identified by NetMHCpan/NetMHCIIpan (61% accuracy, ANCER™ vs NetMHCpan/NetMHCIIpan p= 0.0192). Improved predictions were also observed when determining the positive and negative predictive value (PPV and NPV) of each model (Fig. 1C, left panel), with increased PPV and NPV obtained for ANCER™ (PPV = 34.1%, NPV = 87.6%) compared to TMB- (PPV = 29.5%, NPV = 86.1%) or NetMHCpan/NetMHCIIpan-based predictors (PPV = 29.5%, NPV = 84.8%). Results also showcased the importance of predicting both Class I and Class II neo-epitopes (Fig. 1C, center panel) and of filtering self-like (including potential inhibitory or cross-reactive) neo-epitopes (Fig. 1C, right panel) when predicting 5-year survival.

Conclusions:

Our analysis suggests that optimal host-immune recognition of CD8, CD4, and Treg epitopes plays a key role in cancer survival. While defining CD8 neo-epitope burden enhanced associations with OS, the inclusion of CD4 Teff neo-epitope burden substantially helped identify long-term survivors. These results suggest that defining the number of true neo-epitopes using ANCER™ represents a novel and useful prognostic or predictive biomarker.

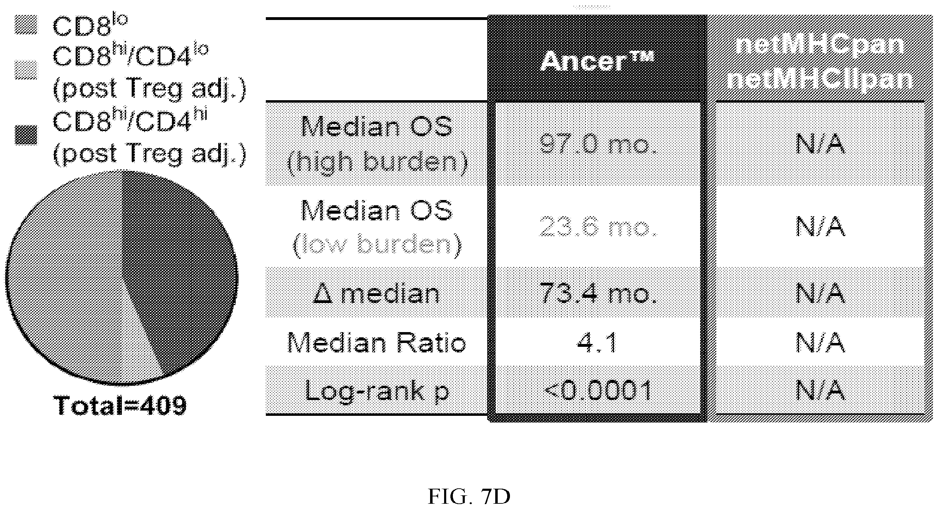

Improved stratification of patients over the TMB analysis was obtained when separating patients based on their Class I neo-epitope content, as estimated by ANCER™ (Fig. 7A, 7B). No improvement over the TMB analysis was obtained when estimating Class I neo-epitope content with public tools. (Fig. 7B) Incremental improvements were obtained when considering tumor "raw" Class II neo-epitope content (i.e. not filtered by JANUSMATRIX™) in addition to the Class I content, as estimated by ANCER™ (Fig. 7C). An analysis with Class II public prediction tools was unsuccessful to improve patient stratification (Fig. 7C). Refining the Class II neo-epitope content with JANUSMATRIX™ (i.e. excluding putative Treg neoepitopes from the analysis) further improved patient stratification. (Fig. 7D). ANCER™ predicts patient 5-year survival status at a higher accuracy, PPV, and NPV than other predictors (Fig. 1B, 1C).

*Example 8*

Association of MHC Class II effector (Teff) vs. regulatory (Treg) neo-epitopes with outcomes in pancreatic cancer patients.

Pancreatic cancer remains one of the deadliest cancers despite immunotherapy breakthroughs. Patients with poor outcomes may have tumors enriched in neo-epitopes activating regulatory T cells (Tregs).

Whole exome sequencing data derived from 13 pancreatic cancer patients with available HLA I and II typing and treated with GVAX, an autologous cancer vaccine engineered to secrete the stimulatory GMCSF cytokine, was analyzed with ANCER™, an in silico neo-epitope identification platform. Distinctive features of ANCER™ are its ability to accurately predict HLA II ligands and to identify tolerated or Treg epitopes. Using ANCER™, we estimated the ratio of effector versus regulatory neo-epitope content for each patient. Fig. 5A shows the number and frequency of Class I and Class II neo-epitopes classified as having low, average, or high cross-reactivity (XR) potential.

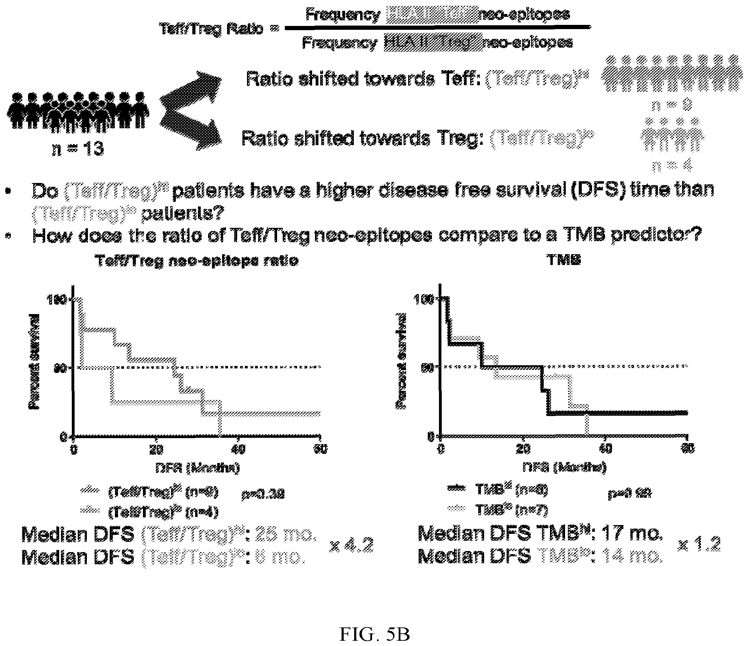

The ratio of Class II effector (Teff) to regulatory (Treg) neo-epitopes was then analyzed to determine whether there is a correlation with disease-free survival. Median disease-free survival (DFS) time of patients with high effector content (25 months) was over four times greater than in patients whose ratio shifted towards high regulatory content (6 months). Analysis of the same patients using their tumor mutational burden (TMB) did not yield conclusive results. In contrast, patients with high TMB had a similar median DFS time (17 months) than patients with low TMB (14 months). Fig. 5B shows patients with higher ratios of Teff/Treg (Teff/Treg)[hi] neo-epitopes had better outcomes that patients with lower ratios of Teff/Treg (Teff/Treg)[lo] neo-epitopes. The ratio of Teff/Treg neo-epitopes was also a better predictor of disease-free survival that tumor mutational burden.

The results indicate that higher numbers of neo-epitopes that may activate Treg in the tumors are in a trend associated with shorter DFS.

## Claims

1. A prognostic method for determining risk of death, of a human subject with a neoplasia, which comprises identifying a population of neoplasia-specific mutations in a neoplasia specimen of a subject; assessing the neoplasia-specific mutations identified to classify Class I and Class II neoantigens encoded by said mutations; analyzing the neoantigens encoded by said mutations to identify and classify neoantigens that engage effector T cells and neo-epitopes that engage regulatory T cells, and computing a prognostic score from the immunogenicity of the population, wherein assessing neoplasia-specific mutations comprises:

   a) determining a binding score for a mutated peptide to one or more MHC molecules, wherein said mutated peptide is encoded by at least one of said neoplasia-specific mutations;
   b) determining a binding score for a non-mutated peptide to the one or more MHC molecules, wherein the non-mutated peptide is identical to the mutated peptide except for the encoded at least one of said neoplasia-specific mutations;
   c) determining the percentile rank of the binding scores of both the mutated peptide of step (a) and the non-mutated peptide of step (b) as compared to an expected distribution of binding scores for at least 10,000 randomly generated peptides using naturally observed amino acid frequencies;
   d) determining the TCR facing amino acid residues of said mutated peptide and said non-mutated peptide; and
   e) using it to calculate a prognostic score when:

      1) the mutated peptide has a determined binding score in the top 5 percentile of the expected distribution and the non-mutated peptide has a determined binding score below the top 10 percentile of the expected distribution; or
      2) the mutated peptide has a determined binding score in the top 5 percentile of the expected distribution, the non-mutated peptide has a determined binding score in the top 10 percentile of the expected distribution, and there is at least one mismatched TCR facing amino acid between the mutated peptide the non-mutated peptide.

2. The method of claim 1, which comprises classification of the Class I and Class II neo-epitopes as effector neoantigens or tolerogenic neoantigens.

3. The method of claim 1, which comprises classification of the Class I and Class II neo-epitopes as effector neoantigens, tolerated neoantigens, or tolerogenic neoantigens.

4. The method of claim 1, which comprises classification of the Class I and Class II neoantigens on a graded scale from a strong effector neoantigen to a strong tolerogenic neoantigen.

5. The method of any one of the previous claims, which comprises identification of neoantigens that are cross-reactive between Class I and Class II.

6. The method of any one of the previous claims, which comprises exclusion of the neoantigen from the computation if it is an effector Class I neoantigen and a tolerogenic Class II neoantigen or an effector Class II neoantigen and a tolerogenic Class I neoantigen, or a tolerated Class I neoantigen and a tolerated Class II antigen, or an effector Class II neoantigen and a tolerated Class I neoantigen.

7. The method of any one of the previous claims, wherein the prognostic score is calculated based on the top 50% of the effector neoantigens and the top 50% of the tolerogenic neoantigens.

8. The method of any one of the previous claims, wherein the prognostic score is calculated based on the top 20% of the effector neoantigens and the top 20% of the tolerogenic neoantigens.

9. The method of any one of the previous claims, wherein the prognostic score is calculated based on the top 5% of the effector neoantigens and the top 5% of the tolerogenic neoantigens.

10. The method of any one of the previous claims, wherein the prognostic score is calculated based at least on the top 100 of the effector neoantigens and at least the top 100 of the tolerogenic neoantigens.

11. The method of any one of the previous claims, which comprises determining strength of binding to Teff and/or Treg cells.

12. The method of claim 11, wherein the strength of binding to Teff and/or Treg cells is known, measured, predicted or calculated.

13. The method of claim 11, wherein strength of binding to a Treg cell is determined by its capacity to inhibit IFNγ production.

14. The method of claim 11, wherein strength of binding to a Teff cell or a Treg cell is determined by comparison to a panel of neoantigens having predetermined Teff and/or Treg activity.


**Patentansprüche**

1. Prognoseverfahren zum Bestimmen des Sterberisikos eines menschlichen Subjekts mit einer Neoplasie, das Identifizieren einer Population neoplasiespezifischer Mutationen in einer Neoplasieprobe eines Subjekts; Beurteilen der identifizierten neoplasiespezifischen Mutationen zum Klassifizieren von Neoantigenen der Klasse I und II, die durch diese Mutationen codiert werden; Analysieren der durch diese Mutationen codierten Neoantigene zum Identifizieren und Klassifizieren von Neoantigenen, die Effektor-T-Zellen aktivieren, und Neoepitopen, die regulatorische T-Zellen aktivieren, und Berechnen eines Prognosewertes aus der Immunogenität der Population umfasst, wobei das Beurteilen neoplasiespezifischer Mutationen Folgendes umfasst:

   a) Bestimmen eines Bindungswerts für ein mutiertes Peptid an ein oder mehrere MHC-Moleküle, wobei das mutierte Peptid durch mindestens eine der neoplasiespezifischen Mutationen codiert wird;
   b) Bestimmen eines Bindungswerts für ein nicht-mutiertes Peptid an das eine oder die mehreren MHC-Moleküle, wobei das nichtmutierte Peptid mit Ausnahme der codierten mindestens einen der neoplasiespezifischen Mutationen mit dem mutierten Peptid identisch ist;
   c) Bestimmen des Prozentrangs der Bindungswerte sowohl des mutierten Peptids aus Schritt (a) als auch des nicht mutierten Peptids aus Schritt (b) im Vergleich zu einer erwarteten Verteilung von Bindungswerten für mindestens 10.000 zufällig generierte Peptide unter Verwendung natürlich beobachteter Aminosäurehäufigkeiten;
   d) Bestimmen der TCR-zugewandten Aminosäurereste des mutierten Peptids und des nicht-mutierten Peptids; und
   e) Verwenden zum Berechnen eines Prognosewertes, wenn:

      1) das mutierte Peptid einen bestimmten Bindungswert im oberen 5-Perzentil der erwarteten Verteilung aufweist und das nichtmutierte Peptid einen bestimmten Bindungswert unterhalb des oberen 10-Perzentils der erwarteten Verteilung aufweist; oder
      2) das mutierte Peptid einen bestimmten Bindungswert im oberen 5-Perzentil der erwarteten Verteilung aufweist, das nicht mutierte Peptid einen bestimmten Bindungswert im oberen 10-Perzentil der erwarteten Verteilung aufweist und zwischen dem mutierten Peptid und dem nicht mutierten Peptid mindestens eine nicht übereinstimmende, dem TCR zugewandte Aminosäure vorliegt.

**2.** Verfahren nach Anspruch 1, das eine Klassifizierung der Neoepitope der Klasse I und II als Effektor-Neoantigene oder tolerogene Neoantigene umfasst.

**3.** Verfahren nach Anspruch 1, das eine Klassifizierung der Neoepitope der Klasse I und II als Effektor-Neoantigene, tolerierte Neoantigene oder tolerogene Neoantigene umfasst.

**4.** Verfahren nach Anspruch 1, das eine Klassifizierung der Neoantigene der Klasse I und II auf einer abgestuften Skala von einem starken Effektor-Neoantigen bis zu einem starken tolerogenen Neoantigen umfasst.

**5.** Verfahren nach einem der vorhergehenden Ansprüche, das eine Identifizierung von Neoantigenen umfasst, die zwischen Klasse I und Klasse II kreuzreaktiv sind.

**6.** Verfahren nach einem der vorhergehenden Ansprüche, das einen Ausschluss des Neoantigens von der Berechnung umfasst, wenn es ein Effektor-Neoantigen der Klasse I und ein tolerogenes Neoantigen der Klasse II oder ein Effektor-Neoantigen der Klasse II und ein tolerogenes Neoantigen der Klasse I oder ein toleriertes Neoantigen der Klasse I und ein toleriertes Antigen der Klasse II oder ein Effektor-Neoantigen der Klasse II und ein toleriertes Neoantigen der Klasse I ist.

**7.** Verfahren nach einem der vorhergehenden Ansprüche, wobei der Prognosewert auf der Grundlage der oberen 50 % der Effektor-Neoantigene und der oberen 50 % der tolerogenen Neoantigene berechnet wird.

**8.** Verfahren nach einem der vorhergehenden Ansprüche, wobei der Prognosewert auf der Grundlage der oberen 20 % der Effektor-Neoantigene und der oberen 20 % der tolerogenen Neoantigene berechnet wird.

**9.** Verfahren nach einem der vorhergehenden Ansprüche, wobei der Prognosewert auf der Grundlage der oberen 5 % der Effektor-Neoantigene und der oberen 5 % der tolerogenen Neoantigene berechnet wird.

**10.** Verfahren nach einem der vorhergehenden Ansprüche, wobei der Prognosewert auf der Grundlage mindestens der oberen 100 der Effektor-Neoantigene und mindestens der oberen 100 der tolerogenen Neoantigene berechnet wird.

**11.** Verfahren nach einem der vorhergehenden Ansprüche, das Bestimmen der Stärke der Bindung an Teff- und/oder Treg-Zellen umfasst.

**12.** Verfahren nach Anspruch 11, wobei die Stärke der Bindung an Teff- und/oder Treg-Zellen bekannt, gemessen, vorhergesagt oder berechnet ist.

**13.** Verfahren nach Anspruch 11, wobei die Stärke der Bindung an eine Treg-Zelle durch ihre Fähigkeit, die IFN-$\gamma$-Produktion zu hemmen bestimmt wird.

**14.** Verfahren nach Anspruch 11, wobei die Stärke der Bindung an eine Teff-Zelle oder eine Treg-Zelle durch Vergleich mit einer Reihe von Neoantigenen mit vorgegebener Teff- und/oder Treg-Aktivität bestimmt wird.

**Revendications**

**1.** Procédé de pronostic permettant de déterminer le risque de décès d'un sujet humain atteint d'une néoplasie, qui comprend l'identification d'une population de mutations spécifiques à la néoplasie dans un échantillon de néoplasie d'un sujet ; l'évaluation des mutations spécifiques à la néoplasie identifiées pour classer les néoantigènes de classe I et de classe II codés par lesdites mutations ; l'analyse des néoantigènes codés par lesdites mutations pour identifier et classer les néoantigènes qui engagent les lymphocytes T effecteurs et les néo-épitopes qui engagent les lymphocytes T régulateurs, et le calcul d'un score de pronostic à partir de l'immunogénicité de la population, dans lequel l'évaluation des mutations spécifiques à la néoplasie comprend :

a) la détermination d'un score de liaison pour un peptide muté à une ou plusieurs molécules du CMH, dans lequel ledit peptide muté est codé par au moins l'une desdites mutations spécifiques à la néoplasie ;
b) la détermination d'un score de liaison pour un peptide non muté à la ou aux molécules du CMH, dans lequel le peptide non muté est identique au peptide muté à l'exception de l'au moins une mutation codée desdites mutations spécifiques à la néoplasie ;

c) la détermination du rang centile des scores de liaison aussi bien du peptide muté de l'étape (a) que du peptide non muté de l'étape (b) par rapport à une distribution attendue des scores de liaison pour au moins 10 000 peptides générés de manière aléatoire en utilisant des fréquences d'acides aminés naturellement observées ;

d) la détermination des résidus d'acides aminés faisant face au TCR dudit peptide muté et dudit peptide non muté ; et

e) l'utilisation de celle-ci pour calculer un score de pronostic lorsque :

1) le peptide muté a un score de liaison déterminé dans les 5 centiles supérieurs de la distribution attendue et le peptide non muté a un score de liaison déterminé inférieur aux 10 centiles supérieurs de la distribution attendue ; ou

2) le peptide muté a un score de liaison déterminé dans les 5 centiles supérieurs de la distribution attendue, le peptide non muté a un score de liaison déterminé dans les 10 centiles supérieurs de la distribution attendue, et il y a au moins un acide aminé faisant face au TCR non apparié entre le peptide muté et le peptide non muté.

2. Procédé selon la revendication 1, qui comprend la classification des néo-épitopes de classe I et de classe II en tant que néoantigènes effecteurs ou néoantigènes tolérogènes.

3. Procédé selon la revendication 1, qui comprend la classification des néo-épitopes de classe I et de classe II en tant que néoantigènes effecteurs, néoantigènes tolérés ou néoantigènes tolérogènes.

4. Procédé selon la revendication 1, qui comprend la classification des néoantigènes de classe I et de classe II sur une échelle graduée allant d'un néoantigène effecteur fort à un néoantigène tolérogène fort.

5. Procédé selon l'une quelconque des revendications précédentes, qui comprend l'identification de néoantigènes qui présentent une réaction croisée entre la classe I et la classe II.

6. Procédé selon l'une quelconque des revendications précédentes, qui comprend l'exclusion du néoantigène du calcul s'il s'agit d'un néoantigène effecteur de classe I et d'un néoantigène tolérogène de classe II ou d'un néoantigène effecteur de classe II et d'un néoantigène tolérogène de classe I, ou d'un néoantigène toléré de classe I et d'un antigène toléré de classe II, ou d'un néoantigène effecteur de classe II et d'un néoantigène toléré de classe I.

7. Procédé selon l'une quelconque des revendications précédentes, dans lequel le score de pronostic est calculé sur la base des 50 % supérieurs des néoantigènes effecteurs et des 50 % supérieurs des néoantigènes tolérogènes.

8. Procédé selon l'une quelconque des revendications précédentes, dans lequel le score de pronostic est calculé sur la base des 20 % supérieurs des néoantigènes effecteurs et des 20 % supérieurs des néoantigènes tolérogènes.

9. Procédé selon l'une quelconque des revendications précédentes, dans lequel le score de pronostic est calculé sur la base des 5 % supérieurs des néoantigènes effecteurs et des 5 % supérieurs des néoantigènes tolérogènes.

10. Procédé selon l'une quelconque des revendications précédentes, dans lequel le score de pronostic est calculé sur la base au moins des 100 supérieurs des néoantigènes effecteurs et des 100 supérieurs des néoantigènes tolérogènes.

11. Procédé selon l'une quelconque des revendications précédentes, qui comprend la détermination de la force de liaison à des cellules Teff et/ou Treg.

12. Procédé selon la revendication 11, dans lequel la force de liaison à des cellules Teff et/ou Treg est connue, mesurée, prédite ou calculée.

13. Procédé selon la revendication 11, dans lequel la force de liaison à une cellule Treg est déterminée par sa capacité à inhiber la production d'IFNγ.

14. Procédé selon la revendication 11, dans lequel la force de liaison à une cellule Teff ou à une cellule Treg est déterminée par comparaison avec un panel de néoantigènes ayant une activité Teff et/ou Treg prédéterminée.

FIG. 1A

FIG. 1B

FIG. 1C

FIG. 2

Fig. 2 CONT'D

Fig. 3

Prime + boost x2, bi-weekly. Subcutaneous injections.
Splenocytes collected at Day 35 and restimulated in IFNγ ELISpot assay with the 20 vaccine peptides.

Co-administration of Ancer™-derived CT26 self-like neo-epitopes identified with JanusMatrix™ significantly reduces IFNγ responses by 5-fold.

FIG. 4

FIG 5A

FIG. 5B

FIG. 6A

FIG. 6B

EP 3 963 335 B1

FIG. 6C

FIG. 6D

FIG. 6E

EP 3 963 335 B1

| | TMB |
| --- | --- |
| Median OS (high burden) | 61.4 mo. |
| Median OS (low burden) | 22.9 mo. |
| Δ median | 38.5 mo. |
| Median Ratio | 2.7 |
| Log-rank p | 0.0001 |

FIG. 7A

EP 3 963 335 B1

Legend:
- CD8^lo
- CD8^hi

Total=409

| | Ancer™ | netMHCpan |
|---|---|---|
| Median OS (high burden) | 86.8 mo. | 61.4 mo. |
| Median OS (low burden) | 27.0 mo. | 27.4 mo. |
| Δ median | 59.8 mo. | 34.0 mo. |
| Median Ratio | 3.2 | 2.2 |
| Log-rank p | <0.0001 | 0.0011 |

FIG. 7B

Legend:
- CD8$^{lo}$
- CD8$^{hi}$/CD4$^{lo}$
- CD8$^{hi}$/CD4$^{hi}$

Total=409

| | Ancer™ | netMHCpan netMHCIIpan |
|---|---|---|
| Median OS (high burden) | 97.0 mo. | 61.4 mo. |
| Median OS (low burden) | 27.4 mo. | 27.4 mo. |
| Δ median | 69.6 mo. | 34.0 mo. |
| Median Ratio | 3.5 | 2.2 |
| Log-rank p | 0.0001 | 0.0020 |

FIG. 7C

EP 3 963 335 B1

EP 3 963 335 B1

CD8$^{lo}$

CD8$^{hi}$/CD4$^{lo}$
(post Treg adj.)

CD8$^{hi}$/CD4$^{hi}$
(post Treg adj.)

**Total=409**

| | Ancer™ | netMHCpan netMHCIIpan |
|---|---|---|
| Median OS (high burden) | 97.0 mo. | N/A |
| Median OS (low burden) | 23.6 mo. | N/A |
| Δ median | 73.4 mo. | N/A |
| Median Ratio | 4.1 | N/A |
| Log-rank p | <0.0001 | N/A |

FIG. 7D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62842800
- US 62880965
- US 62932651
- US 62932654
- US 62936654
- US 2020020089 W
- US 62811207

### Non-patent literature cited in the description

- **NISHIKAWA et al.** Regulatory T Cells in Tumor Immunity. *Int. J. Cancer,* 2010, vol. 127, 759-767
- **GRAUER et al.** Elimination of Regulatory T Cells is Essential for an Effective Vaccination with Tumor Lysate-Pulsed Dendritic Cells in a Murine Glioma Model. *Int. J. Cancer,* 2008, vol. 122, 1794-1802
- **ZHOU et al.** Depletion of Endogenous Tumor-Associated Regulatory T Cells Improves the Efficacy of Adoptive Cytotoxic T-Cell Immunotherapy in Murine Acute Myeloid Leukemia. *Blood,* 2009, vol. 114, 3793-3802
- **BROWN et al.** *Genome Research,* 2014, vol. 24 (5), 743-750
- **MASUCCI et al.** *Journal for ImmunoTherapy of Cancer,* 2016, vol. 4 (1), 1-25
- **RICHARD et al.** *AACR Annual Meeting,* 2018
- **MOISE L. et al.** *Hum Vaccin Immunother,* 2015, vol. 11 (9), 2312-21
- **LIU R. et al.** *Hum Vaccin Immunother.,* 2015, vol. 11 (9), 2241-2252
- **WEBER CA et al.** *Adv Drug Deliv,* 2009, vol. 61 (11), 965-76
- **DE GROOT AS et al.** *AIDS Res Hum Retroviruses,* 1997, vol. 13 (7), 539-41
- **SCHAFER JR et al.** *Vaccine,* 1998, vol. 16 (19), 1880-4
- **DE GROOT AS et al.** *Vaccine,* 2001, vol. 19 (31), 4385-95
- **DE GROOT AR et al.** *Vaccine,* 2003, vol. 21 (27-30), 4486-504
- **CREECH et al.** The Role of Mass Spectrometry and Proteogenomics in the Advancement of HLA Epitope Prediction. *Proteomics,* 2018, vol. 18 (12), e 1700259
- **ABELIN et al.** Mass Spectrometry Profiling of HLA-Associated Peptidomes in Mono-allelic Cells Enables More Accurate Epitope Prediction. *Immunity,* 2017, vol. 46 (2), 315-326
- **HOOF I ; PETERS B ; SIDNEY J et al.** NetMHCpan, a method for MHC class I binding prediction beyond humans. *Immunogenetics.,* 2009, vol. 61, 1-13
- **NIELSEN M ; ANDREATTA M.** NetMHCpan-3.0; improved prediction of binding to MHC class I molecules integrating information from multiple receptor and peptide length datasets. *Genome Med.,* 2016, vol. 8 (33
- **JURTZ V ; PAUL S ; ANDREATTA M et al.** NetMHCpan-4.0: Improved Peptide-MHC Class I Interaction Predictions Integrating Eluted Ligand and Peptide Binding Affinity Data. *J. Immunol.,* 2017, vol. 199, 3360LP-3368
- **KUMAR, P. et al.** A Comprehensive Review on the Role of Co-signaling Receptors and Treg Homeostasis in Autoimmunity and Tumor Immunity. *J. Autoimmun.,* 2018, vol. 95, 77
- **JOSEFOWICZ, S.Z. et al.** Regulatory T cells: mechanisms of differentiation and function. *Annu Rev Immunol,* 2012, vol. 30, 531
- **JORDAN, M.S. et al.** Thymic selection of CD4(+)CD25(+) regulatory T cells induced by an agonist self-peptide. *Nat Immunol,* 2001, vol. 2, 468
- **BENTZEN et al.** T cell receptor fingerprinting enables in-depth characterization of the interactions governing recognition of peptide-MHC complexes. *Nature Biotechnology,* 2018, vol. 36, 1191
- **JURTZ et al.** *NetTCR: sequence-based prediction of TCR binding to peptide-MHC complexes using convolutional neural networks.,* 2018
- **KLINGER et al.** Multiplex identification of antigen-specific T cell receptors using a combination of immune assays and immune receptor sequencing. *PloS One,* 2015, vol. 10, e0141561
- **OGISHI ; YOTSUYANAGI.** Quantitative prediction of the landscape of T cell epitope immunogenicity in sequence space. *Front. Immunol.,* 2019, vol. 10, 827

- **STURNIOLO, T. et al.** *Nat. Biotechnol.,* 1999, vol. 17, 555-561
- **WULLNER D ; ZHOU L ; BRAMHALL E ; KUCK A ; GOLETZ TJ ; SWANSON S ; CHIRMULE N ; JAWA V.** Considerations for Optimization and Validation of an In vitro PBMC Derived T cell Assay for Immunogenicity Prediction of Biotherapeutics. *Clin Immunol,* October 2010, vol. 137 (1), 5-14
- **BOWIE JU et al.** *Science,* 1990, vol. 247 (4948), 130610
- **SAMBROOK et al.** Molecular Cloning, A Laboratory Manual. Cold Spring Harbor Laboratory, 1989
- **MOISE et al.** *Hum. Vaccines Immunother,* 2015
- **WADA et al.** *Sci. Rep.,* 2017
- **MOISE et al.** iVax: An integrated toolkit for the selection and optimization of antigens and the design of epitope- driven vaccines. *Human Vaccin Immunother,* 2015, vol. 11 (9), 2312-21
- **STEERE AC et al.** Antibiotic-Refractory Lyme Arthritis is Associated with HLA-DR Molecules that Bind a Borrelia burgdorferi Peptide. *J Exp Med,* 2006, vol. 203 (4), 961-71
- **WULLNER D ; ZHOU L ; BRAMHALL E ; KUCK A ; GOLETZ TJ ; SWANSON S ; CHIRMULE N ; JAWA V.** Considerations for Optimization and Validation of an In vitro PBMC Derived T cell Assay for Immunogenicity Prediction of Biotherapeutics. *Clin Immunol,* October 2010, vol. 137 (1), 5-14